# FASCICULE DE BREVET EUROPEEN

(11) **EP 1 248 771 B1**
(45) Date de publication et mention de la délivrance du brevet: **03.05.2006**
(21) Numéro de dépôt: 01903885.0
(22) Date de dépôt: 08.01.2001
(51) Int. Cl.: C07D 213/81, A01N 43/40

(54) **PROCEDE DE PREPARATION DE DERIVES DE L'ACIDE 3-HYDROXYPICOLINIQUE**
VERFAHREN ZUR HERSTELLUNG VON 3-HYDROXYPICOLINSÄURE-DERIVATEN
METHOD FOR PREPARING HYDROXYPICOLINIC ACID DERIVATIVES

(30) Priorité: 06.01.2000 FR 0000140
(43) Date de publication de la demande: 16.10.2002
(73) Titulaire: Bayer CropScience S.A., 69009 Lyon (FR); Aventis Pharma S.A., 92165 Antony Cédex (FR)
(72) Inventeur: BACQUE, Eric, F-91390 Morsang-sur-Orge (FR); BARRIERE, Jean-Claude, décédé (FR); VORS, Jean-Pierre, F-69009 Lyon (FR); NIETO-ROMAN, Francisco, E-34005 Palencia (ES); VILLIER, Alain, F-69370 Saint Didier au Mont d'Or (FR)
(74) Mandataire: Nowak, Alexander
(86) Numéro de dépôt international: PCT/FR2001/000044
(87) Numéro de publication internationale: WO 2001/049667

(56) Documents cités:
- EP-A- 1 013 169
- WO-A-00/26191
- WO-A-95/25723
- T. SAKAMOTO ET AL.: "Site-selectivity in the cyanation of 3-substituted pyridine 1-oxides with trimethylsilanecarbonitrile" CHEMICAL AND PHARMACEUTICAL BULLETIN., vol. 33, no. 2, 1985, pages 565-571, XP002166800 PHARMACEUTICAL SOCIETY OF JAPAN. TOKYO., JP ISSN: 0009-2363
- DATABASE WPI Section Ch, Week 199944 Derwent Publications Ltd., London, GB; Class B03, AN 1999-522674 XP002150334 & JP 11 228542 A (MEIJI SEIKA KAISHA LTD), 24 août 1999 (1999-08-24) cité dans la demande

## Description

La présente invention concerne un procédé original de préparation de dérivés de l'acide 3-hydroxypicolinique, et plus particulièrement de dérivés de l'acide picolinique substitué en position 3 par un atome d'oxygène et éventuellement substitué en position 4.

De tels dérivés de l'acide 3-hydroxypicolinique sont connus dans la littérature et notamment dans la demande de brevet WO-A-99/11127 et dans la publication de Kuzo SHIBATA et coll. *(The Journal of Antibiotics, **51*** (12), (1998), 1113-1116), pour leurs proporiétés fongicides contre les champignons phytopathogènes des plantes.

Cependant les procédés de préparation présentés ne permettent par exemple pas l'accès aux dérivés de l'acide 3-hydroxypicolinique substitués en position 4. En effet, les composés décrits dans ces publications proviennent de moûts de fermentation de composés naturels.

D'autres dérivés amides de l'acide 3-hydroxypicolinique sont également connus par les publications de la demande de brevet JP-11228542 et de la demande de brevet EP-A-0 690 061. Là encore les procédés de préparation présentés ne permettent pas l'accès à tous les dérivés de la présente description.

Ainsi, la présente invention concerne un nouveau procédé de préparation de dérivés de l'acide 3-hydroxypicolinique de formule générale (I) : 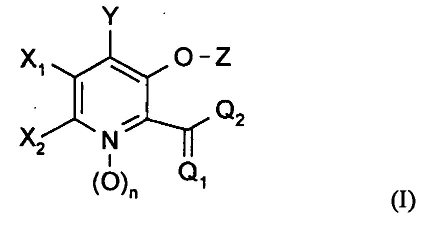 dans lesquels :
- n représente 0 ou 1,
- Q₁ est choisi parmi l'atome d'oxygène, de soufre, le groupe NR₁ et le groupe N-NR₄R₅,
- Q₂ est choisi parmi le groupe OR₂, SR₃ et le groupe -NR₄R₅, ou bien
- Q₁ et Q₂ peuvent former ensemble un cycle de 5 à 7 atomes contenant 2 à 3 atomes d'oxygène et/ou d'azote, éventuellement substitué par un ou plusieurs radicaux identiques ou différents choisis parmi les halogènes, les radicaux alkyle et haloalkyle,
- Z est choisi parmi l'atome d'hydrogène, le radical cyano, un radical alkyle, allyle, aryle, arylalkyle, propargyle, cycloalkyle, halocycloalkyle, alcényle, alcynyle, cyanoalkyle, haloalkyle, alkoxyalkyle, haloalkoxyalkyle, alkylthioalkyle, haloalkylthioalkyle, N-alkylaminoalkyle, N,N-dialkylaminoalkyle, acylaminoalkyle, alkoxycarbonylaminoalkyle, aminocarbonylaminoalkyle, alkoxycarbonyle, N-alkylamino-carbonyle, N,N-dialkylaminocarbonyle, acyle, thioacyle, alkoxythiocarbonyle, N-alkylaminothiocarbonyle, N,N-dialkylaminothiocarbonyle, alkylsulfinyle, haloalkylsulfinyle, alkylsulfonyle, haloalkylsulfonyle, alkoxysulfonyle, aminosulfonyle, N-alkylaminosulfonyle, N,N-dialkylaminosulfonyle, arylsulfinyle, arylsulfonyle, aryloxysulfonyle, N-arylaminosulfonyle, N,N-diarylaminosulfonyle, et N,N-arylalkylaminosulfonyle ;
- Y est choisi parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, alkoxysulfonyle,
   un groupe cycloalkyle, halocycloalkyle, alcényle, alcynyle, alcényloxy, alcynyloxy, alcénylthio, alcynylthio,
   un groupe amino, N-alkylamino, N,N-dialkylamino, -NHCOR₁₀, -NHCSR₁₀, N-alkylaminocarbonylamino, N,N-dialkylaminocarbonylamino, aminoalkyle, N-alkyl-aminoalkyle, N,N-dialkylaminoalkyle, acylaminoalkyle, thioacylamino, alkoxythiocarbonylamino, N-alkylaminothiocarbonylamino, N,N-dialkylaminothiocarbonylamino, N,N-arylalkylaminocarbonylamino, N-alkylsulfinylamino, N-alkylsulfonylamino, N-arylsulfinylamino, N-arylsulfonylamino, N-alkoxysulfonylamino, N-alkoxysulfinylamino, N-haloalkoxysulfinylamino, N-haloalkoxysulfonylamino, N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino, N,N-arylalkylaminothiocarbonylamino, un radical acyle, carboxy, carbamoyle, N-alkylcarbamoyle, N,N-dialkylcarbamoyle, alkoxycarbonyle inférieur, N-arylcarbamoyle, N,N-diarylcarbamoyle, aryloxycarbonyle, N,N-arylalkylcarbamoyle, et un groupe imino de formule : 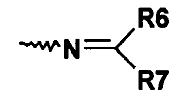
- X₁ et X₂ sont identiques ou différents et sont choisis indépendamment l'un de l'autre parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, et alkoxysulfonyle, ou bien
- X₁ et X₂ peuvent également être joints ensemble, formant ainsi un cycle de 4 à 8 chaînons, saturé, partiellement insaturé ou totalement insaturé, et comportant éventuellement un ou plusieurs hétéroatomes choisis parmi le soufre, l'oxygène, l'azote et le phosphore,
- R₂ et R₃ sont identiques ou différents et sont choisis indépendamment l'un de l'autre parmi un radical alkyle comprenant de 1 à 12 atomes de carbone, haloalkyle, cycloalkyle, halocycloalkyle, alkoxy, haloalkoxy, alkylthio, haloalkylthio, alkoxyalkyle, haloalkoxyalkyle, alkylthioalkyle, haloalkylthioalkyle, cyanoalkyle, acyle, le radical nitro, cyano, carboxy, carbamoyle, 3-oxétanyloxycarbonyle, un radical N-alkylcarbamoyle, N,N-dialkylcarbamoyle, alkoxycarbonyle, alkylthiocarbonyle, haloalkoxycarbonyle, alkoxythiocarbonyle, haloalkoxythiocarbonyle, alkylthiothiocarbonyle, alcényle, alcynyle, N-alkylamino, N,N-dialkylamino, N-alkylaminoalkyle, et N,N-dialkylaminoalkyle, ou bien
   un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement -T-R₈, ou bien,
- R₁, R₄, R₅, R₆ et R₇ sont identiques ou différents et sont choisis indépendamment l'un de l'autre parmi l'atome d'hydrogène, un radical alkyle comprenant de 1 à 12 atomes de carbone en chaîne linéaire ou ramifiée, éventuellement substitué, haloalkyle, cycloalkyle, halocycloalkyle, alkoxy, aryloxy, arylalkoxy, haloalkoxy, alkylthio, haloalkylthio, alkoxyalkyle, haloalkoxyalkyle, alkylthioalkyle, haloalkylthioalkyle, cyanoalkyle, acyle, le radical nitro, cyano, carboxy, carbamoyle, 3-oxétanyloxycarbonyle, un radical N-alkylcarbamoyle, N,N-dialkylcarbamoyle, alkoxycarbonyle, alkylthiocarbonyle, haloalkoxycarbonyle, alkoxythiocarbonyle, haloalkoxythiocarbonyle, alkylthiothiocarbonyle, alcényle, alcynyle, N-alkylamino, N,N-dialkylamino, N-alkylaminoalkyle, et N,N-dialkylaminoalkyle, ou bien
   un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement -T-R₈, ou bien,
- R₄ et R₅ d'une part ou R₆ et R₇ d'autre part peuvent être joints ensemble, formant ainsi un cycle de 4 à 8 chaînons, saturé, partiellement insaturé ou totalement insaturé, et comportant éventuellement un ou plusieurs hétéroatomes choisis parmi le soufre, l'oxygène, l'azote et le phosphore,
- T représente une liaison directe ou un radical divalent choisi parmi un radical -(CH₂)ₘ-, m prenant une valeur comprise entre 1 et 12, bornes incluses, le dit radical étant éventuellement interrompu ou borné par un ou deux hétéroatomes choisis parmi l'azote, l'oxygène et/ou le soufre, un radical oxyalkylène, alkoxyalkylène, carbonyle (-CO-), oxycarbonyle(-O-CO-), carbonyloxy(-CO-O-), sulfinyle (-SO-), sulfonyle (-SO₂-), oxysulfonyle (-O-SO₂-), sulfonyloxy (-SO₂-O-), oxysulfinyle (-O-SO-), sulfinyloxy (-SO-O-), thio (-S-), oxy (-O-), vinyle (-C=C-), éthinyle (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O-, et -O-NR₉-CO-,
- R₈ est choisi parmi l'atome d'hydrogène et un radical aryle, ou hétérocyclyle,
- R₉ et R₁₀, identiques ou différents, sont choisis indépendamment l'un de l'autre parmi l'atome d'hydrogène, un atome d'halogène, le radical hydroxy, mercapto, nitro, thiocyanato, azido, cyano ou pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, arylalkyle, cyanoalkyle, cyanoalkoxy, cyanoalkylthio, alkylsulfinyle, haloalkylsulfinyle, alkylsulfonyle, haloalkylsulfonyle, et alkoxysulfonyle,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis.

Les formes tautomères des composés de formule (I) tels que définis précédemment, sont également comprises dans l'invention. Par formes tautomères, on entend toutes formes isomériques décrites dans l'ouvrage "The tautomerism of heterocycles, Advances in Heterocyclic Chemistry, Supplement 1" par J. Elguero, C. Martin, A.R. Katritzky et P. Linda, édité par Academic Press, New York, 1976, pages 1-4.

Par ailleurs, les termes génériques suivants sont utilisés avec les significations suivantes :
- l'atome d'halogène signifie l'atome de fluor, de chlore, de brome ou d'iode,
- les radicaux alkyle, ainsi que les groupements comportant ces radicaux alkyle (alkoxy, alkylcarbonyle ou acyle, etc.) comportent, sauf indication contraire, de 1 à 6 atomes de carbone en chaîne linéaire ou ramifiée et sont éventuellement substitués,
- les radicaux alkyle, alkoxy et halocycloalkyle halogénés peuvent comporter un ou plusieurs atomes d'halogènes identiques ou différents,
- les radicaux cycloalkyle comportent de 3 à 6 atomes de carbone et sont éventuellement substitués,
- les radicaux alcényle et alcynyle, ainsi que les groupements comportant ces radicaux, comportent, sauf indication contraire, de 2 à 6 atomes de carbone en chaîne linéaire ou ramifiée et sont éventuellement substitués,
- le radical acyle signifie alkylcarbonyle, ou cycloalkylcarbonyle, la partie alkyle contenant de 1 à 6 atomes de carbone et la partie cycloalkyle comprenant de 3 à 6 atomes de carbone, sauf précisions contraires et sont éventuellement substitués,
- le radical alkylène désigne le radical bivalent -(CH₂)ₘ- où m représente un entier égal à 1, 2, 3, 4, 5 ou 6,
- le terme "aryl", dans "aryle" et "arylalkyle" signifie phényle ou naphtyle, éventuellement substitué,
- le terme "hétérocyclyl" dans "hétérocyclyle" et "hétérocyclylalkyle" signifie un cycle de 4 à 10 chaînons saturé, partiellement insaturé ou insaturé, éventuellement substitué, comprenant un ou plusieurs hétéroatomes, identiques ou différents, choisis parmi azote, oxygène, soufre, silicium et phosphore,
- lorsque le radical amino est disubstitué, les deux substituants sont identiques ou différents ou bien peuvent constituer ensemble avec l'atome d'azote qui les porte un hétérocycle azoté saturé, partiellement insaturé ou insaturé, de 5 ou 6 atomes au total,
- lorsque le radical carbamoyle est disubstitué, les deux substituants sont identiques ou différents ou bien peuvent constituer ensemble avec l'atome d'azote qui les porte un hétérocycle azoté saturé, partiellement insaturé ou insaturé, de 5 ou 6 atomes au total,
- sauf précision contraire, l'expression "éventuellement substitué" qualifiant un groupe organique s'applique aux différents radicaux constituant ce groupe et signifie que les différents radicaux sont éventuellement substitués par un ou plusieurs radicaux R₉ et/ou aryle et/ou arylalkyle, identiques ou différents.

Selon une variante de la présente invention, celle-ci concerne un procédé de préparation des dérivés de l'acide 3-hydroxypicolinique, de formule générale (I) telle que définie ci-dessus et pour lesquels :
- X₁ et X₂ représentent chacun un atome d'hydrogène,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Selon une autre variante de la présente invention, celle-ci concerne un procédé de préparation des dérivés de l'acide 3-hydroxypicolinique, de formule générale (I) telle que définie ci-dessus et pour lesquels :
- Q₁ est choisi parmi l'atome d'oxygène et de soufre,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Selon une troisième variante de la présente invention, celle-ci concerne un procédé de préparation des dérivés de l'acide 3-hydroxypicolinique, de formule générale (I) telle que définie ci-dessus et pour lesquels :
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Une autre variante de la présente invention concerne un procédé de préparation des dérivés de l'acide 3-hydroxypicolinique, de formule générale (I) telle que définie ci-dessus et pour lesquels :
- Y est choisi parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, et N,N-arylalkylaminothiocarbonylamino,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Selon encore une autre variante de la présente invention, celle-ci concerne un procédé de préparation des dérivés de l'acide 3-hydroxypicolinique, de formule générale (I) telle que définie ci-dessus et pour lesquels :
- Q₂ représente un groupe -NR₄R₅, dans lequel R₄ représente l'atome d'hydrogène, et R₅ est choisi parmi un radical alkyle comprenant de 1 à 12 atomes de carbone en chaîne linéaire ou ramifiée, éventuellement substitué, haloalkyle, alkoxy, haloalkoxy, alkylthio, haloalkylthio, alcényle, alcynyle et un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement -T-R₈,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Plus particulièrement, la présente invention concerne un procédé de préparation des dérivés de l'acide 3-hydroxypicolinique, de formule générale (I) telle que définie ci-dessus possédant les caractéristiques suivantes prises isolément ou en combinaison :
- X₁ et X₂ représentent chacun un atome d'hydrogène,
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- Y est choisi parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, N,N-arylalkylaminothiocarbonylamino,
- Q₁ est choisi parmi l'atome d'oxygène et de soufre,
- Q₂ représente un groupe -NR₄R₅, dans lequel R₄ représente l'atome d'hydrogène, et R₅ est choisi parmi un radical alkyle comprenant de 1 à 12 atomes de carbone en chaîne linéaire ou ramifiée, éventuellement substitué, haloalkyle, alkoxy, haloalkoxy, alkylthio, haloalkylthio, alcényle, alcynyle et un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement-T-R₈,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Plus particulièrement encore, la présente invention concerne un procédé de préparation des dérivés de l'acide 3-hydroxypicolinique, de formule générale (I) telle que définie ci-dessus possédant les caractéristiques suivantes :
- X₁ et X₂ représentent chacun un atome d'hydrogène,
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- Y est choisi parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, alkylsulfinyle, haloalkylsulfinyle, alkylsulfonyle, haloalkylsulfonyle, alkoxysulfonyle, un groupe amino, N-alkylamino, N,N-dialkylamino, -NHCOR₁₀, -NHCSR₁₀, N-arylamino, N,N-diarylamino, arylcarbonylamino, arylthiocarbonylamino, aryloxythiocarbonylamino, et N,N-arylalkylaminothiocarbonylamino,
- Q₁ est choisi parmi l'atome d'oxygène et de soufre,
- Q₂ représente un groupe -NR₄R₅, dans lequel R₄ représente l'atome d'hydrogène, et R₅ est choisi parmi un radical alkyle comprenant de 1 à 12 atomes de carbone en chaîne linéaire ou ramifiée, éventuellement substitué, haloalkyle, alkoxy, haloalkoxy, alkylthio, haloalkylthio, alcényle, alcynyle et un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement -T-R₈,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis,

Plus spécifiquement encore, la présente invention concerne un procédé de préparation des dérivés de l'acide 3-hydroxypicolinique, de formule générale (I) telle que définie ci-dessus possédant les caractéristiques suivantes :
- X₁ et X₂ représentent chacun un atome d'hydrogène,
- Z est choisi parmi un radical alkyle et l'atome d'hydrogène ou un radical clivable susceptible de redonner l'hydrogène, par exemple, alkoxyalkyle, haloalkoxy-alkyle, alkylthioalkyle, haloalkylthioalkyle, N-alkylaminoalkyle, N,N-dialkylaminoalkyle, acylaminoalkyle, acyle, thioacyle, cyanoalkyle, alkoxythiocarbonyle, N-alkylaminothiocarbonyle, N,N-dialkylaminothiocarbonyle, ou alkylsulfinyle,
- Y est choisi parmi l'atome d'hydrogène, un atome d'halogène, le radical hydroxy, azido, alkoxy, alkylthio, alkylsulfonyle, un groupe amino, -NHCOR₁₀, et -NHCSR₁₀,
- Q₁ représente l'atome d'oxygène,
- Q₂ représente un groupe -NR₄R₅, dans lequel R₄ représente l'atome d'hydrogène, et R₅ est choisi parmi un radical aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement -T-R₈,
- les autres substituants étant tels que définis précédemment,
ainsi que les éventuels N-oxydes, isomères géométriques et/ou optiques, énantiomères et/ou diastéréoisomères, formes tautomères, les sels, complexes métalliques et métalloïdiques des composés de formule (I) tels qu'ils viennent d'être définis.

Dans le cadre de la présente invention, le terme "aryle" signifie phényle ou naphtyle, le terme "arylalkyle" signifiant alors phénylalkyle ou naphtylalkyle, plus particulièrement benzyle, phénéthyle, phénylpropyle, phénylbutyle, naphtylméthyle, naphtyléthyle, naphtylpropyle, ou naphtylbutyle. Il est entendu que ces différents radicaux peuvent être éventuellement substitués par un ou plusieurs radicaux R₉ et/ou aryle et/ou arylalkyle, identiques ou différents.

Les termes hétérocyclyle et hétérocyclylalkyle sont définis de façon similaire, étant entendu que "hétérocycle" signifie un monocycle ou un bicycle de 4 à 10 chaînons saturé, partiellement insaturé ou insaturé, comprenant au moins un hétéroatome choisi parmi azote, oxygène, soufre, silicium et phosphore.

Plus particulièrement le terme "hétérocycle" s'entend comme étant un des cycles (i) à (v) suivants :
- un cycle à 5 maillons décrit par la formule (i) : 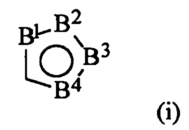
dans laquelle chacun des groupes de la liste B¹, B², B³, B⁴, est choisi parmi les atomes de carbone, d'azote, d'oxygène et de soufre de telle sorte que ladite liste comprenne de 0 à 3 atomes de carbone, de 0 à 1 atome de soufre, de 0 à 1 atome d'oxygène et de 0 à 4 atomes d'azote ;
- un cycle à 6 maillons décrit par la formule (ii) : 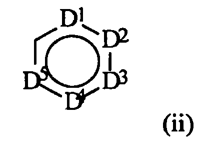
dans laquelle chacun des groupes de la liste D¹, D², D³, D⁴, D⁵, est choisi parmi les atomes de carbone ou d'azote de telle sorte que ladite liste comprenne de 1 à 4 atomes de carbone et de 1 à 4 atomes d'azote ;
- deux cycles fusionnés à 6 maillons chacun, décrits par la formule (iii) : 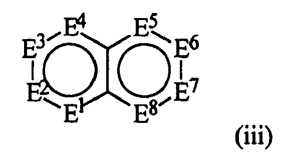
dans laquelle chacun des groupes de la liste E¹, E², E³, E⁴, E⁵, E⁶, E⁷, E⁸ est choisi parmi les atomes de carbone ou d'azote de telle sorte que ladite liste comprenne de 4 à 7 atomes de carbone et de 1 à 4 atomes d'azote ;
- un cycle à 6 maillons et un cycle à 5 maillons fusionnés décrits par la formule (iv) : 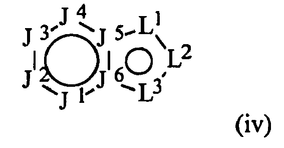 dans laquelle :
   * chacun des groupes de la liste J¹, J², J³, J⁴, J⁵, J⁶ est choisi parmi les atomes de carbone ou d'azote de telle sorte que ladite liste comprenne de 3 à 6 atomes de carbone, et de 0 à 3 atomes d'azote ; et
   * chacun des groupes de la liste L1, L2, L3 est choisi parmi les atomes de carbone, d'azote, d'oxygène ou de soufre de telle sorte que ladite liste comprenne de 0 à 3 atomes de carbone, de 0 à 1 atome de soufre, de 0 à 1 atome d'oxygène et de 0 à 3 atomes d'azote ; et
   * chacun des groupes de la liste J¹, J², J³, J⁴, J⁵, J⁶, L¹, L², L³ est choisi de telle façon que ladite liste comprenne de 3 à 8 atomes de carbone ;
- deux cycles fusionnés à 5 maillons chacun décrits par la formule (v) : 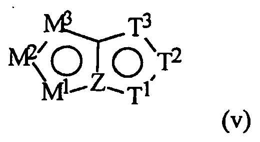 dans laquelle :
   chacun des groupes de la liste M¹, M², M³, représente les atomes de carbone, d'azote, d'oxygène ou de soufre de telle sorte que ladite liste comprenne de 0 à 3 atomes de carbone, de 0 à 1 atome de soufre, de 0 à 1 atome d'oxygène et de 0 à 3 atomes d'azote ;
   chacun des groupes de la liste T¹, T², T³ représentent les atomes de carbone, d'azote, d'oxygène ou de soufre de telle sorte que ladite liste comprenne de 0 à 3 atomes de carbone, de 0 à 1 atome de soufre, de 0 à 1 atome d'oxygène et de 0 à 3 atomes d'azote ;
   Z représente l'atome de carbone ou d'azote ;
   chacun des groupes de la liste M¹, M², M³, T¹, T², T³ est choisi de telle façon que ladite liste comprenne de 0 à 6 atomes de carbone.

Plus particulièrement encore le terme "hétérocycle" signifie dans la présente invention : furanyle, pyrolyle, thiophényle, pyrazolyle, imidazolyle, oxazolyle, isoxazolyle, thiazolyle, isothiazolyle, 1,2,3-oxadiazolyle, 1,2,4-oxadiazolyle, 1,2,5-oxadiazolyle, 1,3,4-oxadiazolyle, 1,2,3-thiadiazolyle, 1,2,4-thiadiazolyle, 1,2,5-thiadiazolyle, 1,3,4-thiadiazolyle, 1,2,3-triazolyle, 1,2,4-triazolyle, tétrazolyle, pyridyle, pyrimidinyle, pyrazinyle, pyridazinyle, 1,2,3-triazinyle, 1,2,4-triazinyle, 1,3,5-triazinyle, 1,2,3,4-tétrazinyle, 1,2,3,5-tétrazinyle, 1,2,4,5-tétrazinyle, benzimidazolyle, indazolyle, benzotriazolyle, benzoxazolyle, 1,2-benzisoxazolyle, 2,1-benzisoxazolyle, benzothiazolyle, 1,2-benzisothiazolyle, 2,1-benzisothiazolyle, 1,2,3-benzoxadiazolyle, 1,2,5-benzoxadiazolyle, 1,2,3-benzothiadiazolyle, 1,2,5-benzothiadiazolyle, quinoléinyle, isoquinoléinyle, quinoxazolinyle, quinazolinyle, cinnolyle ou phtalazyle, ptéridinyle, benzotriazinyle, 1,5-naphthyridinyle, 1,6-naphthyridinyle, 1,7-naphthyridinyle, 1,8-naphthyridinyle, imidazo[2,1-b]thiazolyle, thieno[3,4-b]pyridyle, purine, ou encore pyrolo[1,2-b]thiazolyle.

De manière tout à fait particulière, la présente invention concerne un procédé de préparation des dérivés de l'acide 3-hydroxypicolinique, de formule générale (I) telle que définie ci-dessus, dérivés qui sont :
- le 3-hydroxy-N-{[3-(trifluorométhyl)benzyl]oxy}-2-pyridine carboxamide,
- le 1-{3-hydroxy-2-[(4-phénoxyanilino)carbonyl]-4-pyridinyl}-1,2-triazadién-2-ium,
- le 4-amino-3-hydroxy-N-{4-[4-(trifluorométhyl)phénoxy]phényl}-2-pyridine carboxamide,
- le 4-amino-3-hydroxy-N-[4-(4-méthylphénoxy)phényl]-2-pyridine carboxamide,
- le 4-(formylamino)-3-hydroxy-N-{4-[3-(trifluorométhyl)phénoxy] phényl}-2-pyridine carboxamide,
- le N-[4-(4-chlorophénoxy)phényl]-4-(formylamino)-3-hydroxy-2-pyridine carboxamide,
- le 4-(formylamino)-3-hydroxy-N-{4-[4-(trifluorométhyl)phénoxy] phényl}-2-pyridine carboxamide, et
- le N-[4-(benzyloxy)phényl]-4-(formylamino)-3-hydroxy-2-pyridine carboxamide,
ainsi que les éventuels N-oxydes, isomères géométriques et/ou optiques, énantiomères et/ou diastéréoisomères, formes tautomères, les sels, complexes métalliques et métalloïdiques.

Les composés de formule générale (I) et les composés éventuellement utilisables à titre d'intermédiaires dans les procédés de préparation, et qui seront définis à l'occasion de la description de ces procédés, peuvent exister sous une ou plusieurs formes d'isomères géométriques selon le nombre de doubles liaisons du composé. Les composés de formule générale (I) où Q₁ est -NR₁ ou N-NR₄R₅ peuvent comporter 2 isomères géométriques différents notés *(E)* ou *(Z)* suivant la configuration des deux doubles liaisons. La notation *E* et *Z* peut être remplacée respectivement par les termes syn et anti, ou cis et trans. On se rapportera notamment à l'ouvrage de E. Eliel et S. Wilen "Stereochemistry of Organic Compounds", Ed. Wiley (1994), pour la description et l'utilisation de ces notations.

Les composés de formule générale (I) et les composés éventuellement utilisables à titre d'intermédiaires dans les procédés de préparation, et qui seront définis à l'occasion de la description de ces procédés, peuvent exister sous une ou plusieurs formes d'isomères optiques ou chiraux selon le nombre de centres asymétriques du composé. L'invention concerne donc aussi bien tous les isomères optiques que leurs mélanges racémiques ou scalémiques (on désigne par scalémique un mélange d'énantiomères dans des proportions différentes), ainsi que les mélanges de tous les stéréoisomères possibles en toutes proportions. La séparation des diastéréoisomères et/ou des isomères optiques peut s'effectuer selon les méthodes connues en soi (E. Eliel *ibid.).*

La présente invention concerne donc le procédé de préparation des composés de formule générale (I) et des composés éventuellement utilisables à titre d'intermédiaires dans les procédés de préparation, décrits de manière générale ci-dessous. Bien que générale, cette méthode de préparation fournit l'ensemble des conditions opératoires à mettre en oeuvre pour la synthèse des composés de formule (I) selon la présente invention. Il est bien entendu cependant que l'homme du métier saura adapter cette méthode en fonction des spécifités de chacun des composés qu'il voudra synthétiser.

La préparation des réactifs utilisés dans l'une ou l'autre des méthodes de préparation générale, est habituellement connue en soi et est habituellement décrite spécifiquement dans l'art antérieur ou d'une manière telle que l'homme de l'art peut l'adapter au but souhaité. L'art antérieur utilisable par l'homme de l'art pour établir les conditions de préparation des réactifs, peut être trouvé dans de nombreux ouvrages généraux de chimie comme "Advanced Organic Chemistry" de J.March, Ed. Wiley (1992), "Methoden der organischen Chemie" (Houben-Weyl), Ed. Georg Thieme Verlag ou les "Chemical Abstracts" Ed. American Chemical Society ainsi que dans les bases de données informatiques accessibles au public.

Ainsi, les composés de formule générale (I) peuvent avantageusement être préparés à partir d'un composé de formule (II) (décrit par exemple dans le brevet US-5,652,363) : 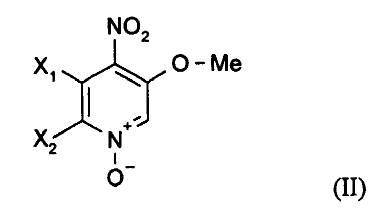 dans lequel X₁ et X₂ sont tels que définis précédemment,
qui est mis au contact d'un cyanure, de dérivés alcalins ou alcalino-terreux de l'acide cyanhydrique en présence d'un agent alkylant et d'un solvant ou de cyanure de triméthylsilyle en présence de chlorure de diméthylcarbamoyle et d'un solvant, pour conduire aux composés de formule (III) : 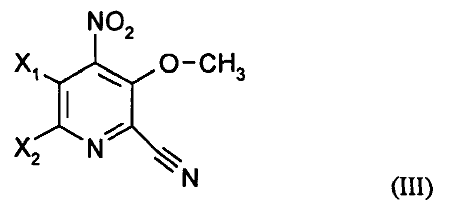 dans leqsuels X₁ et X₂ sont tels que définis précédemment.

Les composés de formule (III) ci-dessus peuvent être transformés en dérivés halogénés correspondants de formule (IVa) : 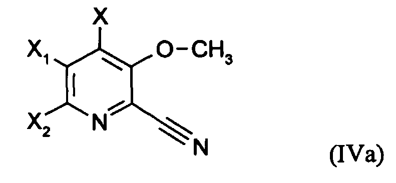 dans lesquels X₁ et X₂ sont tels que précédemment définis et X représente un atome d'halogène choisi parmi fluor, chlore, brome ou iode,
par réaction avec un halogénure d'acyle en présence d'un solvant, tel que, de préférence, mais non exclusivement, un solvant éthéré comme le diéthyléther, le diisopropyléther, le tétrahydrofurane, le dioxane, le 1,2-diméthoxyéthane.

Les dérivés halogénés de formule (IVa) sont alors hydrolysés en composés de formule (Ia) : 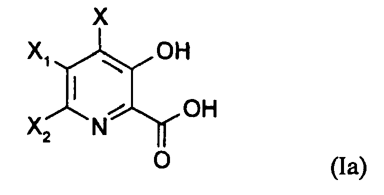 dans lesquels X, X₁ et X₂ sont tels que précédemment définis,
par action d'hydracide à chaud - ou d'une base forte minérale, éventuellement en présence d'eau oxygénée - et éventuellement de tribromure de bore comme décrit dans les ouvrages précités.

Une variante possible pour cette réaction d'hydrolyse consiste à traiter le nitrile de formule (IVa) par un acide, en particulier l'acide chlorhydrique, iodhydrique ou bromhydrique, ou les acides alkylsulfoniques, cette réaction d'hydrolyse étant réalisée dans l'acide en excès, en l'absence ou en présence d'un solvant, au reflux ou à une température comprise entre 20°C et 200°C.

Les composés de formules (III) ou (IVa) peuvent également être mis au contact d'un alcool ou d'un alcoolate en présence d'un solvant tel que, de préférence, mais non exclusivement, un solvant protique ou polaire aprotique, pour conduire aux composés de formule (IVb) : 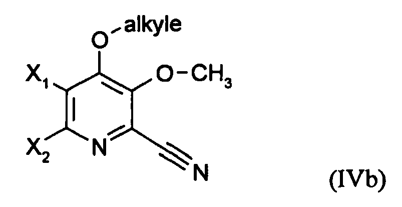 dans lesquels X₁ et X₂ sont tels que précédemment définis,
puis engagés dans une réaction d'hydrolyse selon des conditions opératoires similaires à celles utilisées pour la formation des composés de formule (Ia), pour conduire aux composés de formules respectives (Ib) et (Ib') : 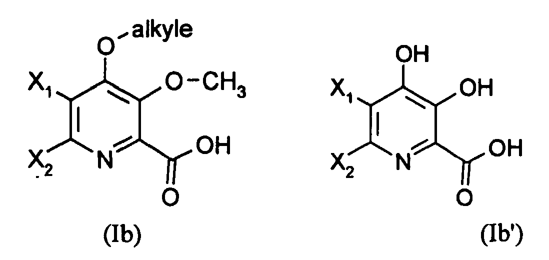 dans lesquels X₁ et X₂ sont tels que précédemment définis.

Les composés de formule (IVa) peuvent également être transformés en dérivés d'acide picolinique de formule (Va) : 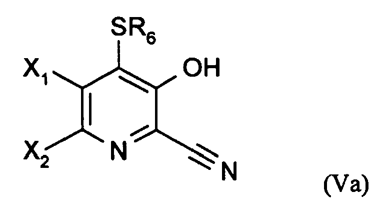 dans lesquels X₁, X₂ et R₆ sont tels que définis précédemment,
en faisant réagir un composé de formule R₆SH, ou un sel alcalin ou alcalino-terreux correspondant, dans un solvant aprotique polaire, comme la diméthylformamide, la diméthylacétamide, la N-méthylpyrrolidone, la diméthylprolylène-urée, le diméthylsulfoxyde, à une température comprise entre 0°C et la température d'ébullition du solvant. Les nitriles de formule (Va) peuvent alors être engagés dans une réaction d'hydrolyse pour conduire aux acides correspondants de formule (Ic) : 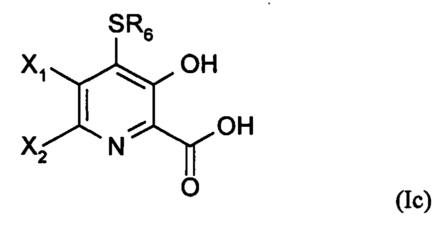 dans lesquels X₁, X₂ et R₆ sont tels que définis précédemment,
selon une réaction similaire à celle employée pour la formation des composés de formule (Ia).

Les halogénures de formule (IVa) peuvent encore être traités avec un sel de l'acide azothydrique, plus particulièrement avec l'azoture de sodium, pour conduire aux composés de formule (Vb) : 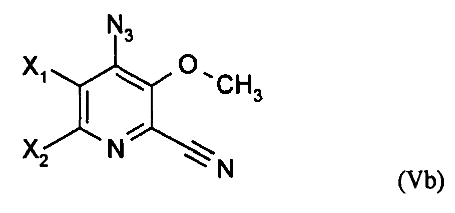 dans lesquels X₁ et X₂ sont tels que définis précédemment,
cette réaction étant conduite préférentiellement dans un solvant polaire aprotique comme la diméthylformamide, la diméthylacétamide, la N-méthylpyrrolidone, la diméthylprolylène-urée ou le diméthylsulfoxyde, à une température comprise entre 0°C et la température d'ébullition du solvant.

Les composés de formule (Vb) peuvent alors être hydrolysés selon des techniques similaires à celles présentées pour la préparation des acides de formule (Ia) ci-dessus, pour conduire aux acides de formule (Id) : 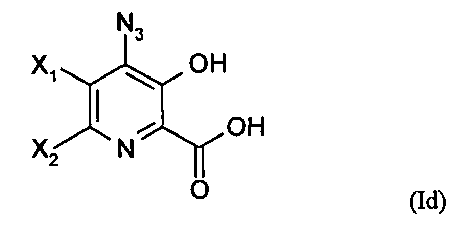 dans lesquels X₁ et X₂ sont tels que définis précédemment.

Les nitrures de formule (Id) sont alors éventuellement réduits en dérivés aminés de formule (Ie) : 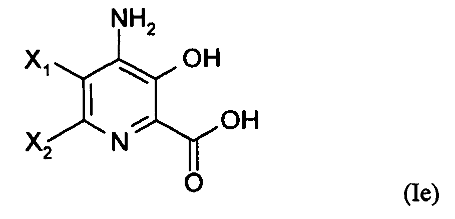 dans lesquels X₁ et X₂ sont tels que définis précédemment,
par action d'un agent réducteur, tel que par exemple l'aluminohydrure de lithium, la triphénylphosphine ou l'hydrogène en présence d'un catalyseur, ou encore tout autre réducteur comme décrit J. March, *ibid,* p 1219-1220.

Les acides de formules (Ia) à (Ie) peuvent être transformés en thioacides, en dérivés imino (-C(=NR₁)) ou encore amino-imino (-C(=N-NR₄R₅)) selon des techniques classiques bien connues de l'homme du métier spécialiste en synthèse organique.

De même, les acides (Ia) à (Ie), ou leurs dérivés thio, imino et imino-amino définis ci-dessus, substitués en position 3 (par rapport à l'atome d'azote pyridinique) par -OH ou -méthoxy peuvent être soumis à diverses réactions déjà connues dans l'art antérieur afin de conduire aux dérivés correspondants substitués en position 3 (par rapport à l'atome d'azote pyridinique) par -O-Z, Z étant tel que défini pour les composés de formule (I).

Suivant les composés de formule générale (I) obtenus selon le procédé décrit précédemment, celui-ci peut comprendre une ou plusieurs étapes supplémentaires. Ainsi les composés de formule (I) obtenus selon le procédé décrit ci-dessus pour lesquels Y représente le radical amino (-NH₂) peuvent être mis au contact d'un agent acylant en présence d'un solvant et éventuellement d'une base afin de conduire aux composés de formule (If) et (Ig) : 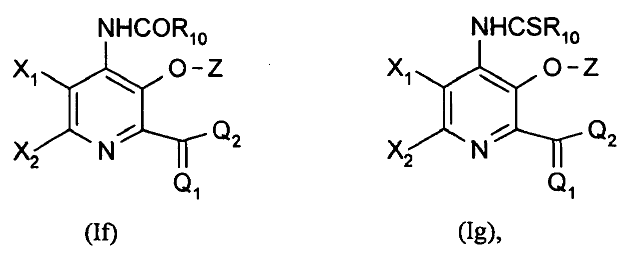 dans lesquels X₁, X₂, Q₁, Q₂, Z et R₁₀ sont tels que définis précédemment. Par agent acylant, on entend préférentiellement mais de manière non limitative, un halogénure d'acyle, un anhydride, un acide, un ester, un amide primaire, et leurs homologues thio-, comme décrit dans J. March, *ibid,* pages 417-424.

De même, les composés de formule (I) obtenus selon le procédé décrit ci-dessus pour lesquels Q₂ représente -OH peuvent être mis en contact avec un réactif de formule R₂OH, R₃SH, ou HNR₄R₅, R₂, R₃, R₄ et R₅ étant tels que définis précédemment, en présence d'un agent activant, pour conduire respectivement aux composés de formules (Ih), (Ii), et (Ij) : 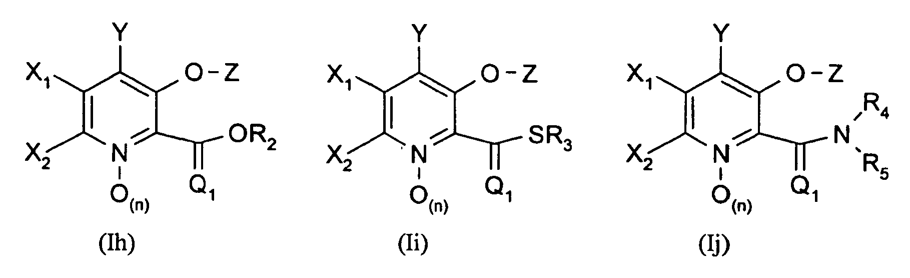 dans lesquels X₁, X₂, Y, Z, n, R₂, R₃, R₄ et R₅ sont tels que définis précédemment.

La réaction précédente est réalisée en présence d'un agent activant comme le chlorure de thionyle, le chlorure d'oxalyle, le dicyclocarbodi-imide, le chlorhydrate de 1-(3-diméthylaminopropyl)-3-éthylcarbodi-imide, le 1-hydroxybenzotriazole, l'oxychlorure de phosphore ou d'autres comme décrits dans les ouvrages de référence en présence base organique ou inorganique, en l'absence ou en présence d'un solvant. Ces réactifs peuvent être le cas échéant liés à une résine polymérique.

La réaction est généralement effectuée à une température comprise entre -80°C et 180°C (de préférence entre 0°C et 150°C) ou au point d'ébullition du solvant utilisé. Le solvant approprié pour cette réaction peut être un hydrocarbure aliphatique comme le pentane, l'hexane, l'heptane, l'octane, un hydrocarbure aromatique comme le benzène, le toluène, les xylènes, les halogénobenzènes, un éther comme le diéthyléther, le di-isopropyléther, le tétrahydrofurane, le dioxane, le diméthoxyéthane, un hydrocarbure halogéné comme le dichlorométhane, le chloroforme, le 1,2-dichloroéthane, le 1,1,1-trichloroéthane, un ester comme l'acétate de méthyle, l'acétate d'éthyle, un nitrile comme l'acétonitrile, le propionitrile, le benzonitrile, un solvant polaire aprotique comme la diméthylformamide, la diméthylacétamide, la N-méthylpyrrolidone, la diméthylprolylèneurée, le diméthylsulfoxyde, la pyridine ou l'eau. Des mélanges de ces différents solvants peuvent être également utilisés.

La durée réactionnelle dépend des conditions utilisées et est généralement comprise entre 0,1 à 48 h. Comme base organique ou inorganique appropriée pour cette réaction, on peut citer les hydroxydes de métaux alcalins et alcalino-terreux comme l'hydroxyde de sodium, de potassium, de césium ou de calcium, les alcoolates de métaux alcalins et alcalino-terreux comme le *tertio*-butylate de potassium, les hydrures de métaux alcalins et alcalino-terreux, comme l'hydrure de sodium, de potassium ou de césium, les carbonates et bicarbonates de métaux alcalins et alcalino-terreux comme le carbonate de sodium, de potassium, de calcium ou le bicarbonate de sodium, de potassium ou de calcium, les bases organiques, de préférences azotées, comme la pyridine, les alkylpyridines, les alkylamines comme la triméthylamine, la triéthylamine ou la di-isopropyléthylamine, les dérivés aza comme le 1,5-diazabicyclo[4.3.0]non-5-ène ou le 1,8-diazabicyclo[5.4.0]undec-7-ène.

La réaction peut-être conduite en utilisant un excès de base liquide à la température réactionnelle, celle-ci agissant alors aussi comme solvant . On peut citer les bases organiques azotés comme la pyridine ou les alkylpyridines.

Il n'y a pas de limitation stricte pour les proportions relatives des composés de formule (I) pour lesquels Q₂ représente -OH et de formule (Ih), (Ii) ou (Ij). Il est cependant avantageux de choisir un rapport molaire (Ih), (Ii) ou (Ij)/ (I) où Q₂ représente -OH compris entre 0,1 et 10, de préférence 0,5 à 2.

Enfin, les composés de formule (I) obtenus selon le procédé décrit ci-dessus pour lesquels n est égal à zéro peuvent être mis en contact avec un agent oxydant comme décrit dans J. March, *ibid.,* pp. 1200, en particulier l'eau oxygénée ou les peracides carboxyliques, boroniques, sulfurique, pour obtenir un composé de formule (Ik) 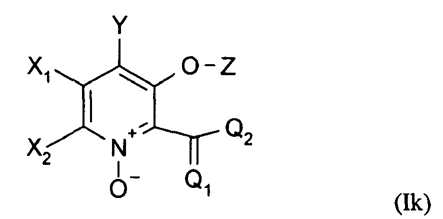 dans laquelle X₁, X₂, Q₁, Q₂, Y et Z, sont tels que définis précédemment.

Il doit être entendu que les réactions décrites dans les paragraphes précédents peuvent être effectuées dans tout ordre différent et convenable pour obtenir les composés de formule (I) souhaités. L'ordre des réactions sera tout particulièrement fixé par les impératifs de compatibilité des différents substituants du noyau pyridinique. Les compatibilités des différents radicaux et réactifs utilisés sont bien connues de l'homme du métier qui pourra de plus se référer aux exemples de préparation des composés de formule (I) exposés plus loin dans cette description.

Les dérivés de l'acide 3-hydroxypicolinique de formule (I) décrits dans la présente invention sont utiles dans le domaine agrochimique et en thérapeutique humaine et animale. En effet, dérivés de l'acide 3-hydroxypicolinique de formule (I) possèdent d'intéressantes propriétés anti-fongiques leur permettant de lutter efficacement contre les maladies fongiques des cultures et également contre les maladies fongiques rencontrées chez les animaux et les humains. Le grand potentiel anti-fongique des composés de formule (I) permet également leur utilisation dans tous les domaines pour lesquels une lutte contre les champignons microscopiques, telles que par exemple les moisissures, est requise et/ou nécessaire.

Les exemples suivants illustrent de manière non limitative quelques exemples de préparation de dérivés de l'acide 3-hydroxypicolinique. Dans les exemples qui suivent, "PF" signifie "Point de Fusion" et est exprimé en ° Celsius (°C).

### Exemple a) : Préparation de la 2-cyano-3-méthoxy-4-nitropyridine

Un mélange de 12,5 g (12,5 moles) du N-oxyde de la 3-méthoxy-4-nitropyridine, 7,72 ml (1,1 eq.) de sulfate de méthyle et 70 ml de 1,2-dichoroéthane est chauffé à 70°C pendant 2,5 heures. On laisse refroidir et on ajoute 70 ml d' eau. On refroidit dans un bain de glace et de sel et on additionne, par portions, 7,55 g (2,1 moles) de cyanure de sodium en contrôlant que la température ne dépasse pas 10°C. Après 4 heures d'agitation, le mélange réactionnel est extrait avec l'éther éthylique, la phase organique est lavée à l'eau, concentrée et le résidu chromatographié (acétate d'éthyle/dichlorométhane). On obtient 7,06 g d' une huile jaune (Rendement 53 %).

### Exemple b) : Préparation de 4-bromo-2-cyano-3-méthoxypyridine

Un mélange de 6 g (0,0335 moles) de 2-cyano-3-méthoxy-4-nitropyridine obtenue dans l'exemple a), 12,37 g (0,100 moles) de bromure d'acétyle et 36 ml de diméthoxy-1,2-éthane est chauffé à 85°C pendant 1,5 heures. On laisse refroidir et on verse la réaction sur 100 g de glace pilée. On ajoute 30 ml de 1,2-dichloroéthane et on neutralise doucement jusqu' à pH = 8 avec une solution aqueuse d'ammoniac à 28%. Après extraction par du 1,2-dichloroéthane, lavage à l'eau, séchage et concentration, le résidu est chromatographié (acétate d'éthyle/heptane, 3:7) pour obtenir 5,32 g (rendement 75%) d'un solide blanc (PF = 116°C). De la même manière, en remplaçant le bromure d'acétyle par le chlorure d'acétyle, est obtenue la 4-chloro-2-cyano-3-méthoxypyridine (rendement 83%) sous forme d'un solide blanc (PF = 91°C).

### Exemple c) : Préparation de 4-azido-2-cyano-3-méthoxypyridine

Sur 1 g (0,0155 moles) de nitrure de sodium dans 25 ml de diméthylformamide à 0°C, on ajoute doucement, 3 g (0,0141 moles) de 4-bromo-2-cyano-3-méthoxypyridine de l'exemple b), dissous dans 40 ml de diméthylformamide. On laisse le mélange sous agitation pendant 6 jours à température ambiante. On dilue la réaction dans 200 ml d'eau glacée et l'on extrait au dichlorométhane. On lave la phase organique deux fois à l'eau, on sèche, on concentre et on chromatographie le résidu (acétate d'éthyle/heptane, 3:7). On obtient 0,87g (rendement 35%) d'un solide blanc (PF = 102°C).

### Exemple d) : Préparation de l'acide 4-chloro-3-hydroxypicolinique

Un mélange de 2 g (0,012 moles) de 4-chloro-2-cyano-3-méthoxypyridine obtenue dans l'exemple b), et 7 ml d'acide chlorhydrique à 37% est chauffé à 100°C pendant 12 heures. Après refroidissement le solide formé est filtré, lavé une fois a l'eau et trois fois avec de l'acétone et séché sous vide pendant 8 heures. On obtient 1,78 g (rendement 86%) d'un solide jaune (PF = 228°C).

De la même manière, sont obtenus les hydroxy-acides suivants :

| Y | Hydracide | Rdt, PF (°C) |
|---|---|---|
| acide 4-bromo-3-hydroxypicolinique | HBr | solide jaune, 82%, 230°C |
| acide 4-azido-3-hydroxypicolinique | HCl | solide violet, 63% |
| acide 3,4-dihydroxypicolinique | HBr | solide blanc, 74%, 264°C |

### Exemple e): Préparation de 2-cyano-3,4-diméthoxypyridine

3 g (0,017 moles) de 2-cyano-3-méthoxy-4-nitropyridine obtenue dans l'exemple a) et une solution de méthylate de sodium préparée avec 0,77g (0,033 moles) de sodium et 65 ml de méthanol sont agités à température ambiante pendant 4 h. On ajoute 100 ml d' eau, on élimine le méthanol et la phase aqueuse est extraite avec le dichlorométhane. La phase organique est lavée à l'eau, séchée, concentrée et le résidu chromatographié (acétate d'éthyle/heptane, 1:1) pour obtenir 1,96 g (rendement 72%) d'un solide blanc (PF = 133°C).

### Exemple f) : Préparation de 2-cyano-3-hydroxy-4-thiométhoxypyridine

2 g de 4-bromo-2-cyano-3-méthoxypyridine obtenue dans l'exemple b) et 2,16 g de thiométhylate de sodium dans 40ml de diméthylformamide anhydre sont chauffés à 85°C pendant 5 heures. Après refroidissement et ajout de 20ml d'eau, le mélange réactionnel est concentré à sec. Le résidu est extrait trois fois avec du méthanol chaud. La phase méthanolique refroidie est filtrée et concentrée. On obtient 1,51 g (rendement 97%) d'un miel marron utilisé brut.

### Exemple g) : Préparation de l'acide 3-hydroxy-4-thiométhoxypicolinique

2,5 g (0,015 moles) de 2-cyano-3-hydroxy-4-thiométhoxypyridine de l'exemple f), 8,5 g d'hydroxyde de potassium et 25 ml d'eau sont chauffés à reflux pendant 2,5 heures. On laisse refroidir et dans un bain glacé on neutralise doucement avec de l'acide chlorhydrique 1N jusqu'à pH = 2-3. On filtre le solide formé. On lave le solide une fois à l'eau et trois fois avec de l'acétone ; on sèche sous vide pendant 8 heures. On obtient 1,81 g (rendement 68%) d'un solide blanc (PF = 247°C).

### Exemple h) : Préparation de l'acide 3,4-diméthoxypicolinique

Un mélange 1 g de 3,4-diméthoxy-2-cyanopyridine obtenue dans l'exemple e) et 3,5 g d'hydroxyde de potassium dans 15 ml d'eau est chauffé à 85°C pendant une demi-heure. On laisse refroidir et dans un bain glacé, on ajoute doucement de l'acide chlorhydrique jusqu'à pH = 2-3. Après concentration à sec, le résidu est extrait trois fois avec du méthanol chaud ; on laisse refroidir, on filtre et on concentre. On obtient un solide utilisé brut.

### Exemple i) : Préparation du N-oxyde de l'acide 3-hydroxypicolinique

À un mélange de 20ml d'acide acétique et 20ml d'eau oxygénée, sont ajoutés 2 g d'acide 3-hydroxypicolinique ; le tout est porté à 80°C pendant 5 heures. Après élimination des solvants sous vide, le solide obtenu est lavé avec de l'alcool chaud pour obtenir 2,02 g de composé attendu sous la forme d'un solide blanc (PF = 182°C).

### Exemple 1 :

### 3-hydroxy-4-méthoxy-N-paraphénoxyphénylpicolinamide

0,046g de paraphénoxyaniline, 0,04g d'acide 3-hydroxy-4-méthoxypicolinique (obtenu selon un procédé similaire à celui décrit dans l'exemple g)), 0,034g de 1-hydroxybenzotriazole et 0,060 g de chlorhydrate de 1-(3-diméthylaminopropyl)-3-éthylcarbodi-imide sont chauffés dans 2 ml de pyridine entre 75 et 85°C pendant 1 à 2 heures. Après refroidissement, le résidu est repris dans une mélange de dichlorométhane et 2ml d'acide chlorhydrique 1N . Après extraction par le dichlorométhane, concentration et chromatographie sur silice on obtient 0,057g du composé en titre, solide jaune (PF = 186°C).

### Exemple 2 :

### 4-amino-3-hydroxy-N-paraphénoxyphénylpicolinamide

À 0,14g de 4-azido-3-hydroxy-N-paraphénoxyphénylpicolinamide (obtenue à partir du composé de l'exemple 1 selon les modes opératoires décrits dans les exemples a) à g))) dissous dans un mélange éthanol/acétate d'éthyle, 1:2, on ajoute une pointe de spatule de palladium sur charbon à 10%. L'hydrogénation est conduite à 20 bars de pression et à température ambiante pendant 4-5 heures. Après filtration, concentration et chromatographie du résidu dans l'acétate d'éthyle, on obtient 0,099g d'un solide blanc (PF : 197°C).

### Exemple 3 :

### 4-formamido-3-hydroxy-N-paraphénoxyphénylpicolinamide

On chauffe à reflux 61,2 mg d'anhydride acétique et 27,6 mg d' acide formique pendant 4 heures et on ajoute 46 mg de 4-amino-3-hydroxy-N-paraphénoxyphénylpicolinamide de l'exemple 2, dissous dans 5 ml de tétrahydrofurane. Après 8 heures de reflux, le mélange réactionnel est concentré et purifié par chromatographie pour donner 39 mg d'un solide jaune PF 208°C.

Les composés décrits dans les tableaux 1 et 2 suivants sont préparés de manière analogue :

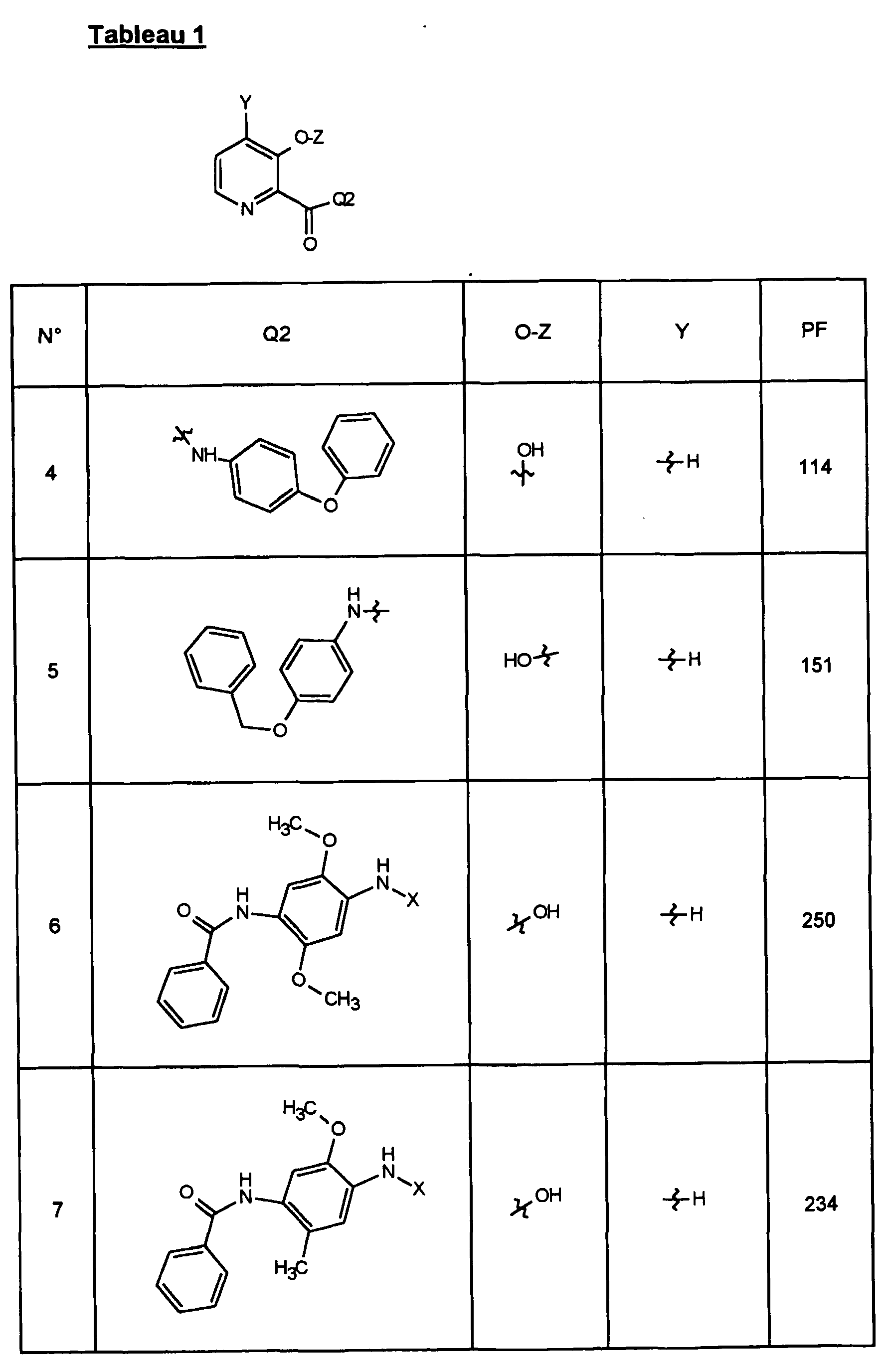

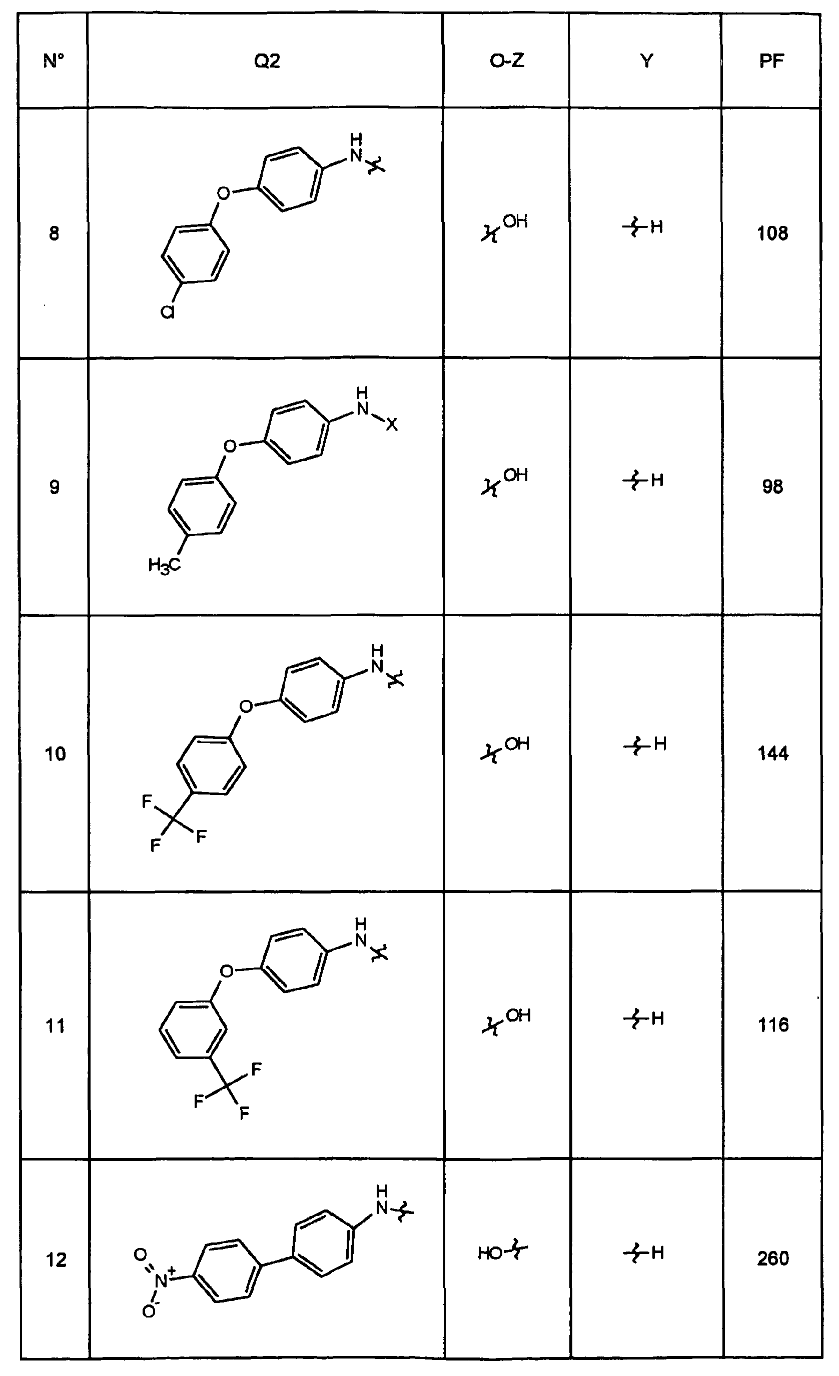

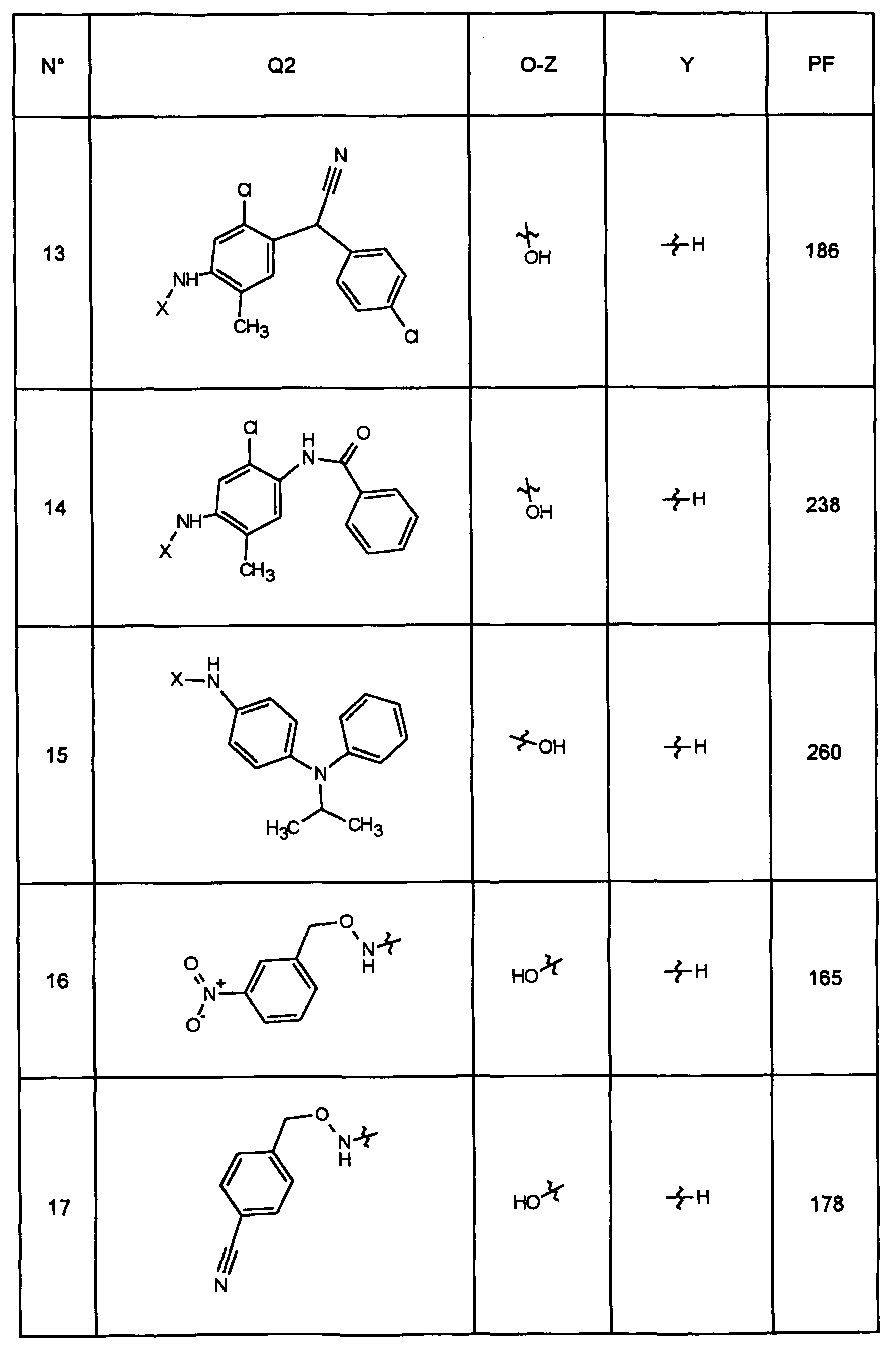

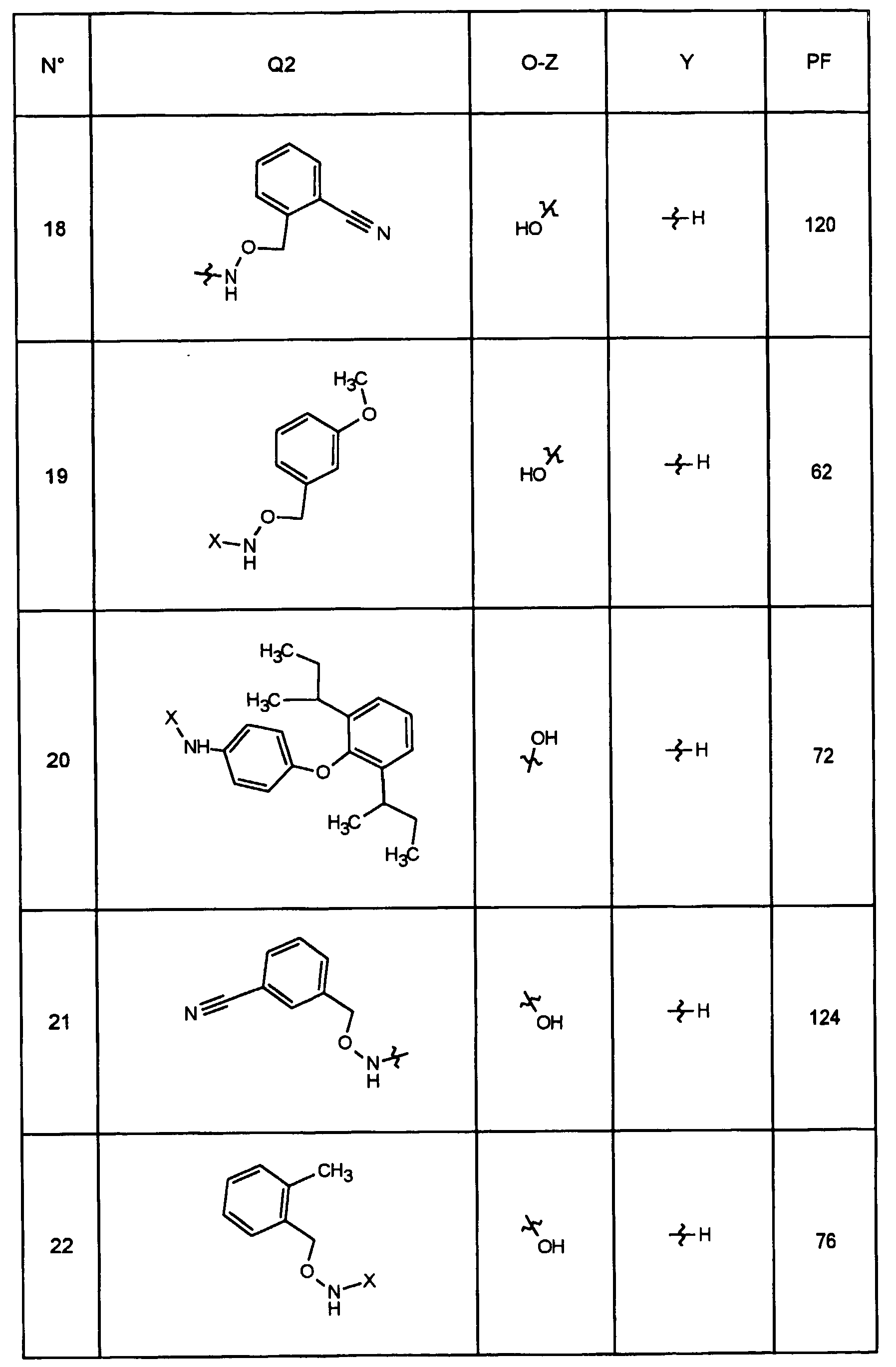

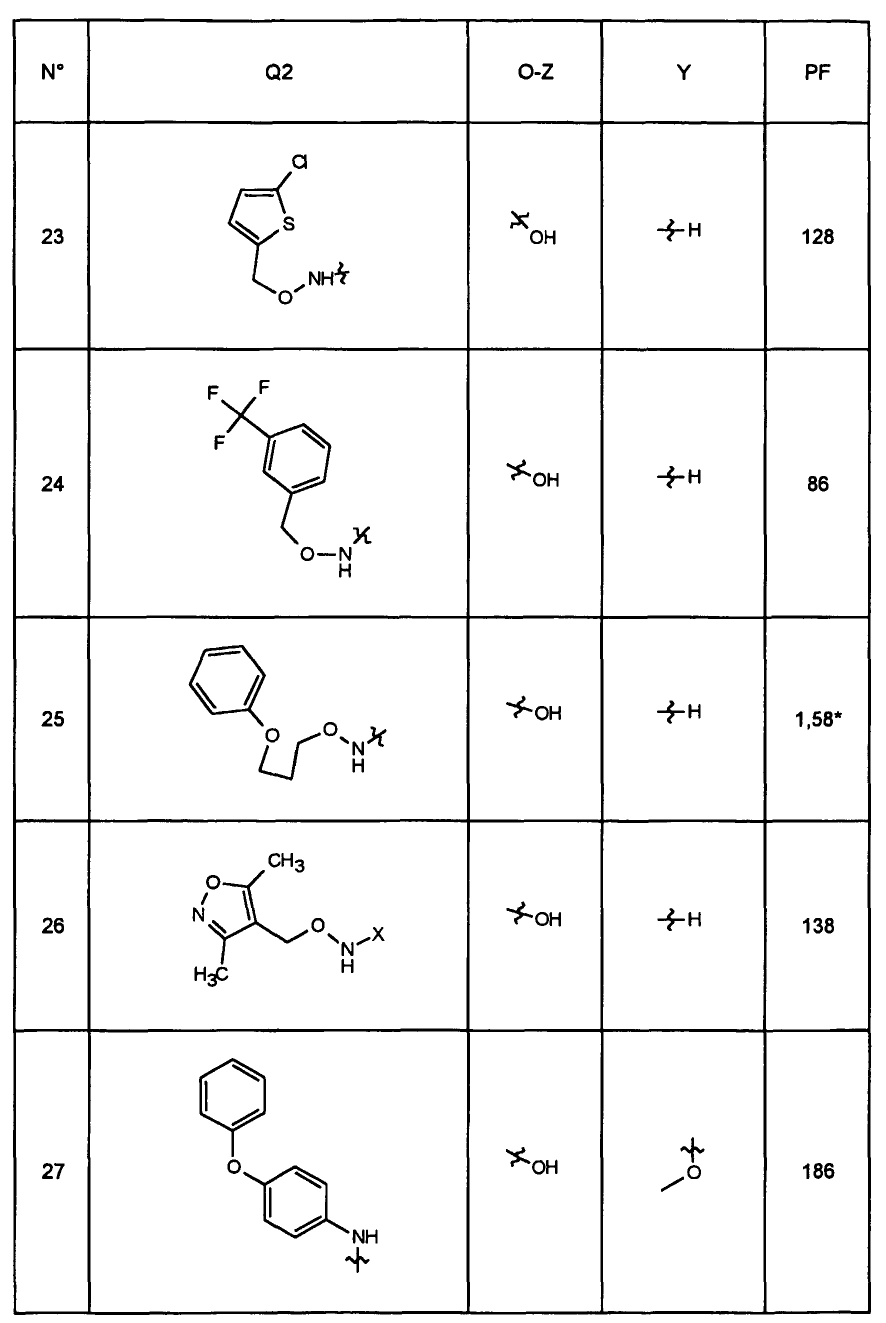

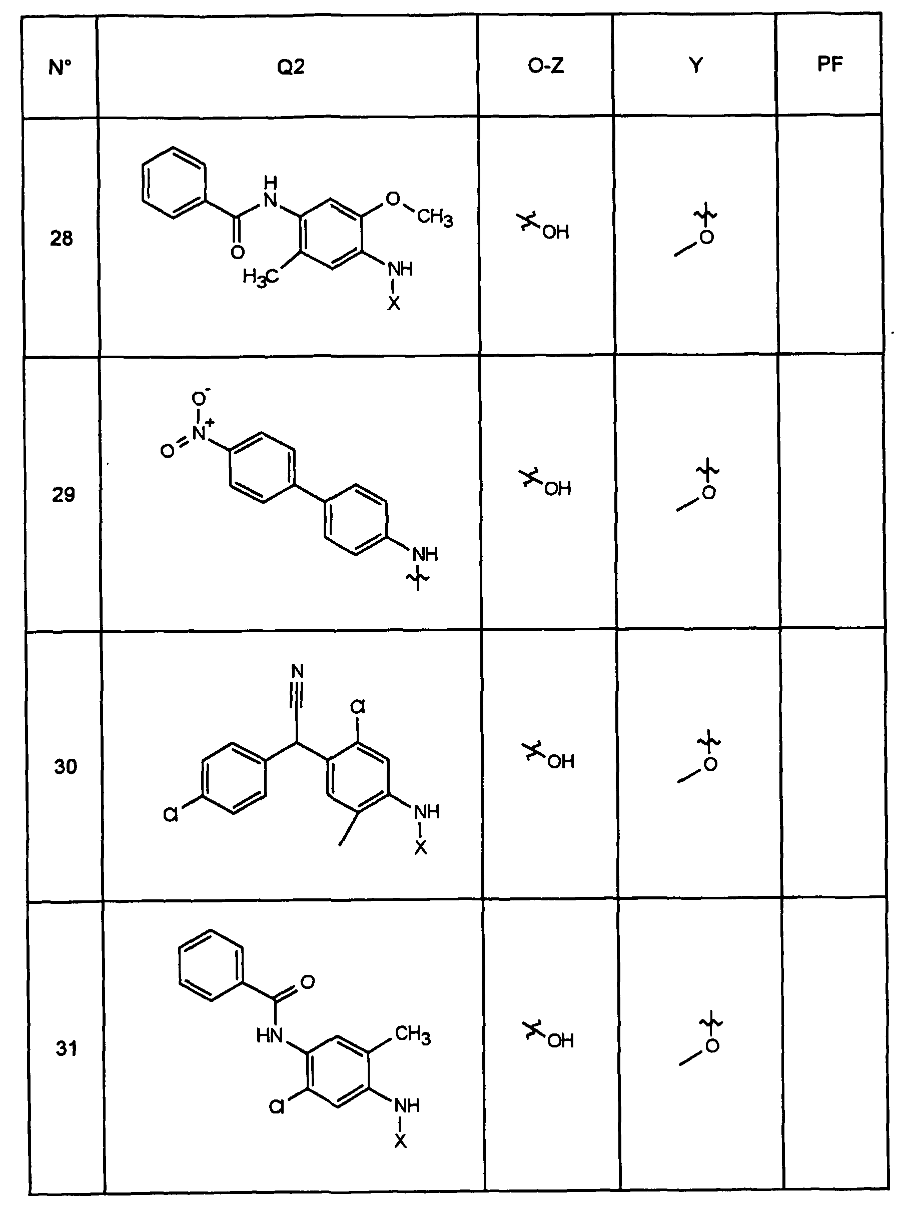

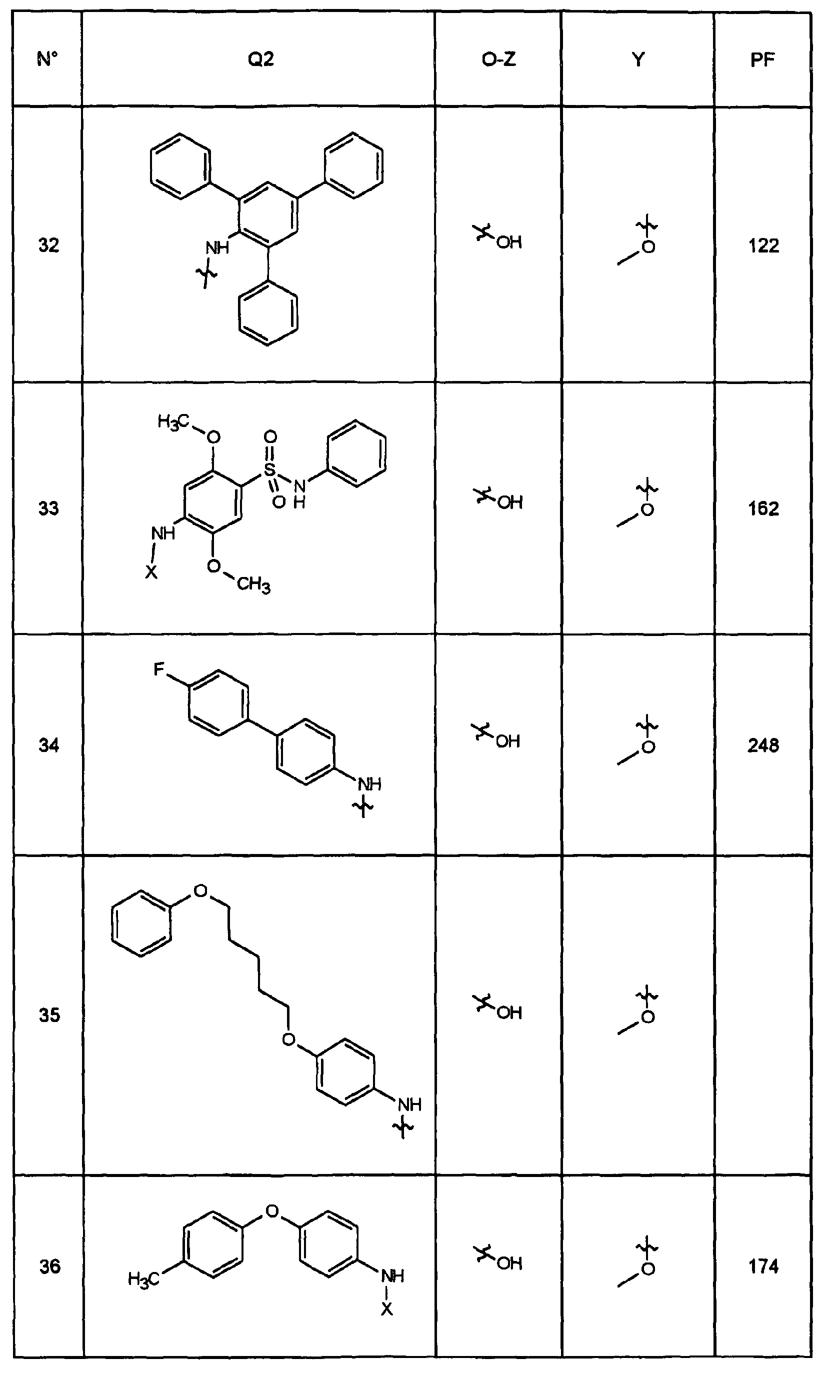

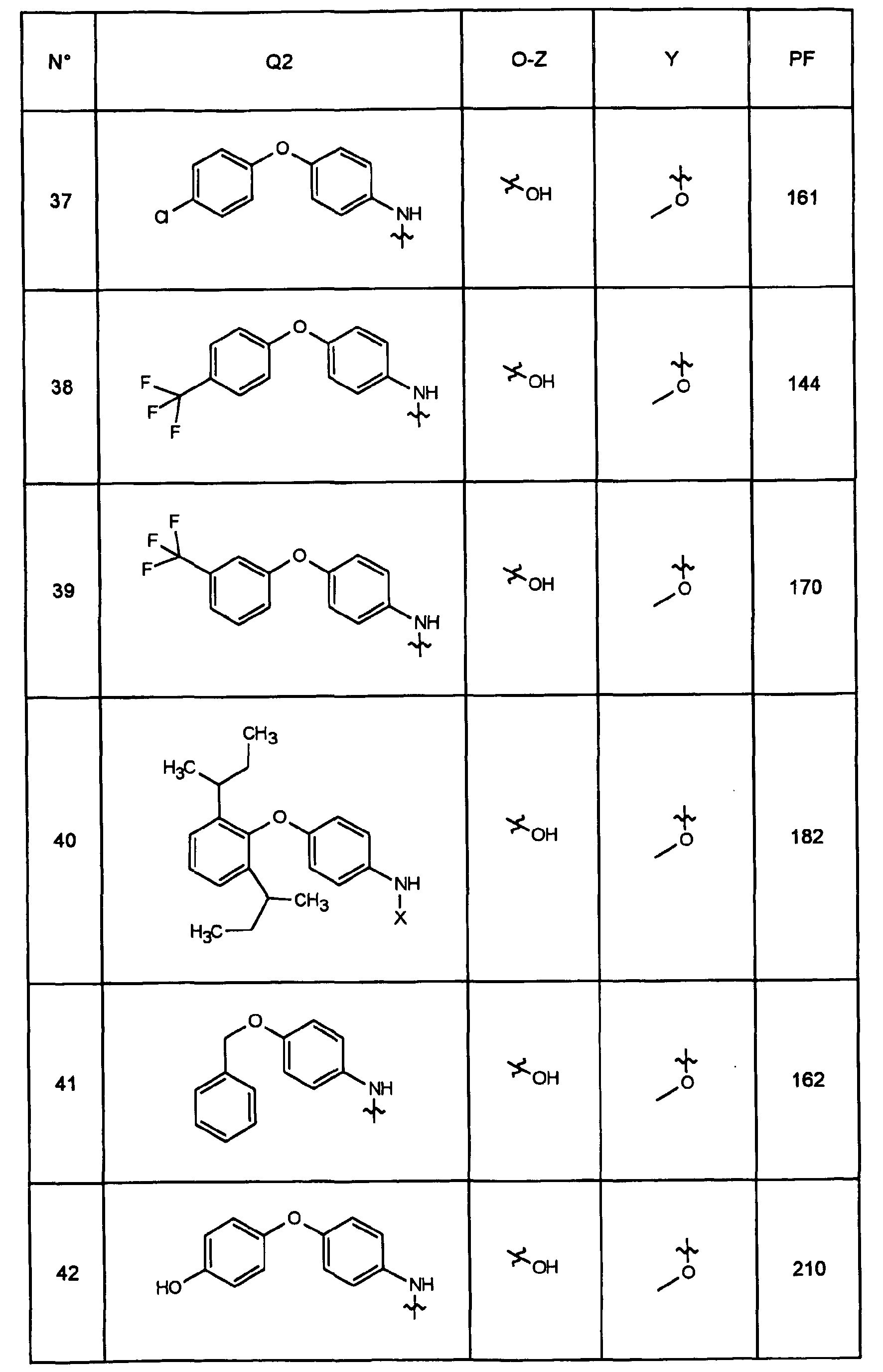

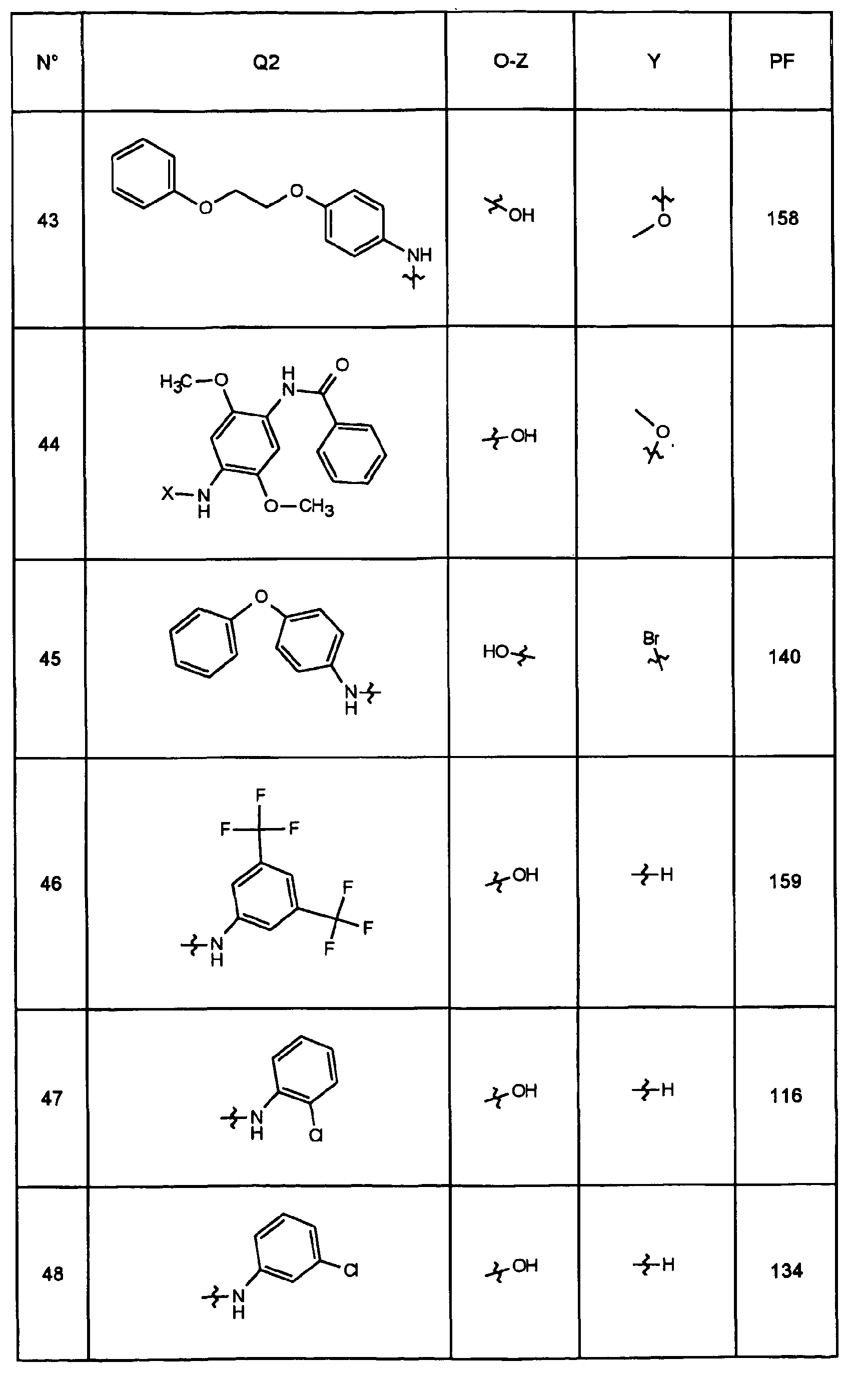

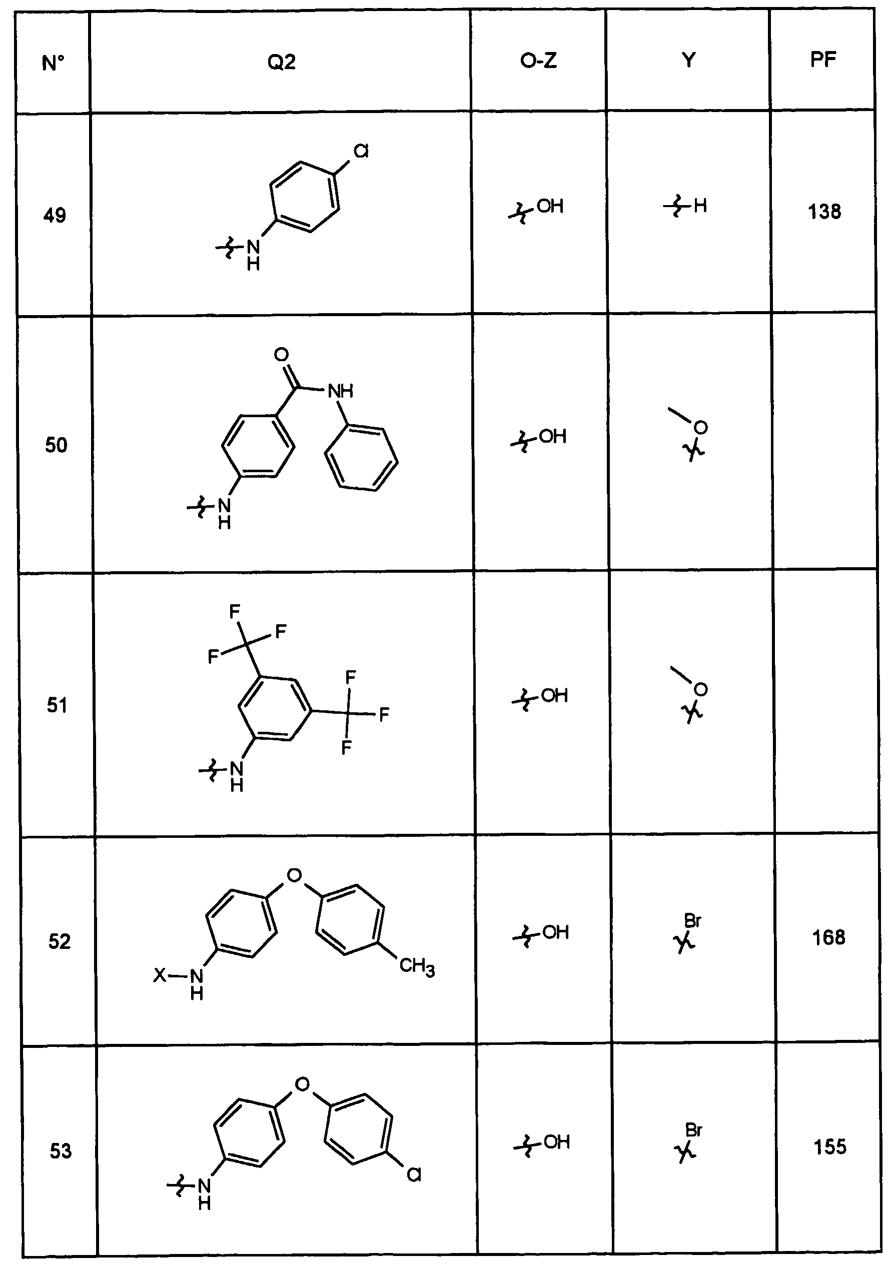

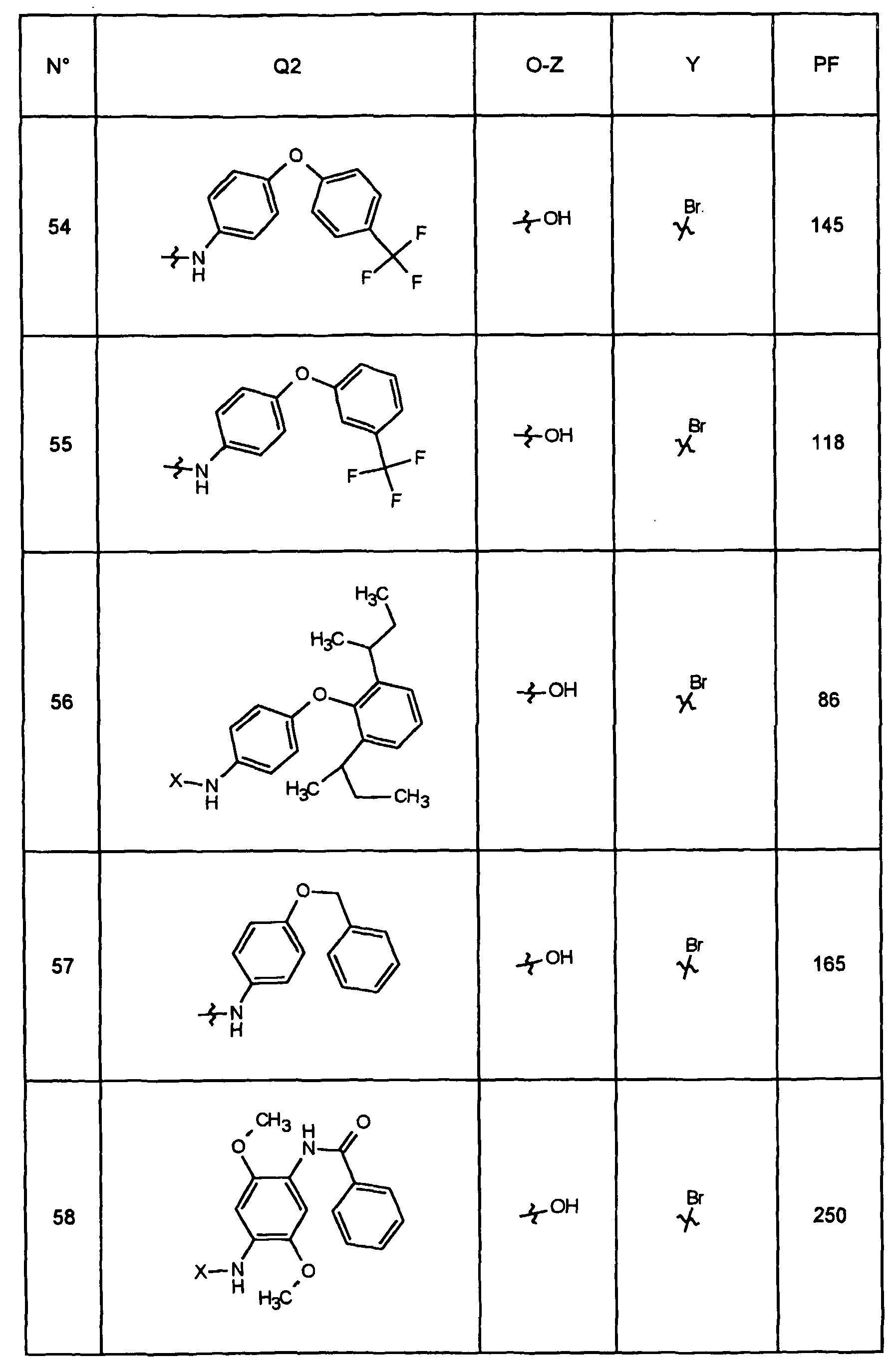

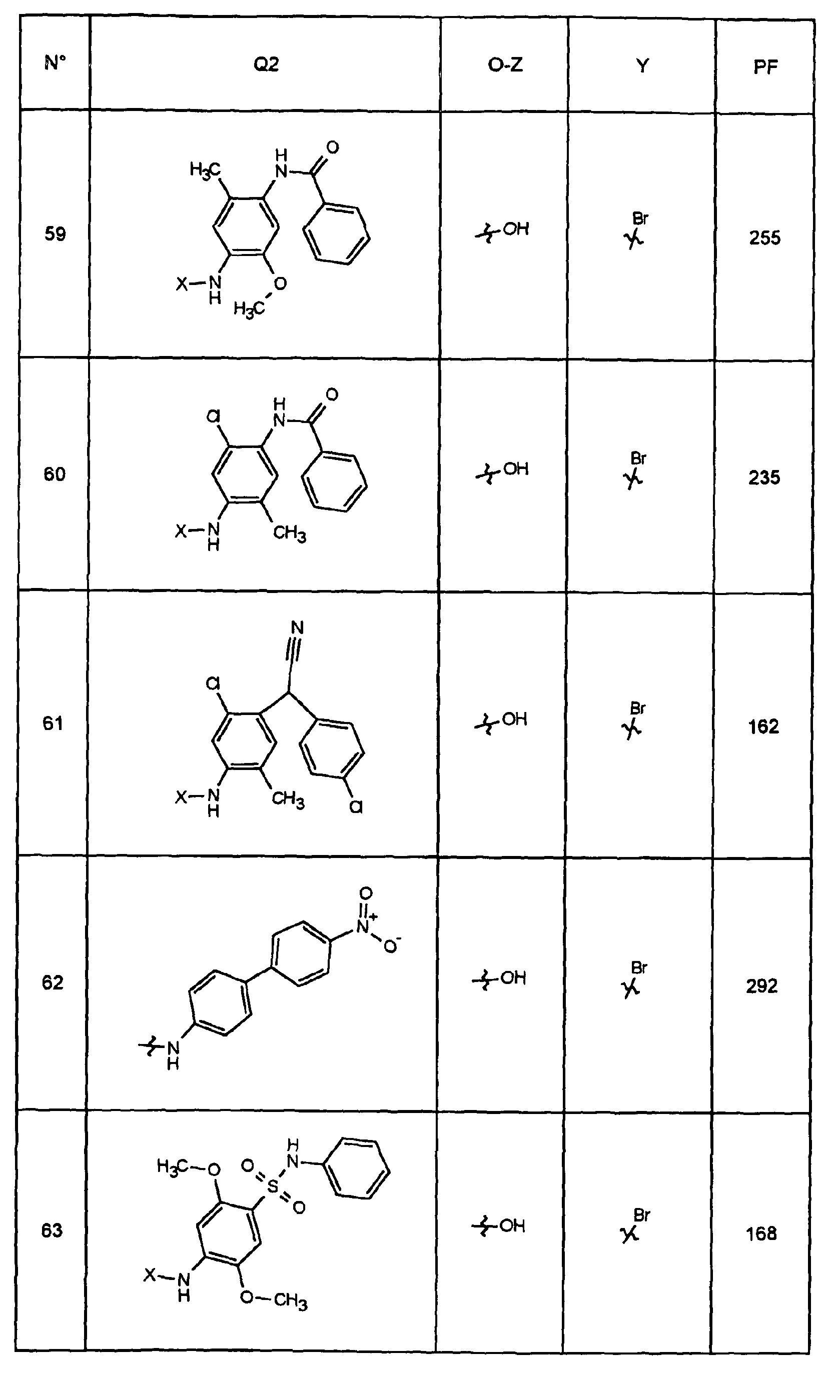

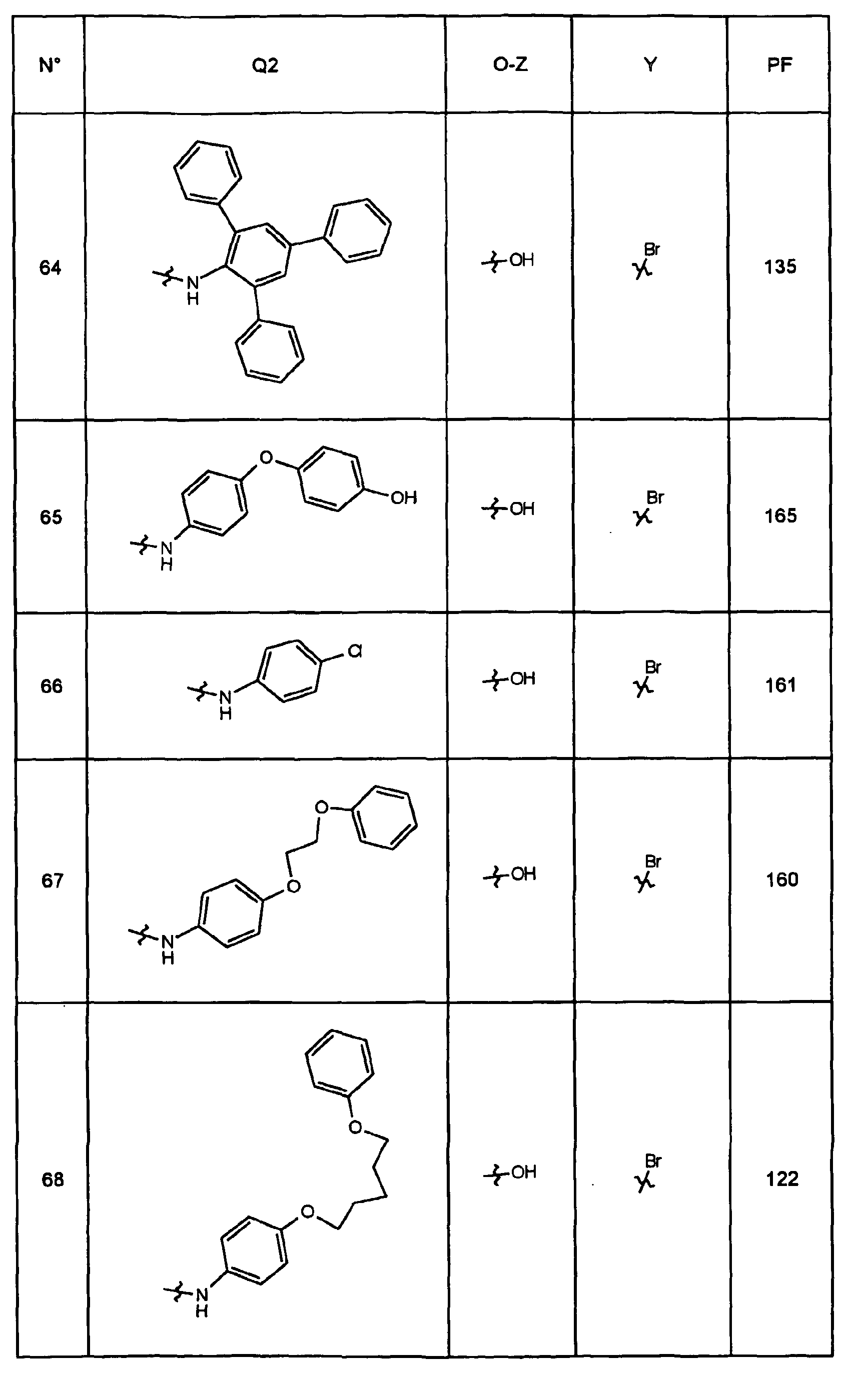

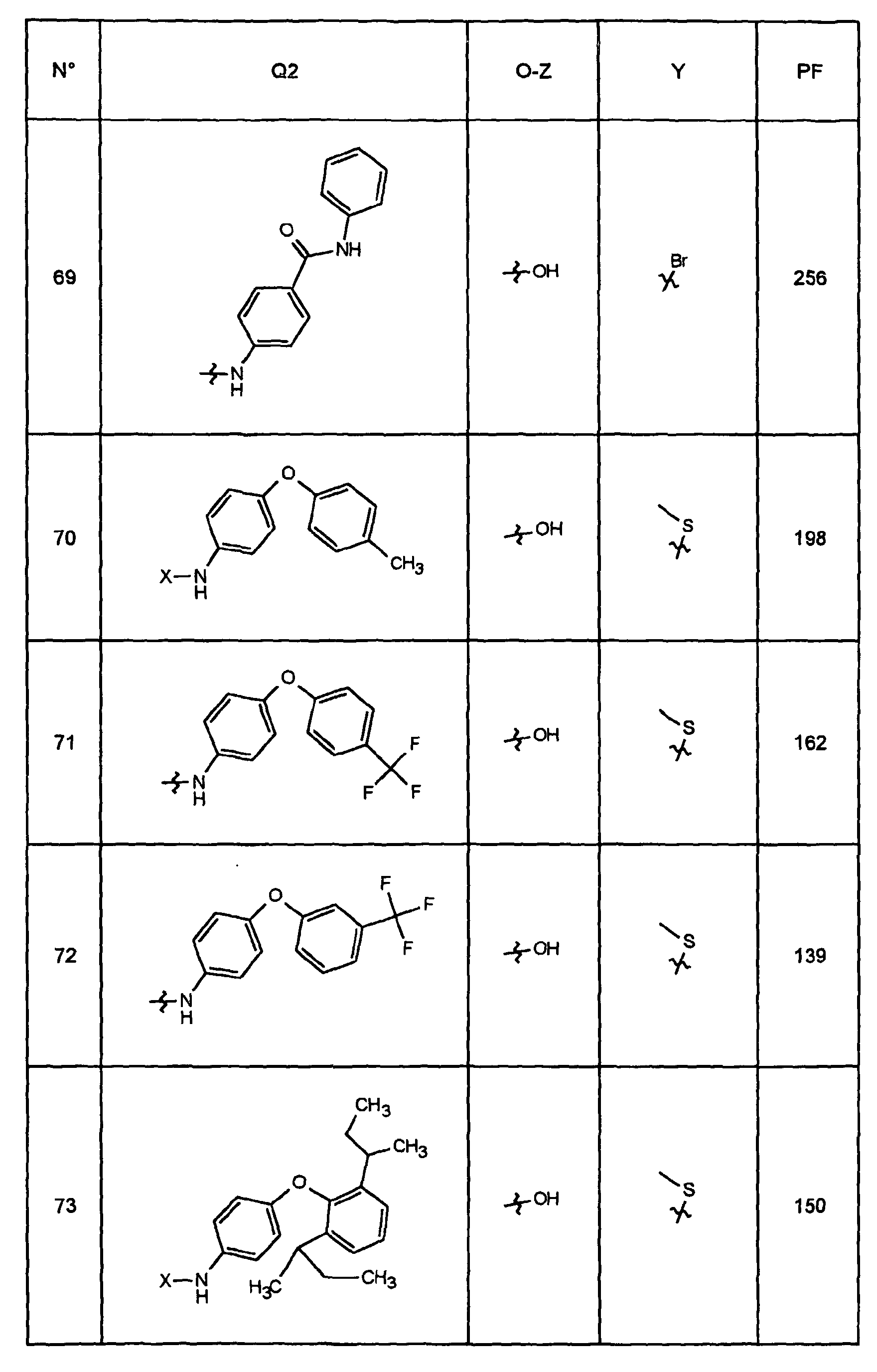

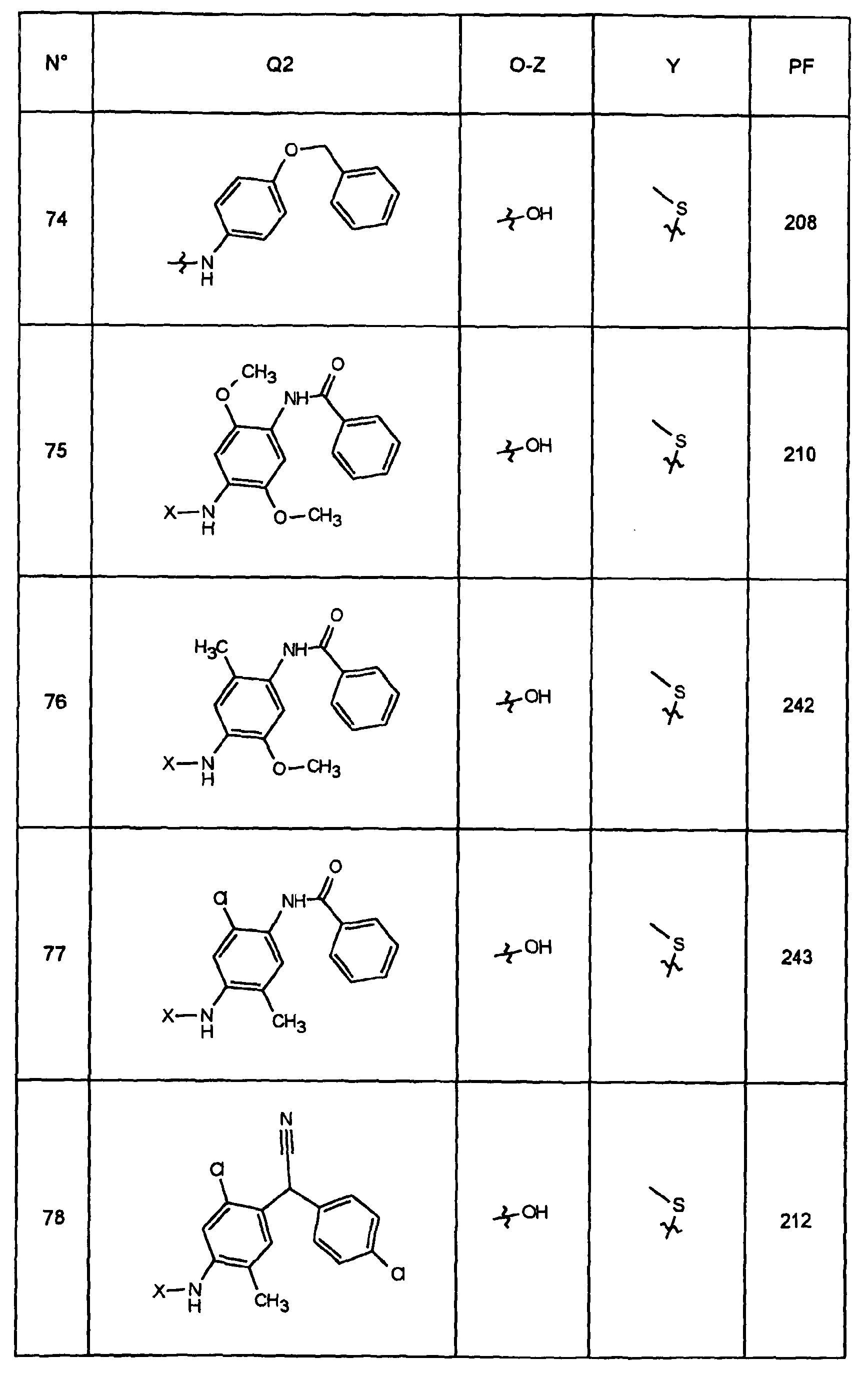

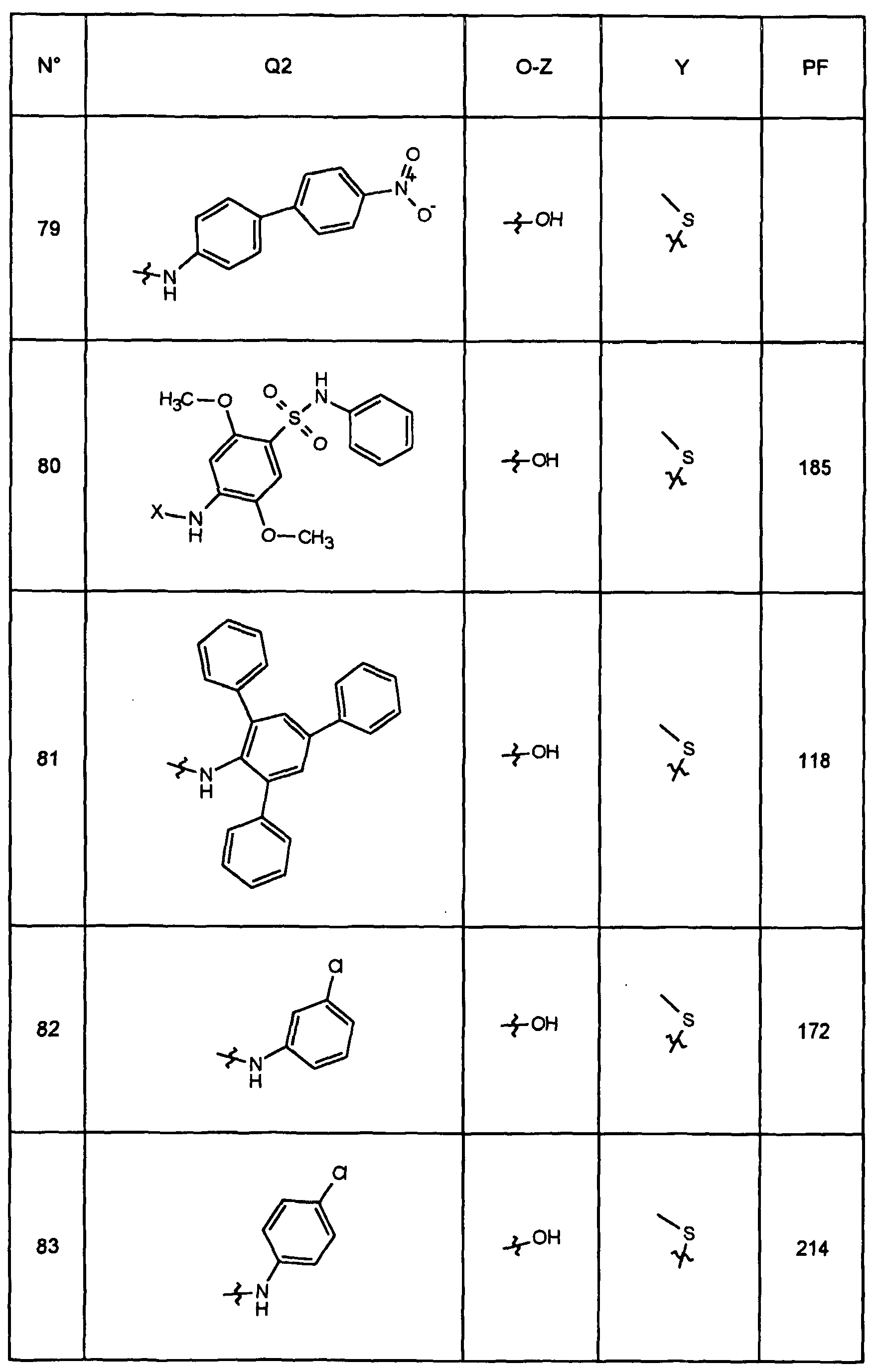

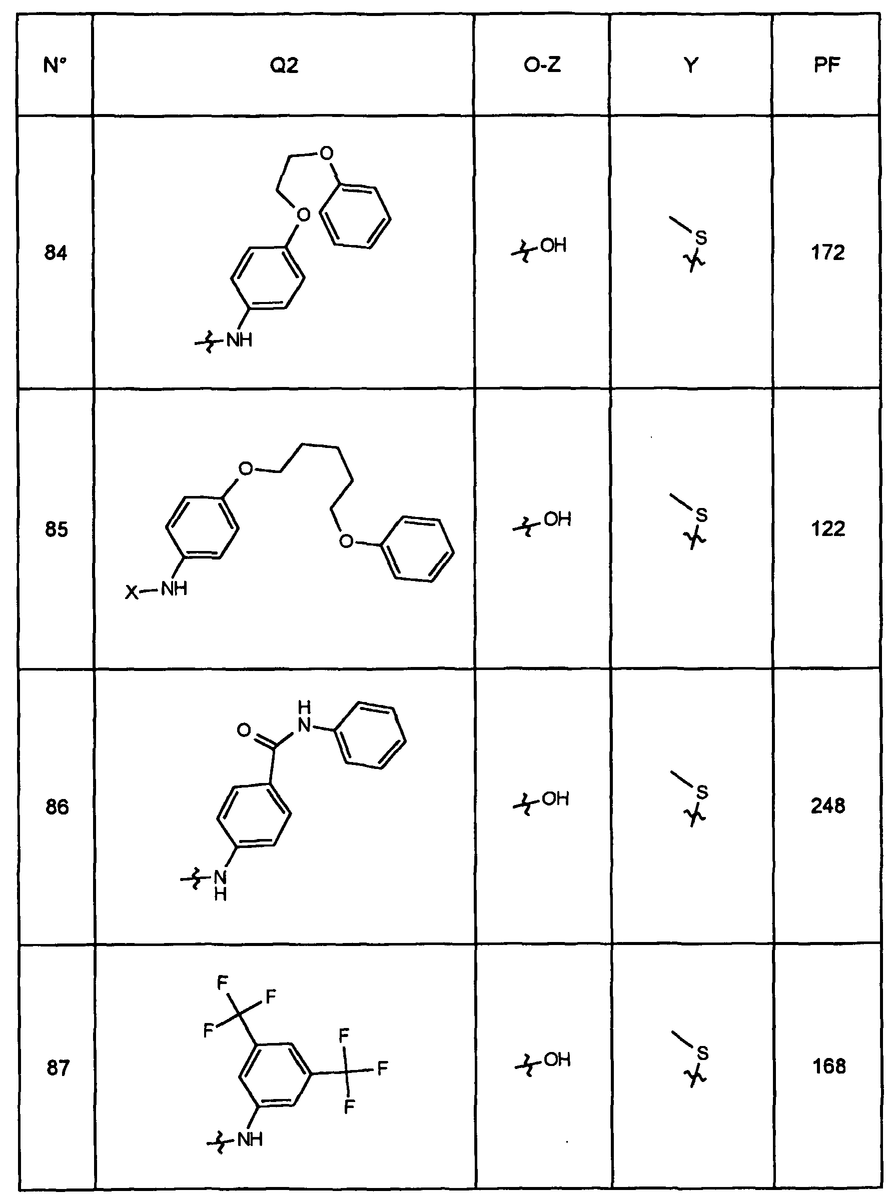

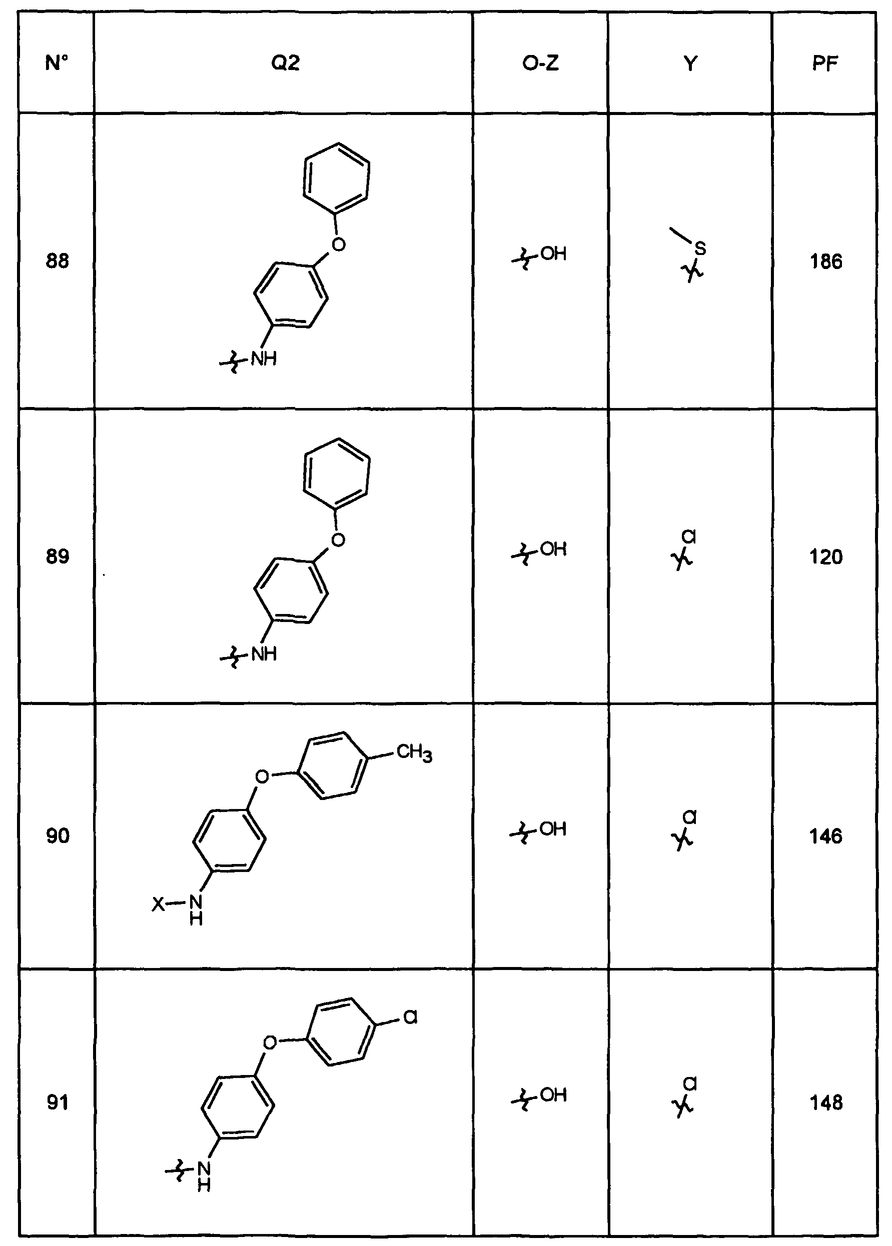

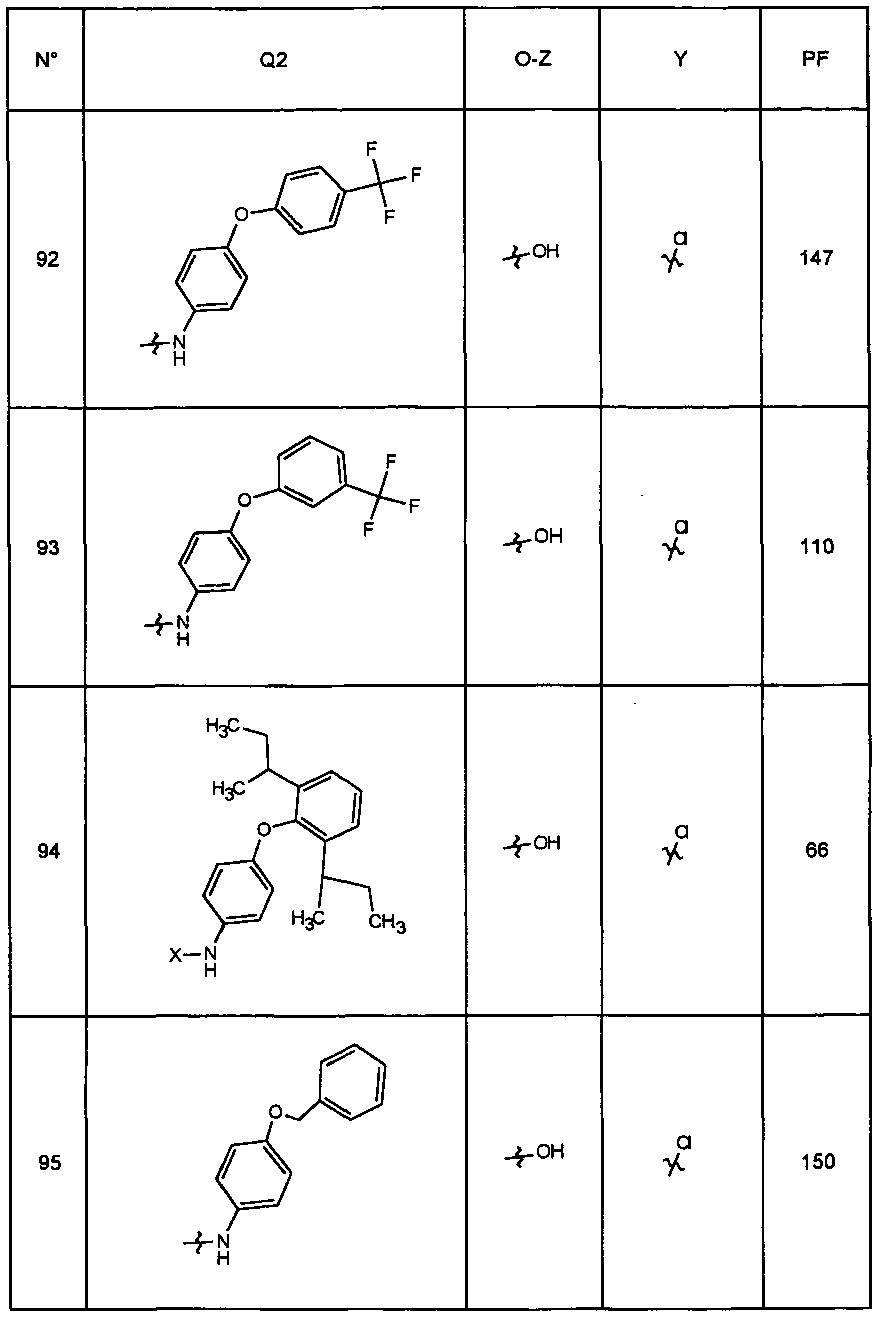

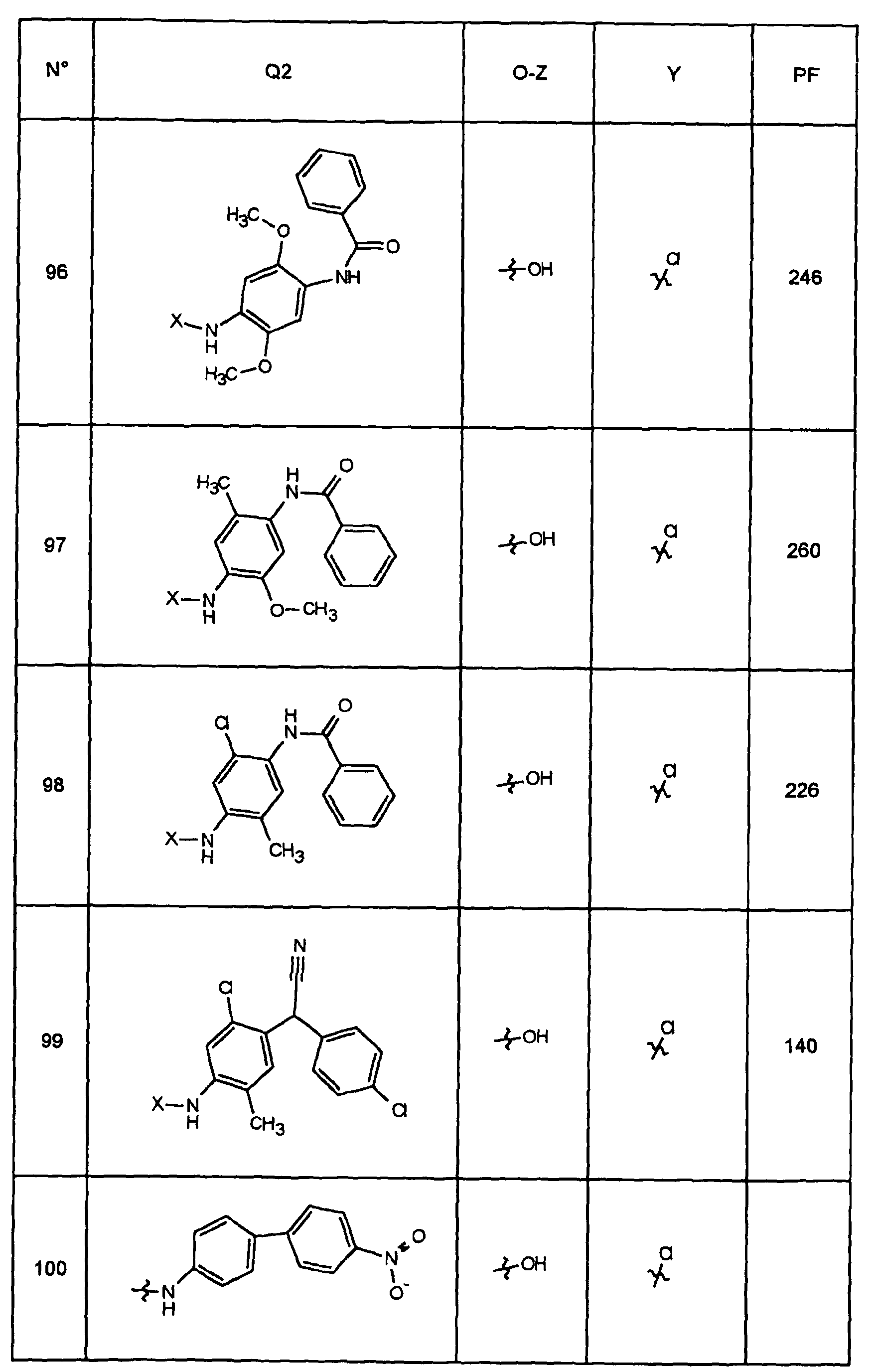

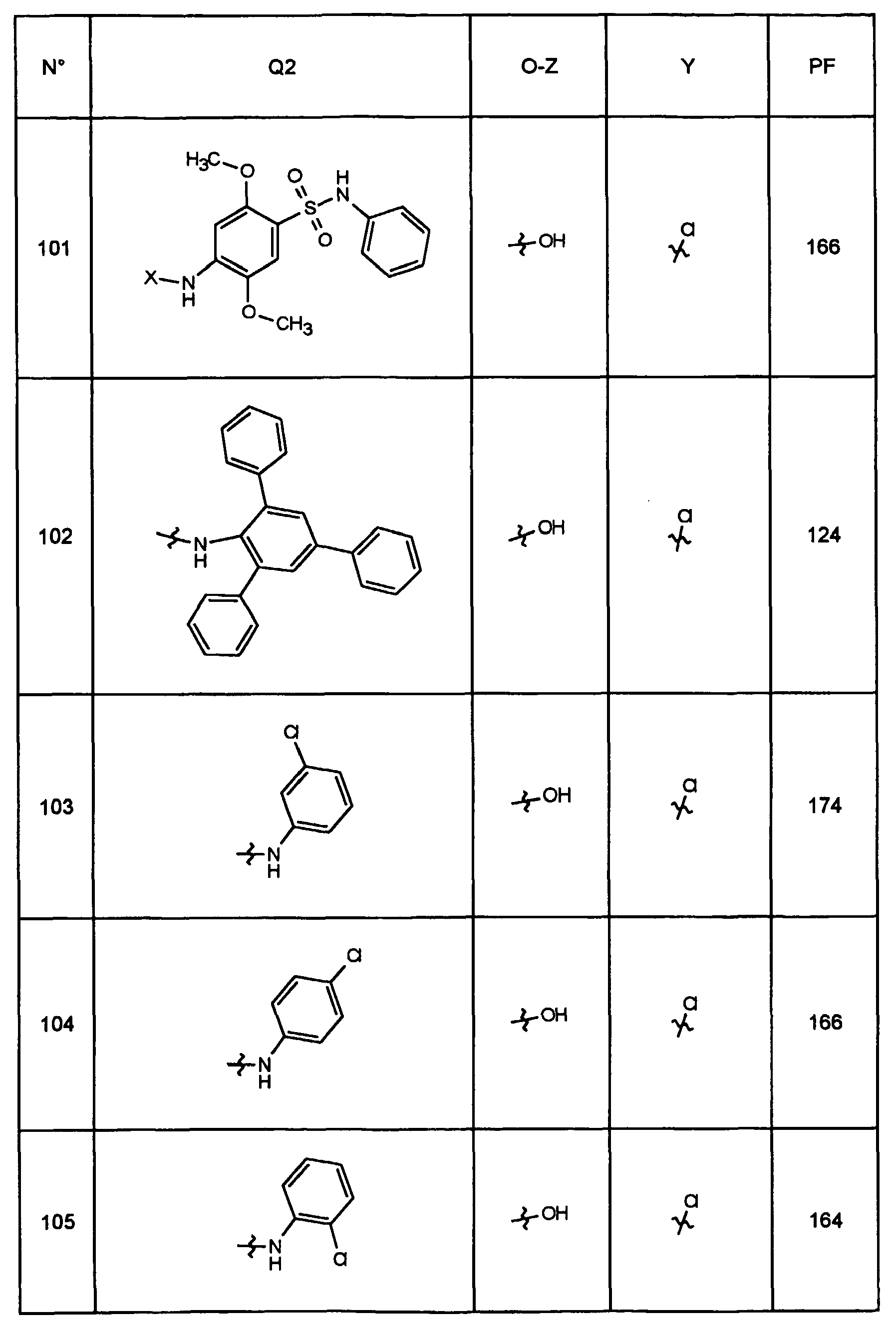

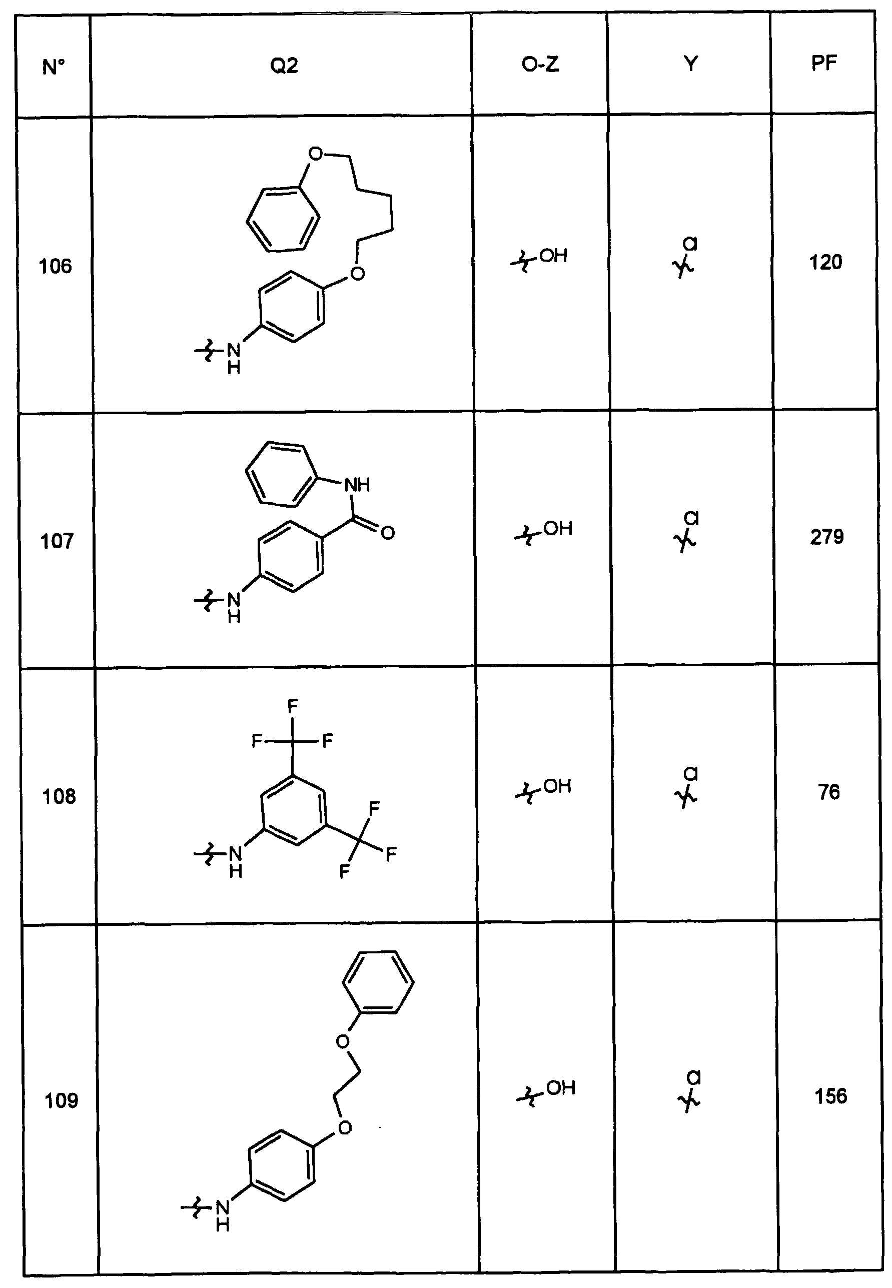

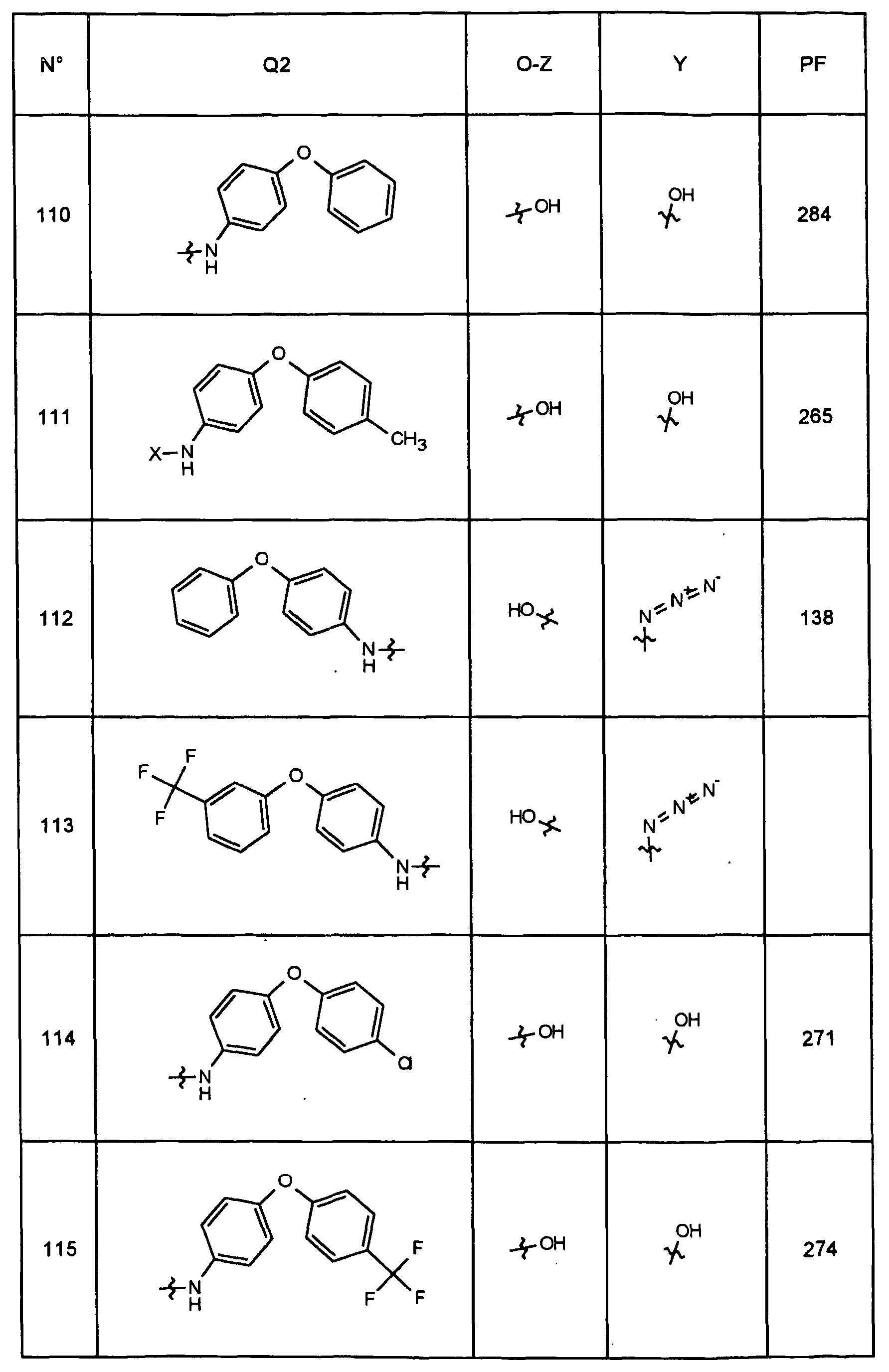

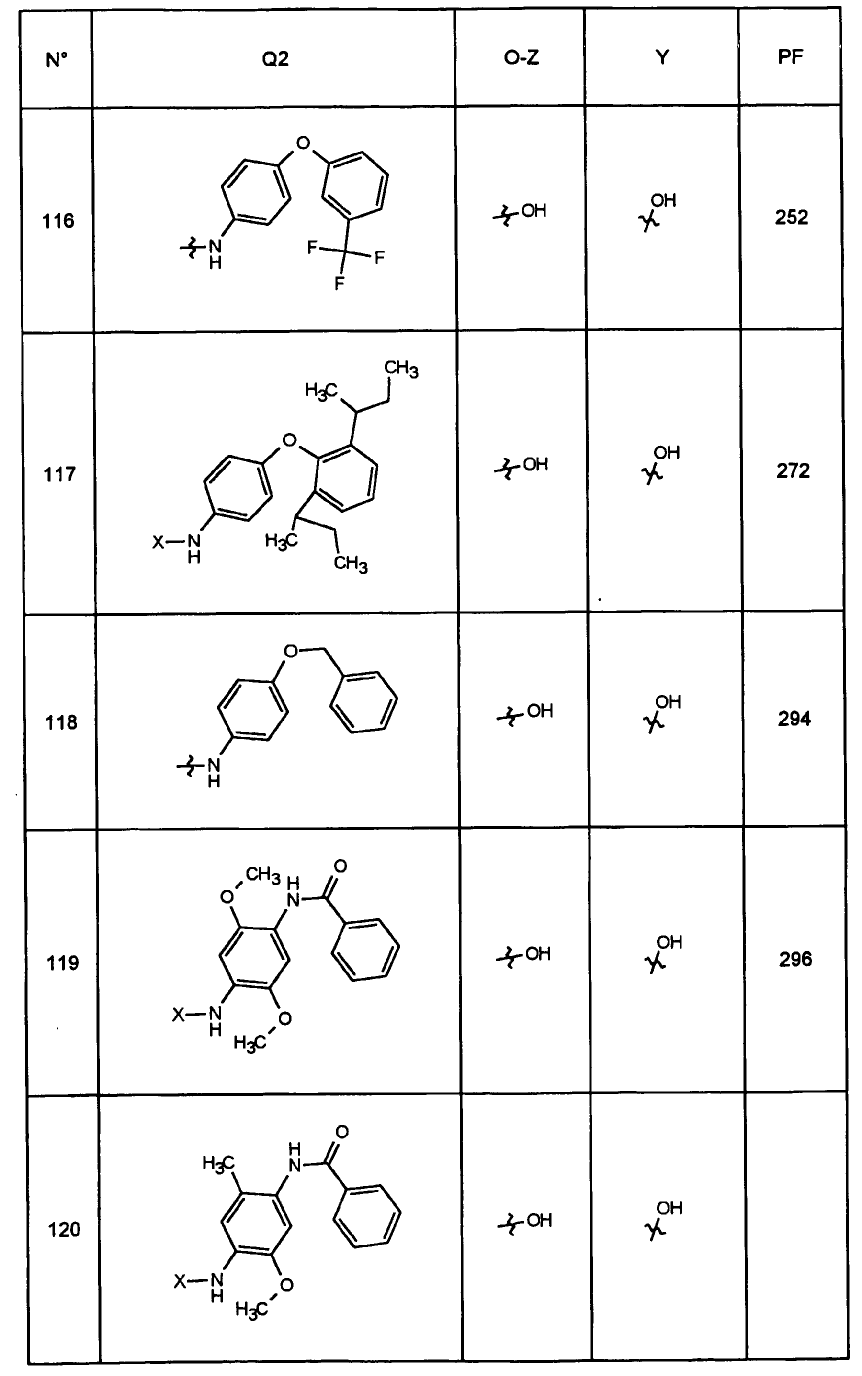

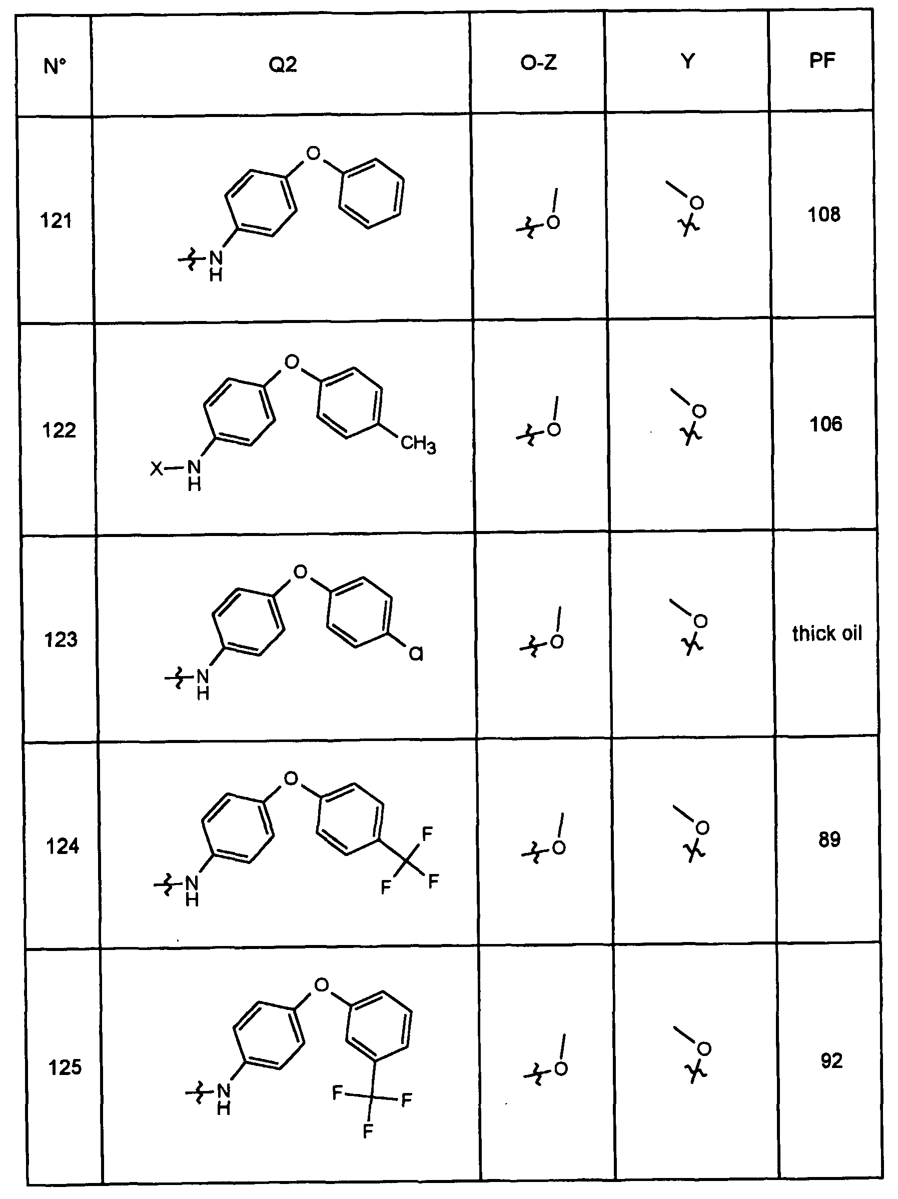

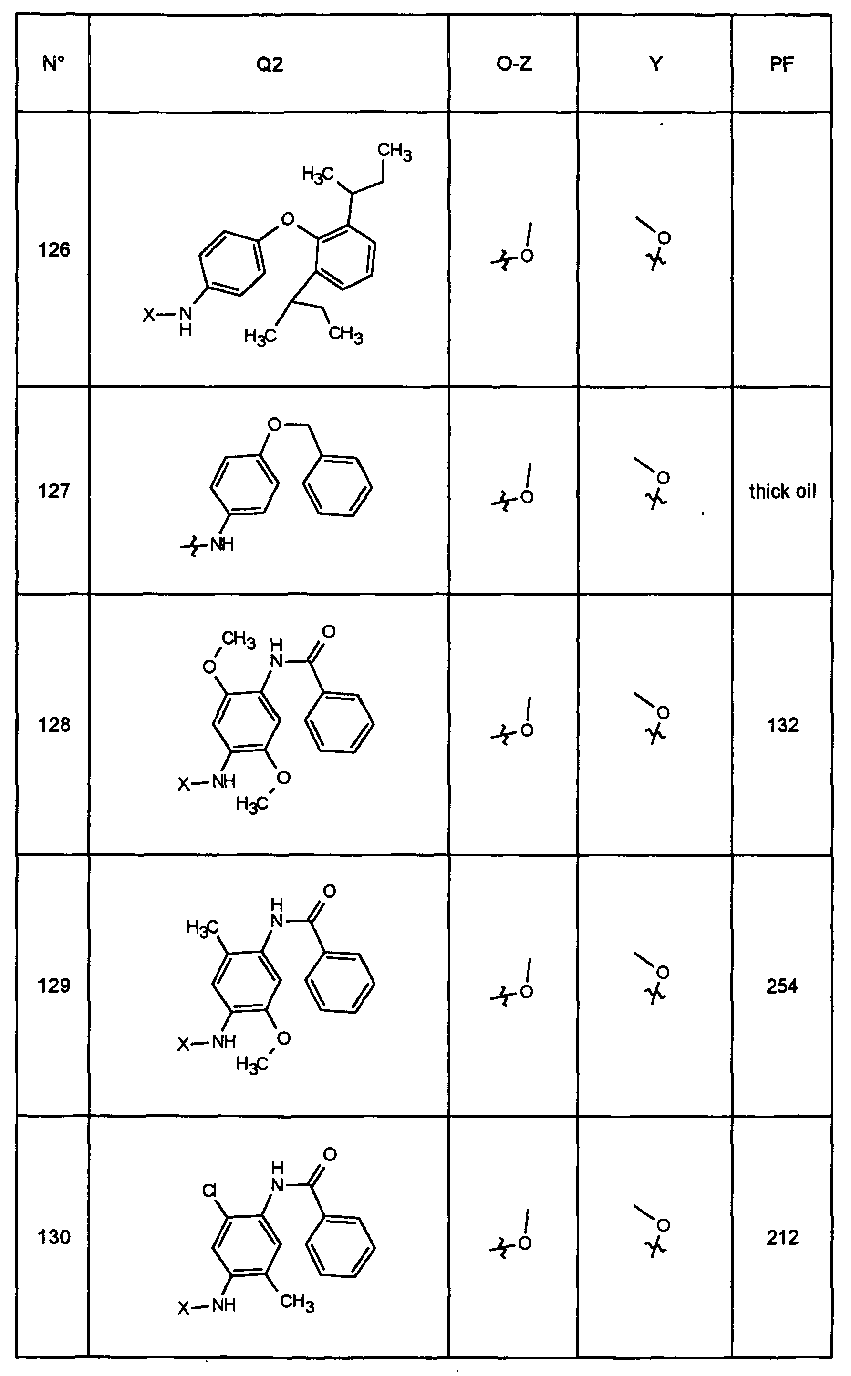

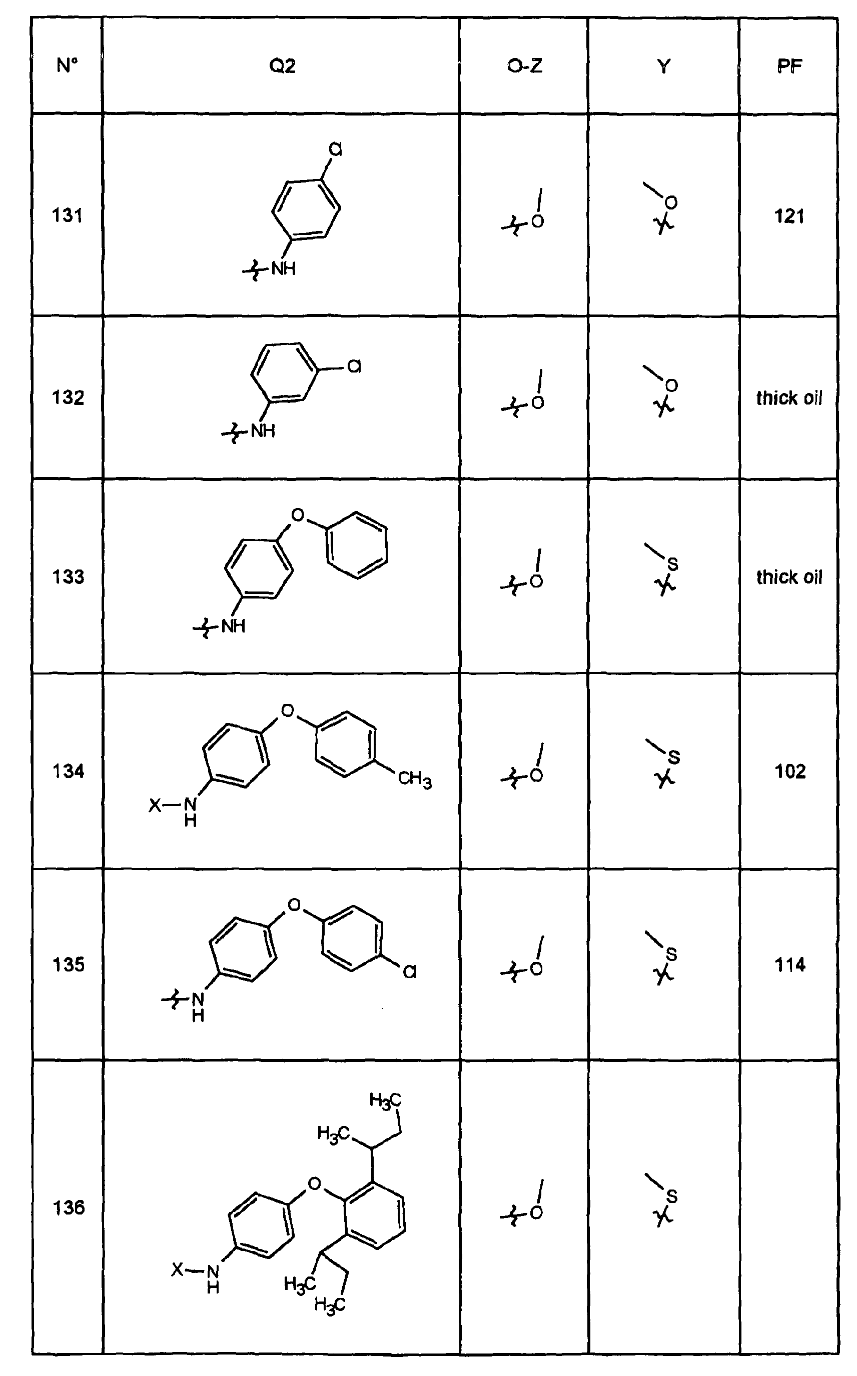

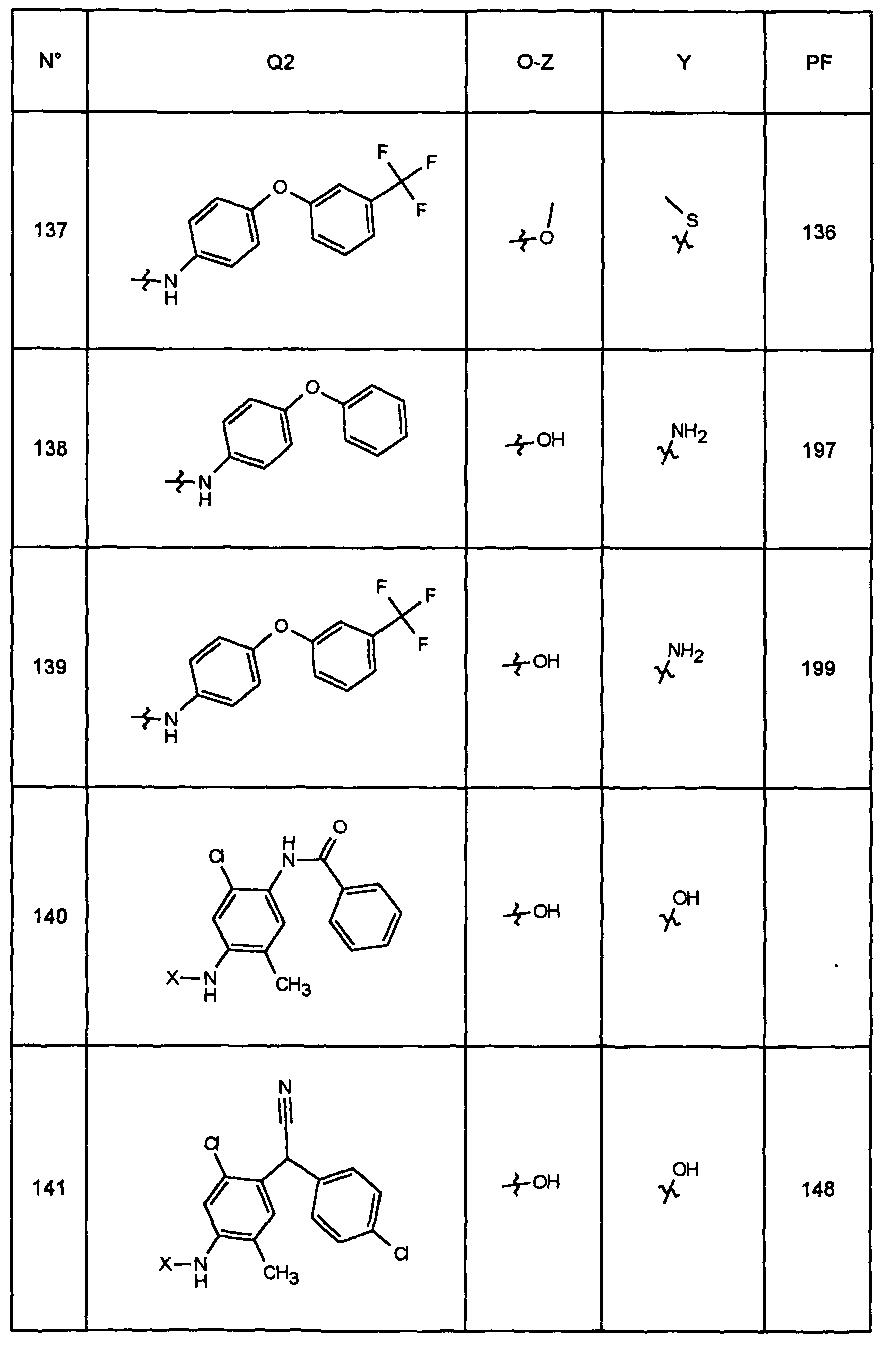

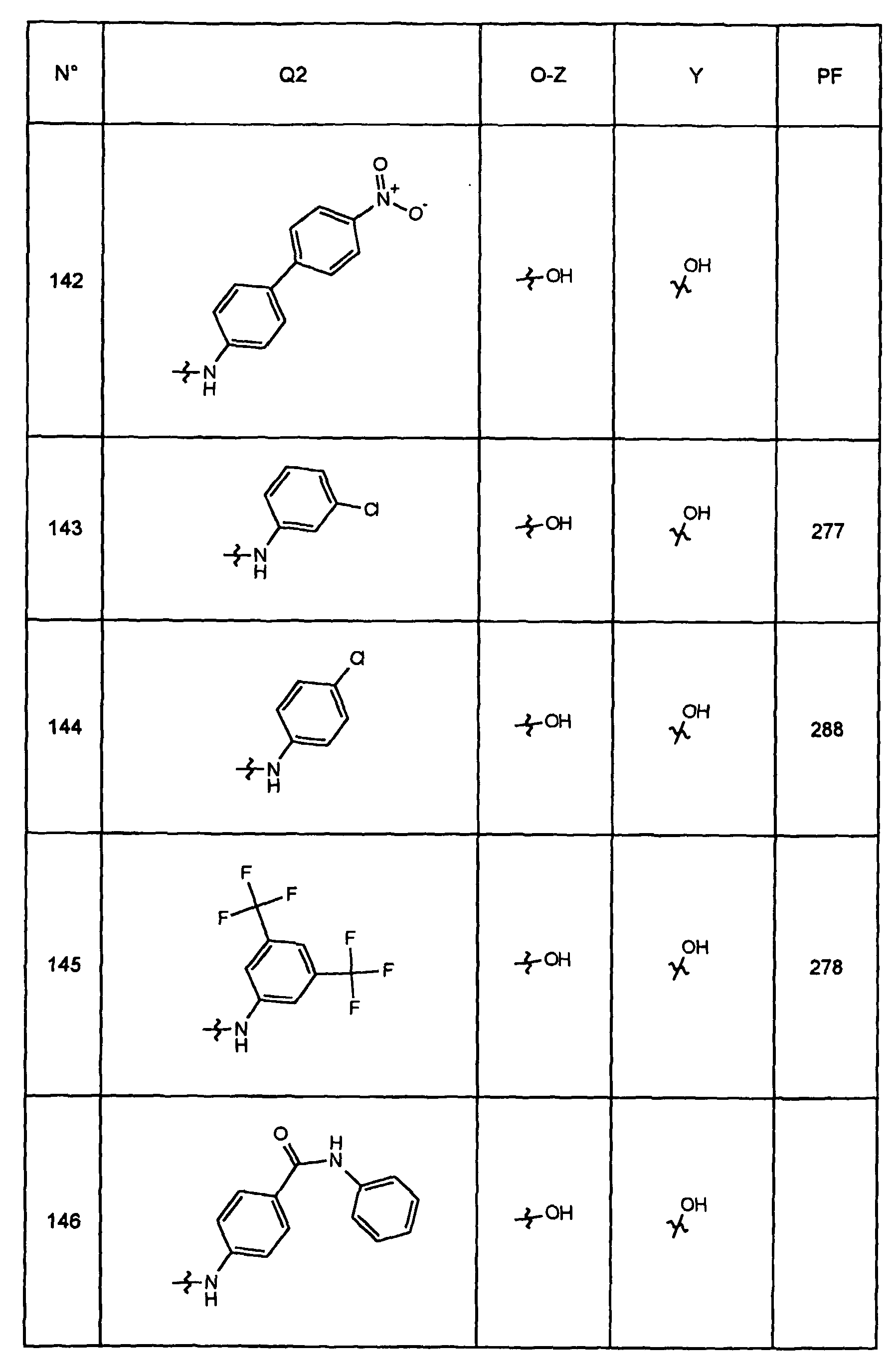

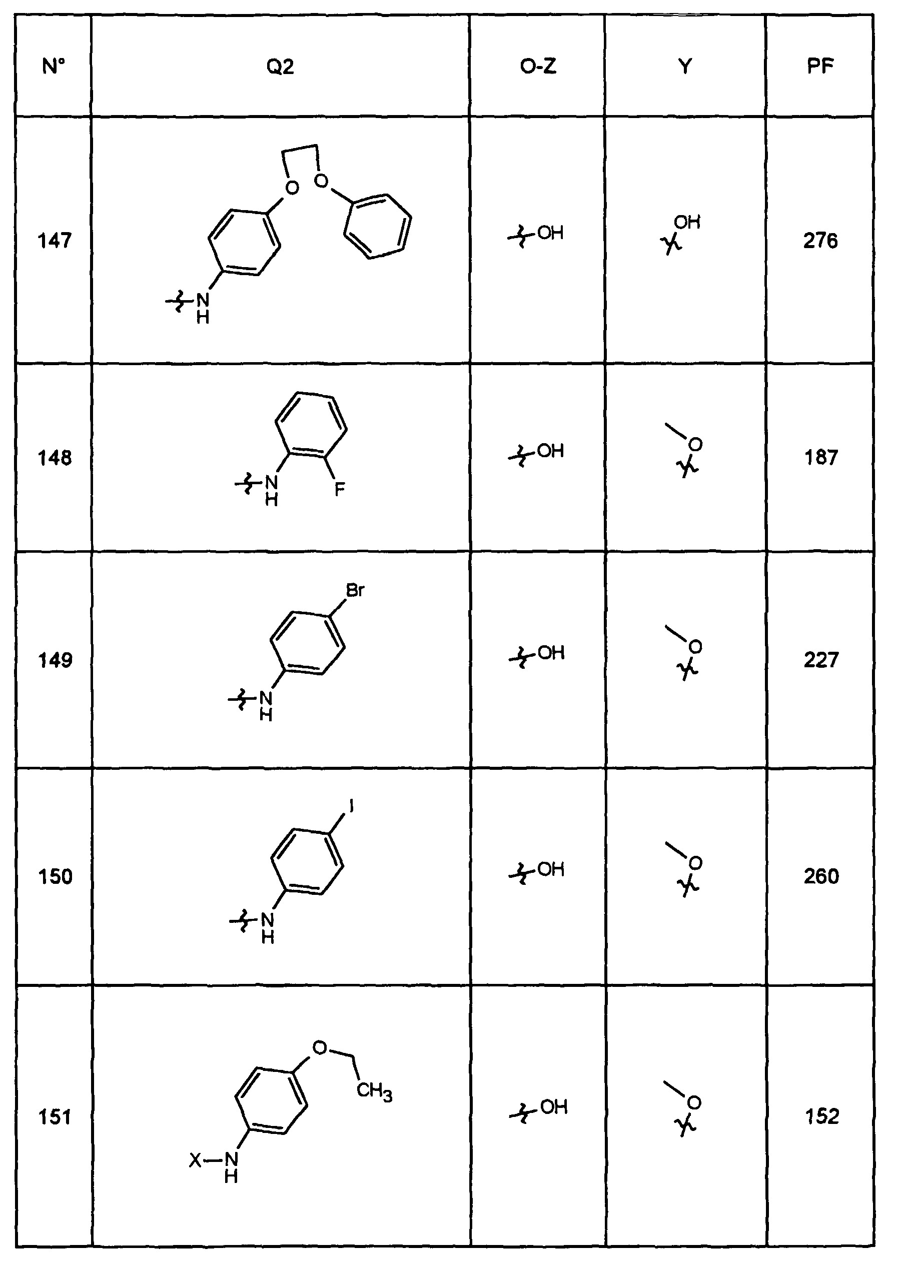

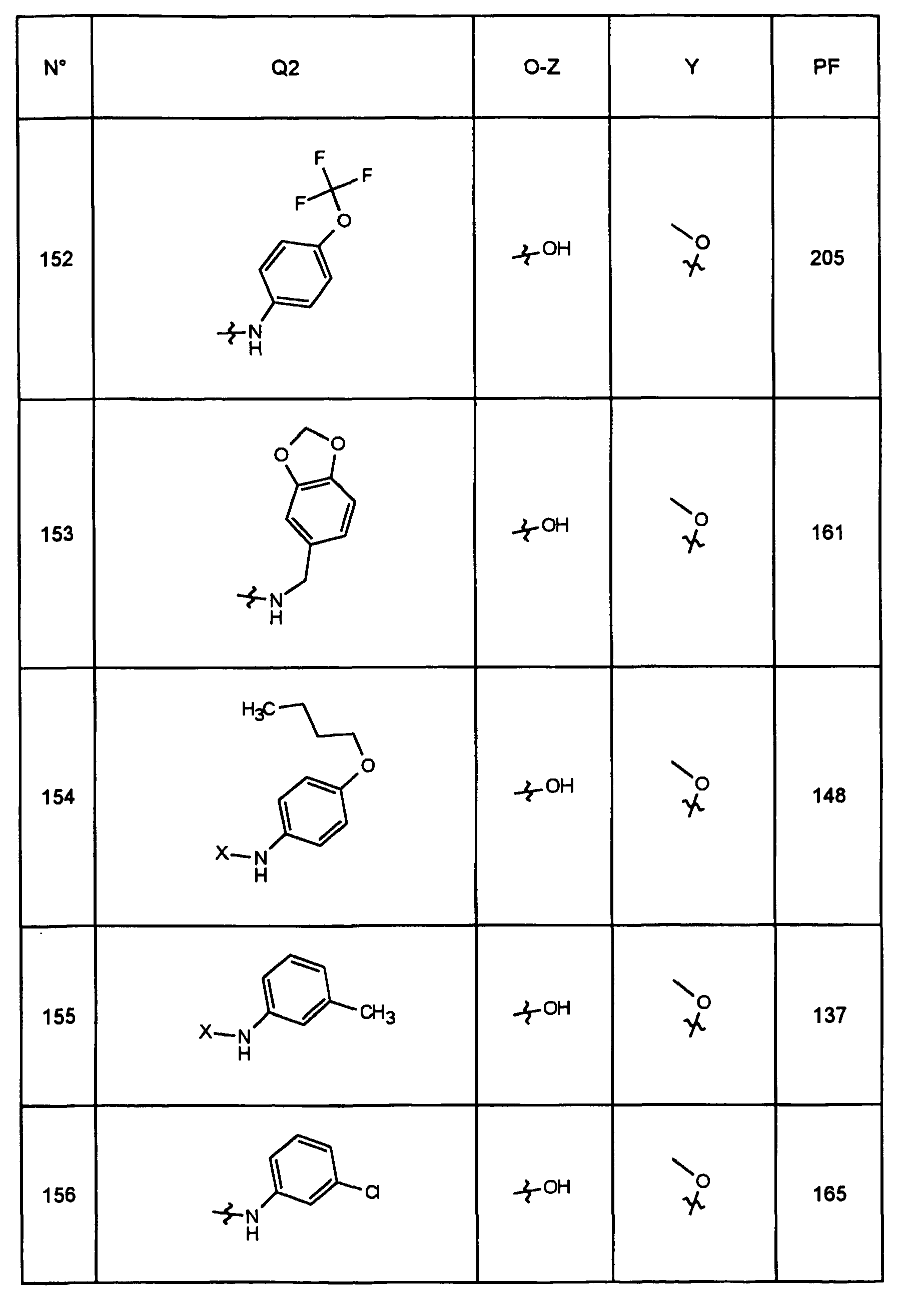

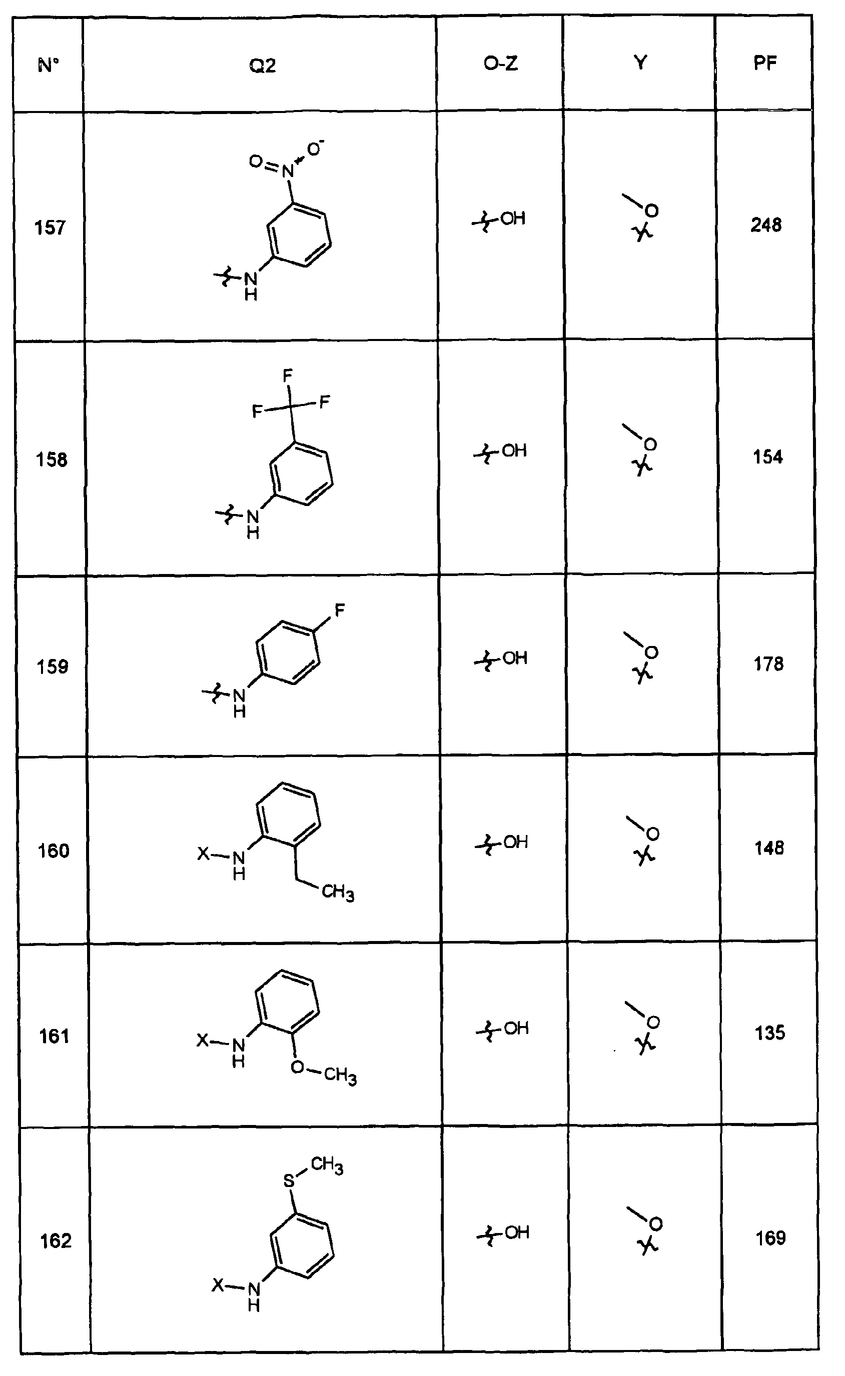

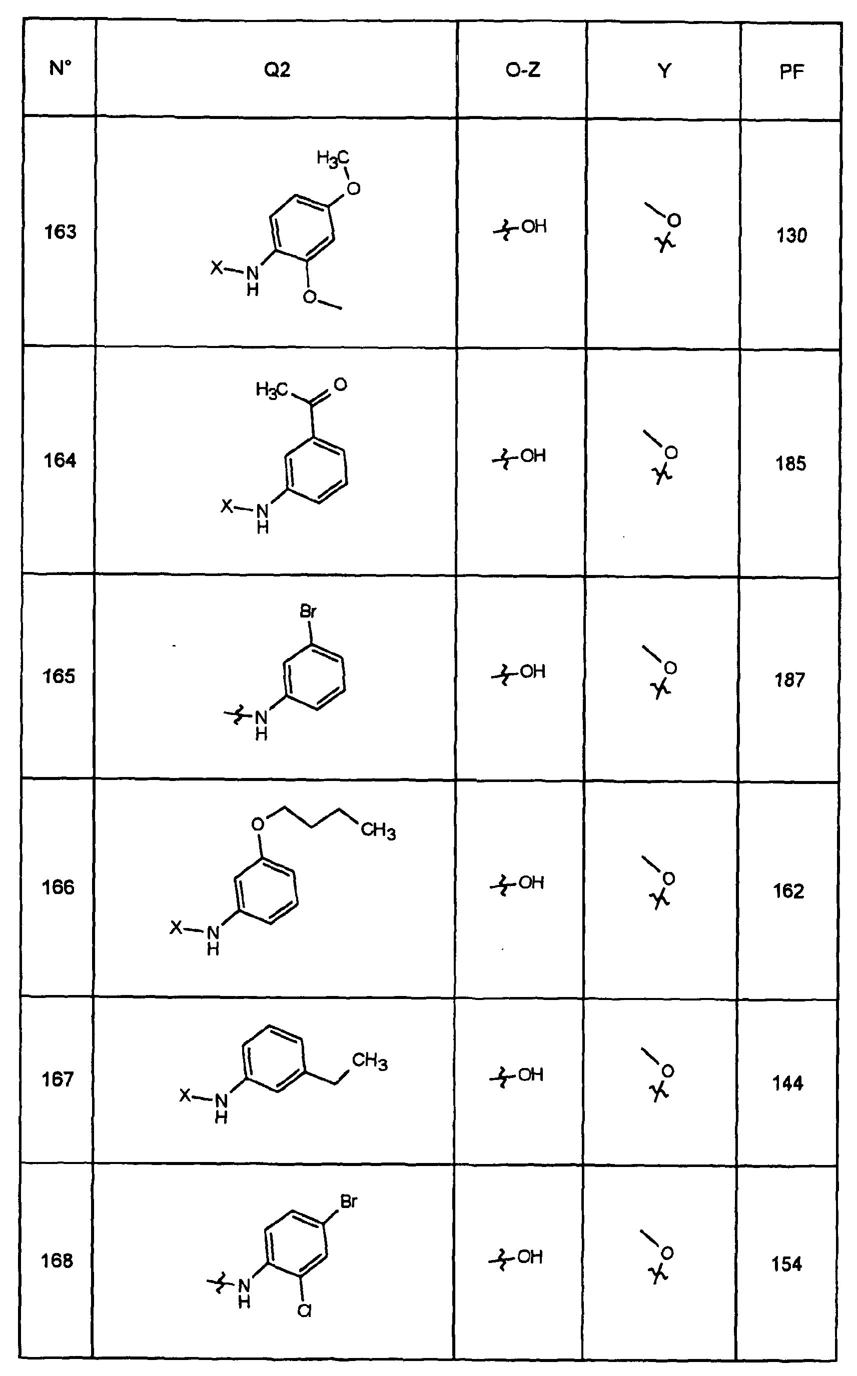

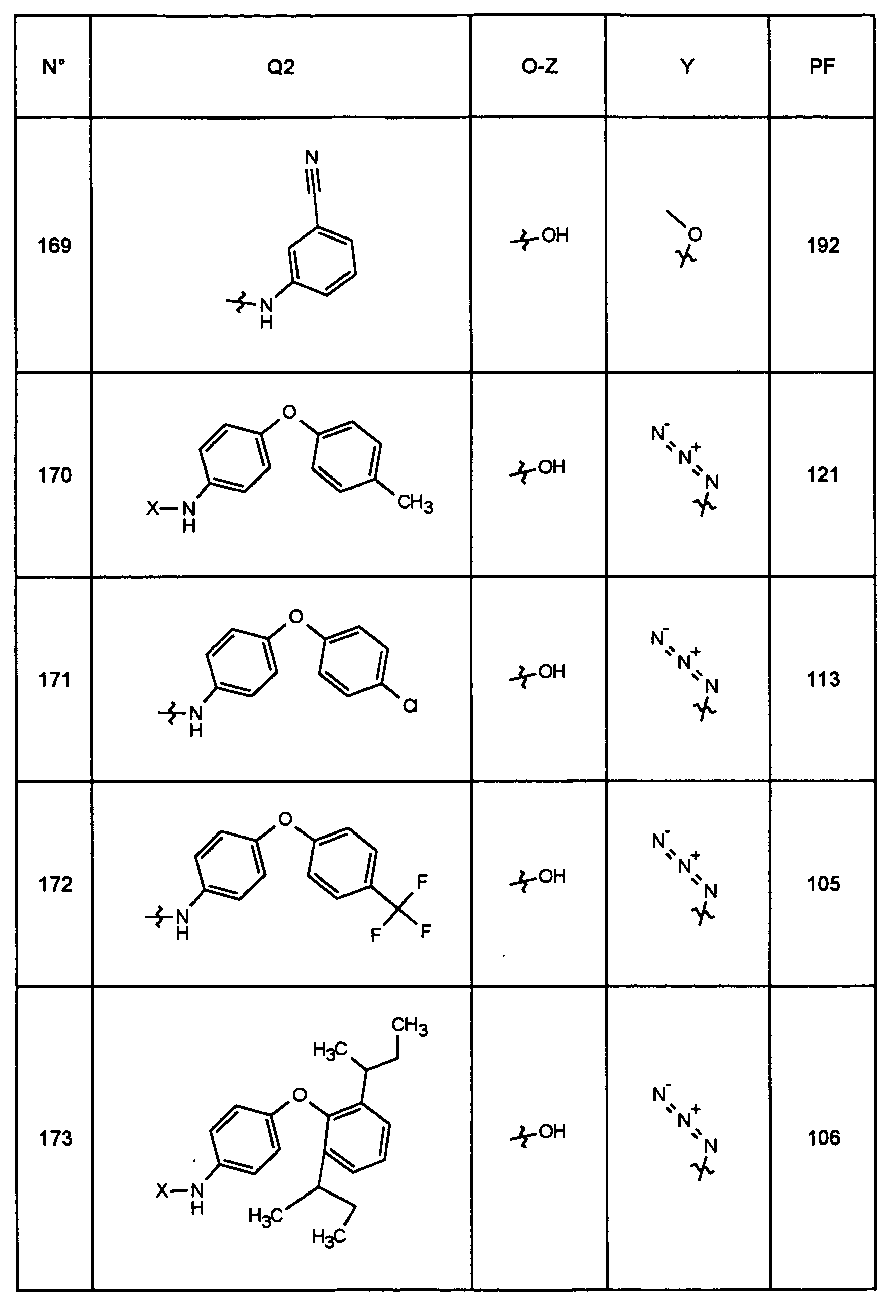

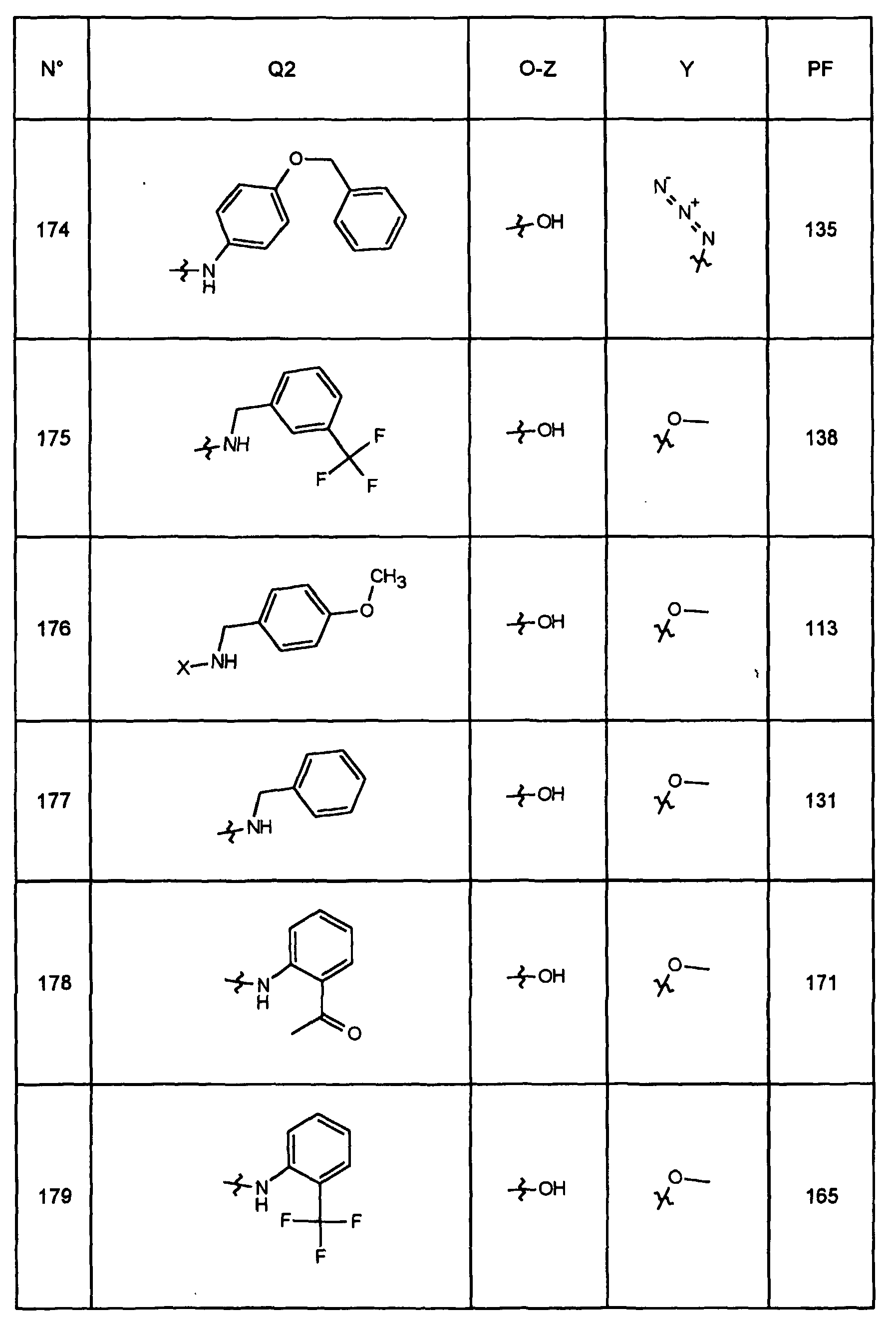

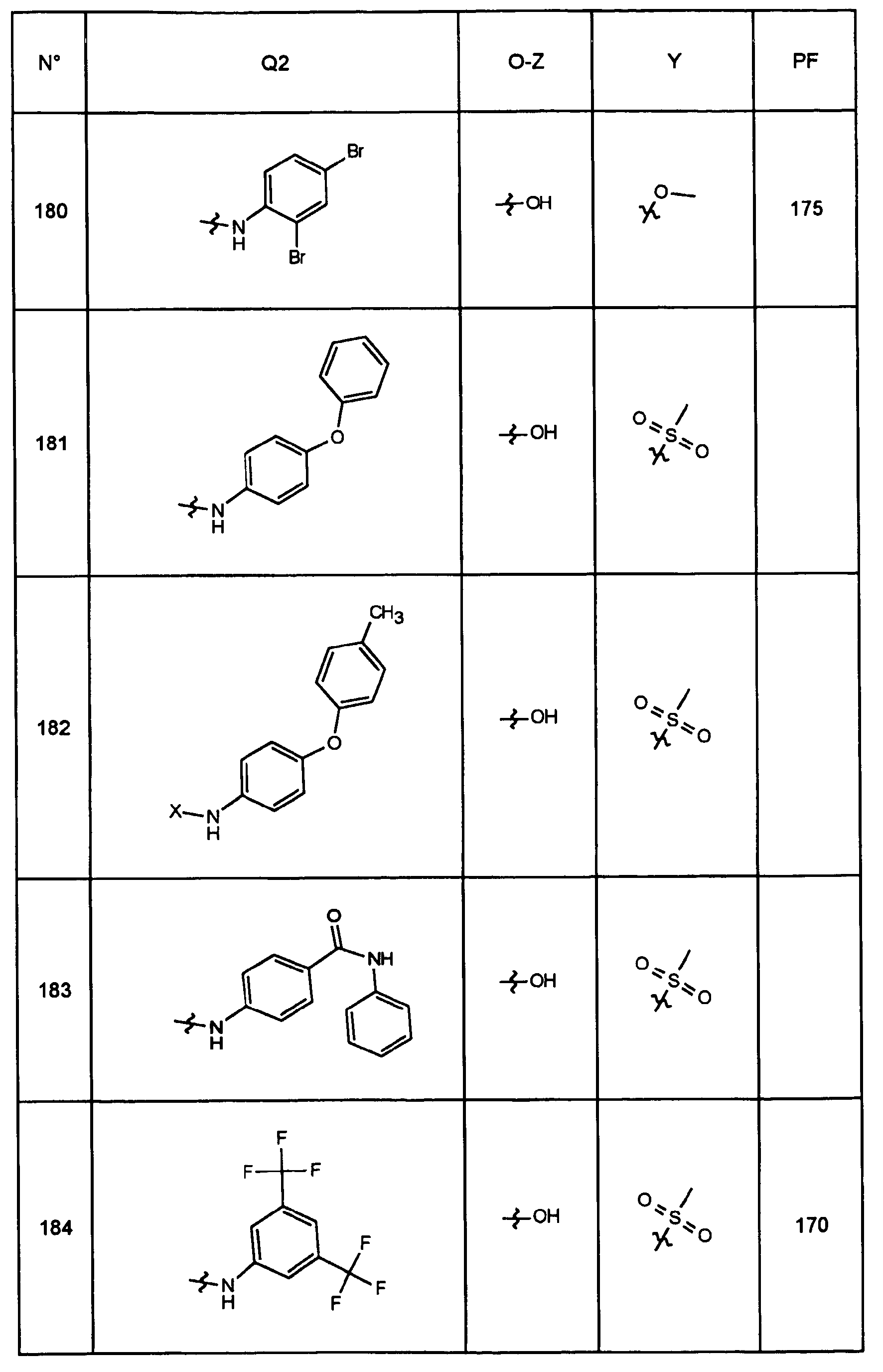

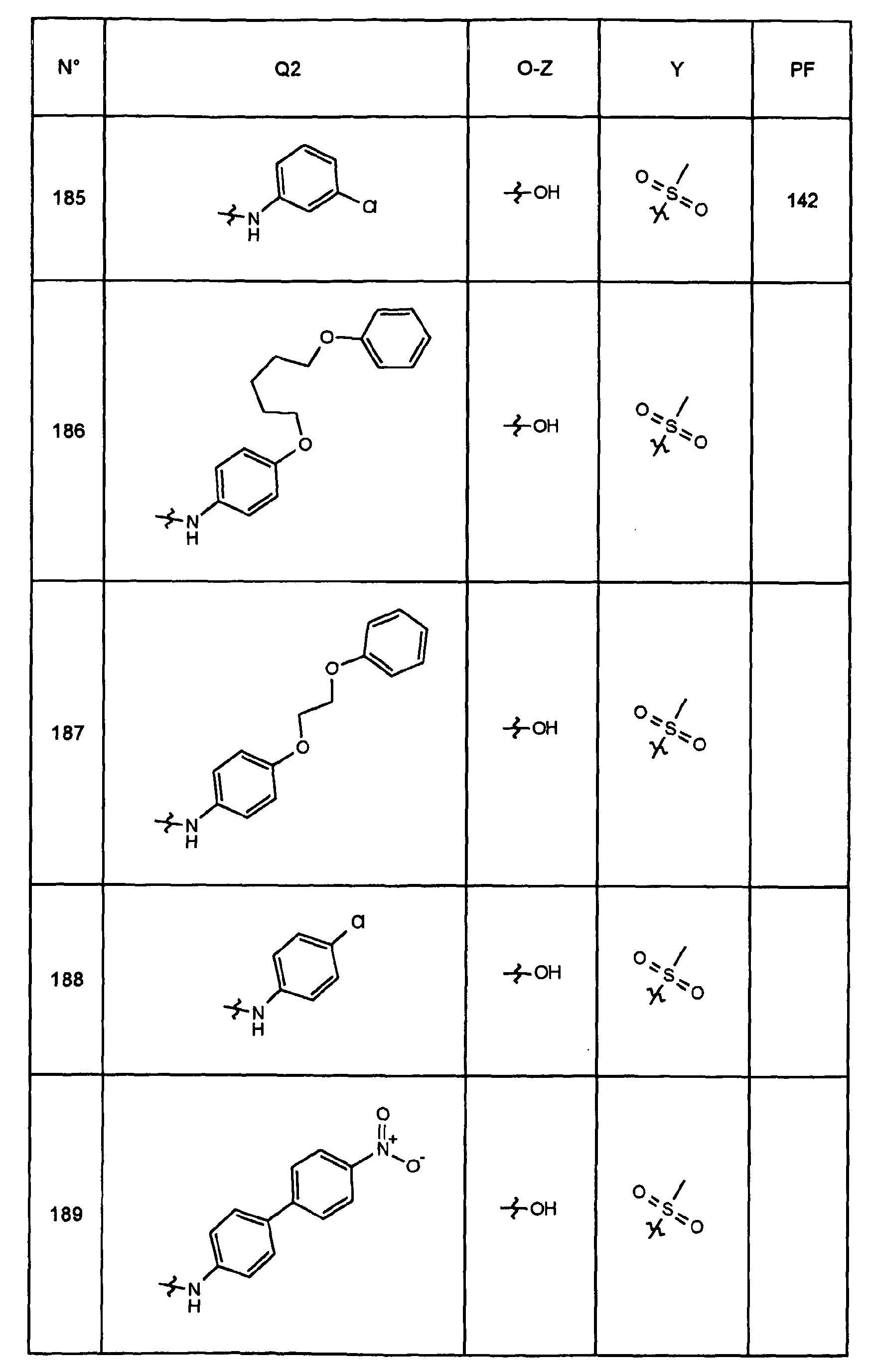

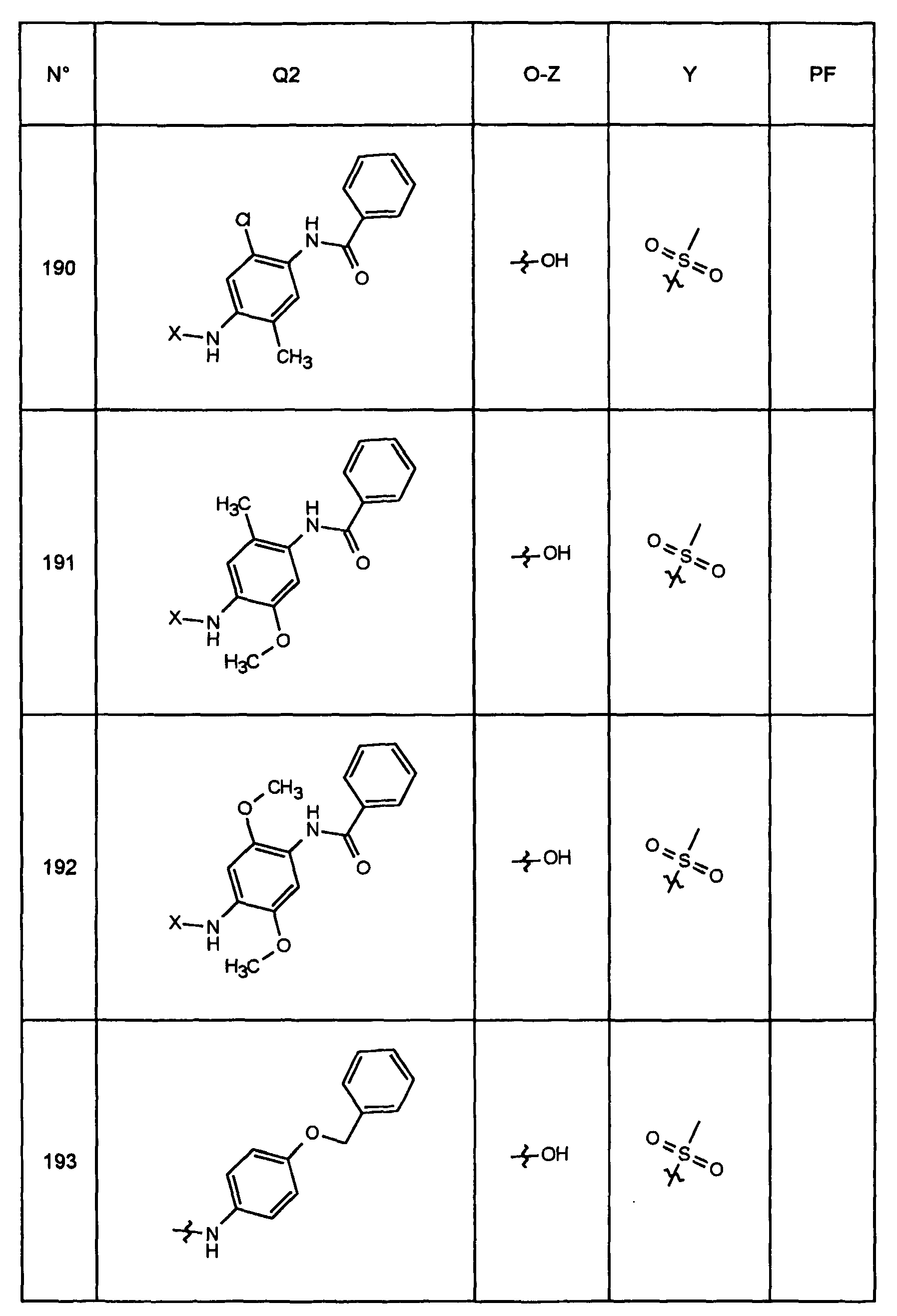

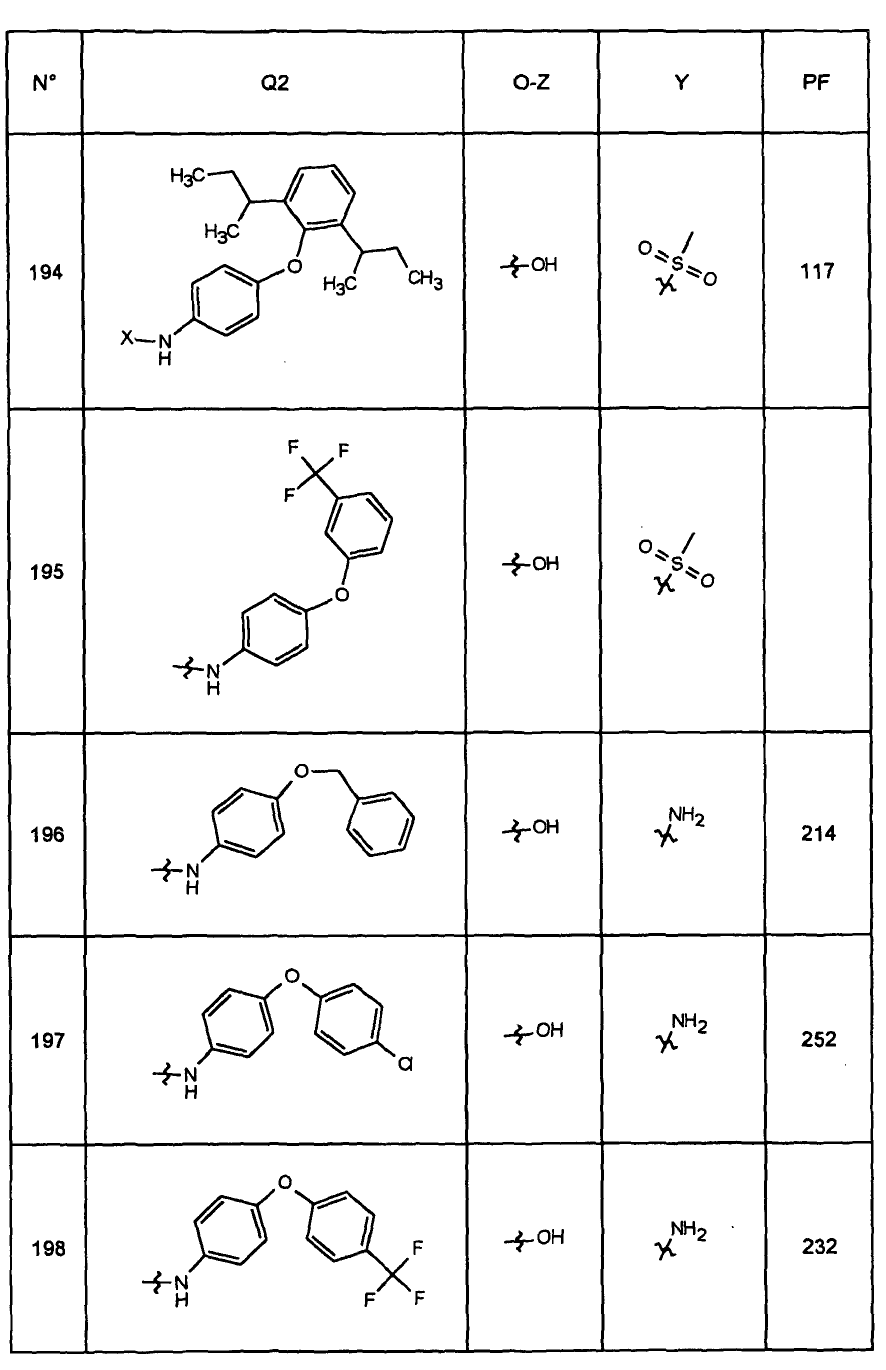

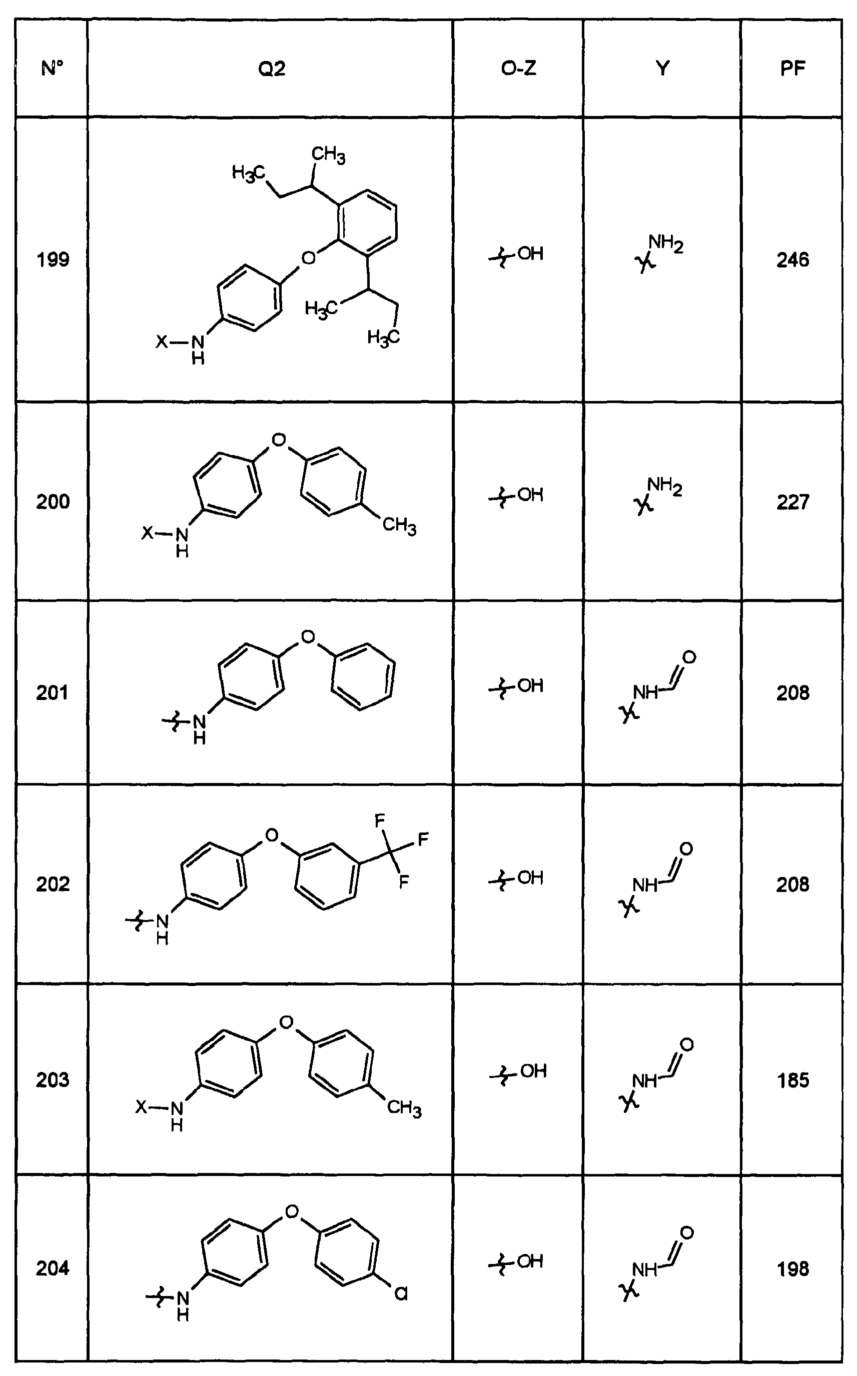

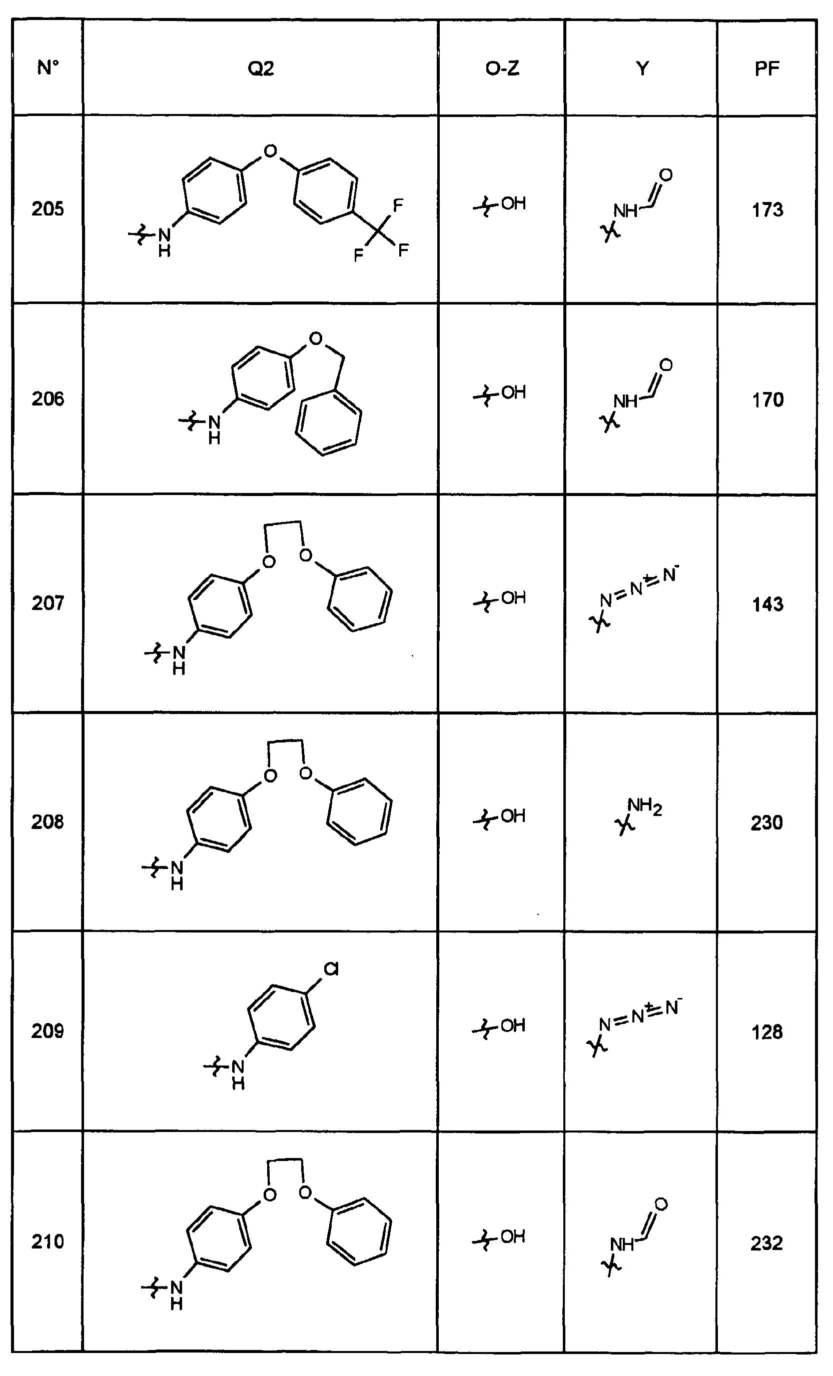

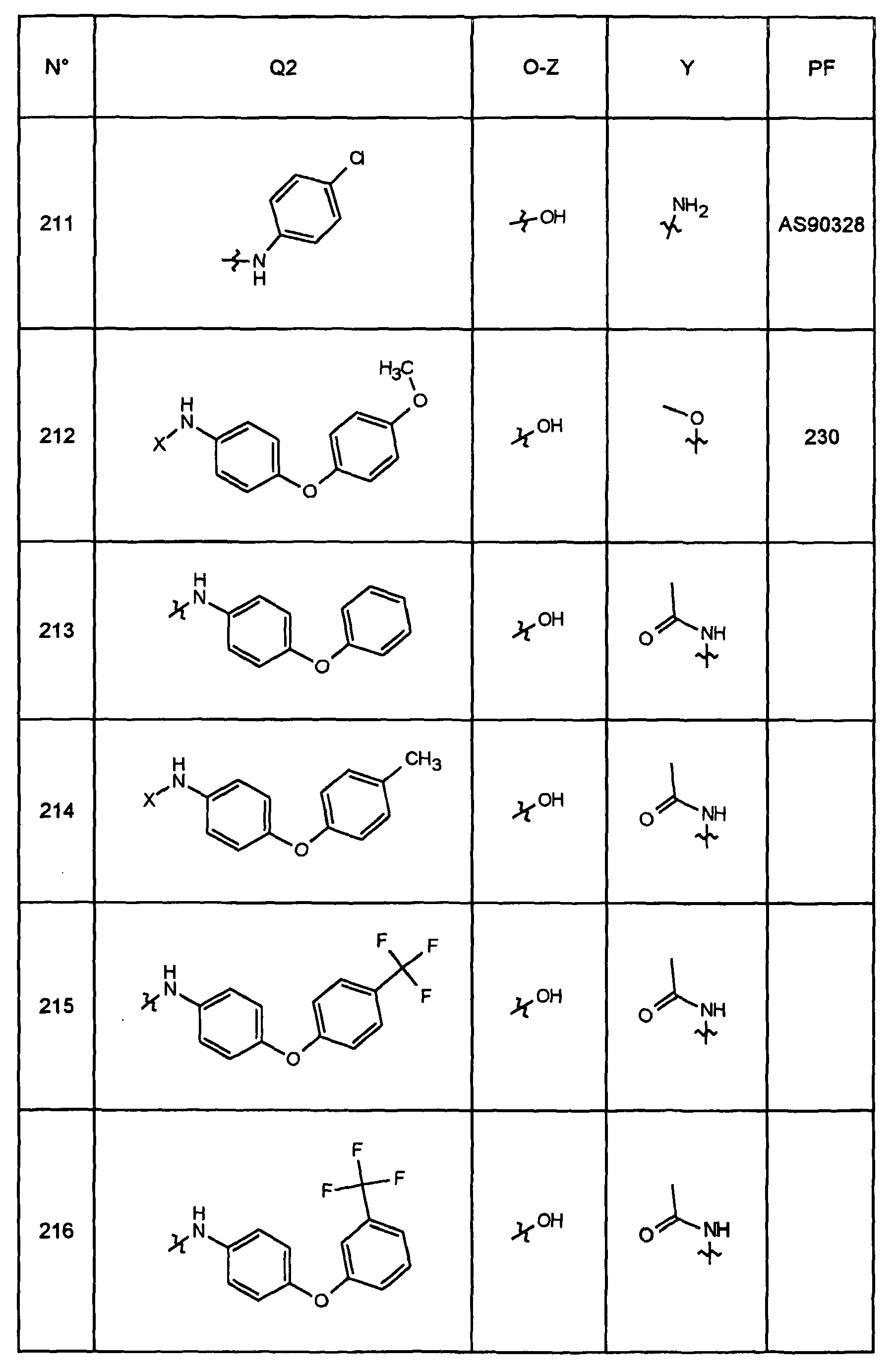

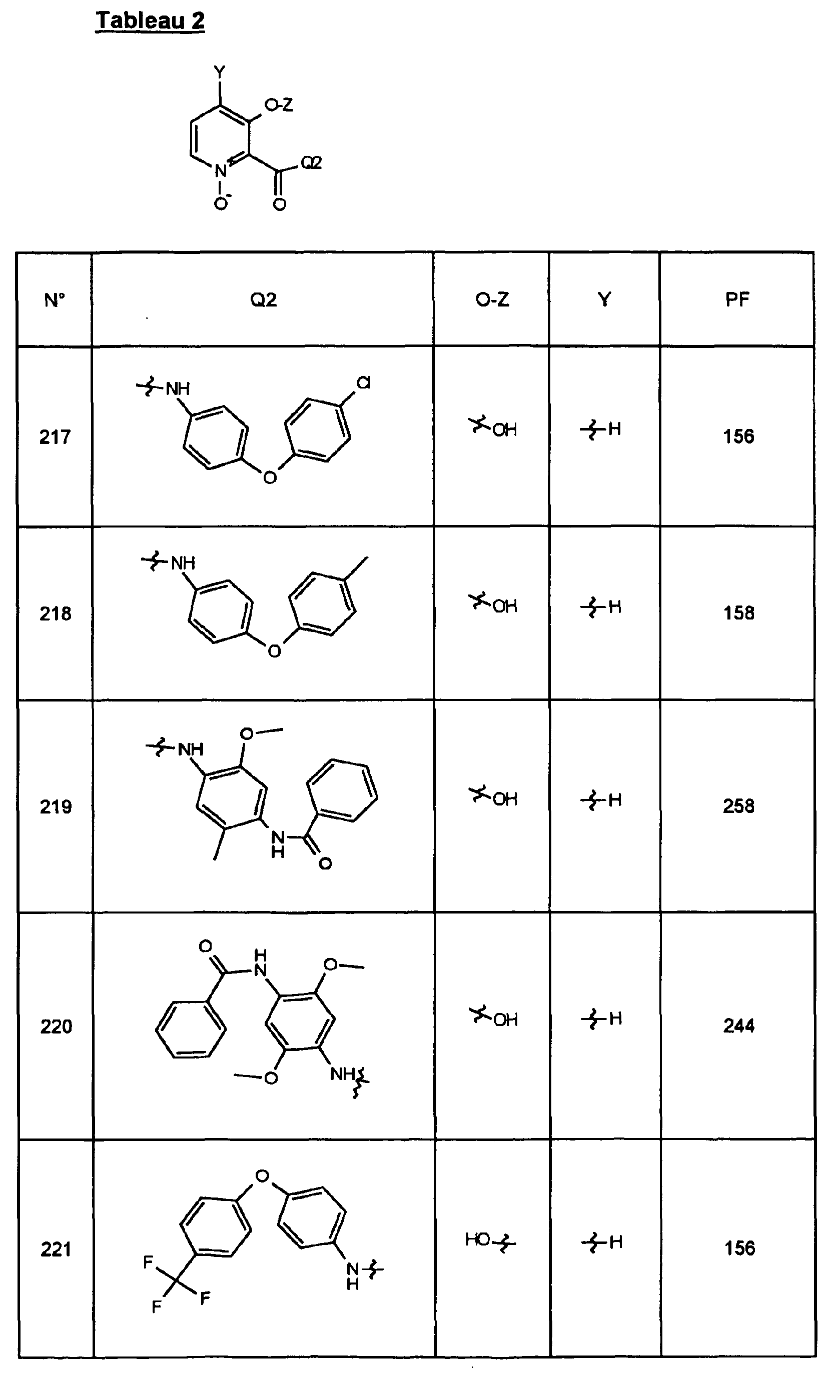

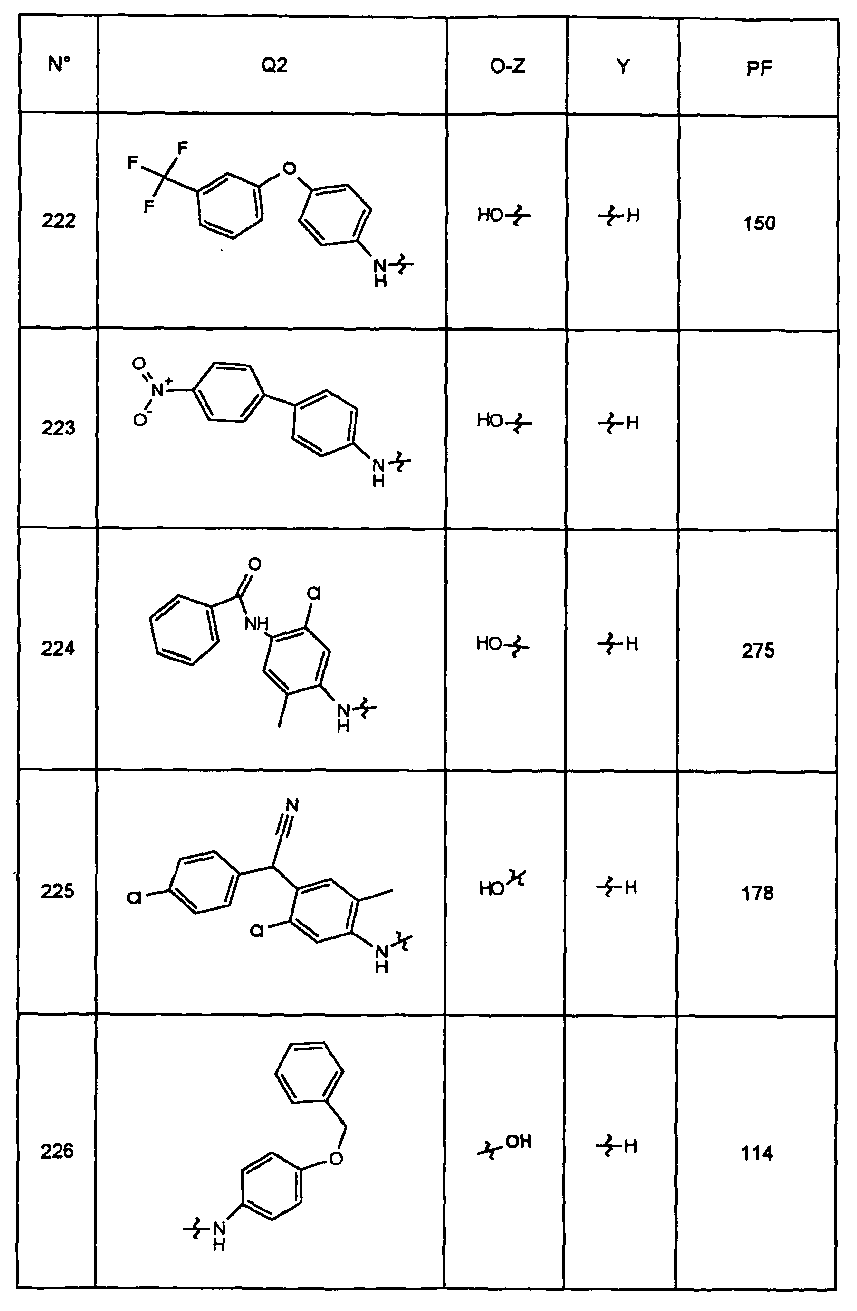

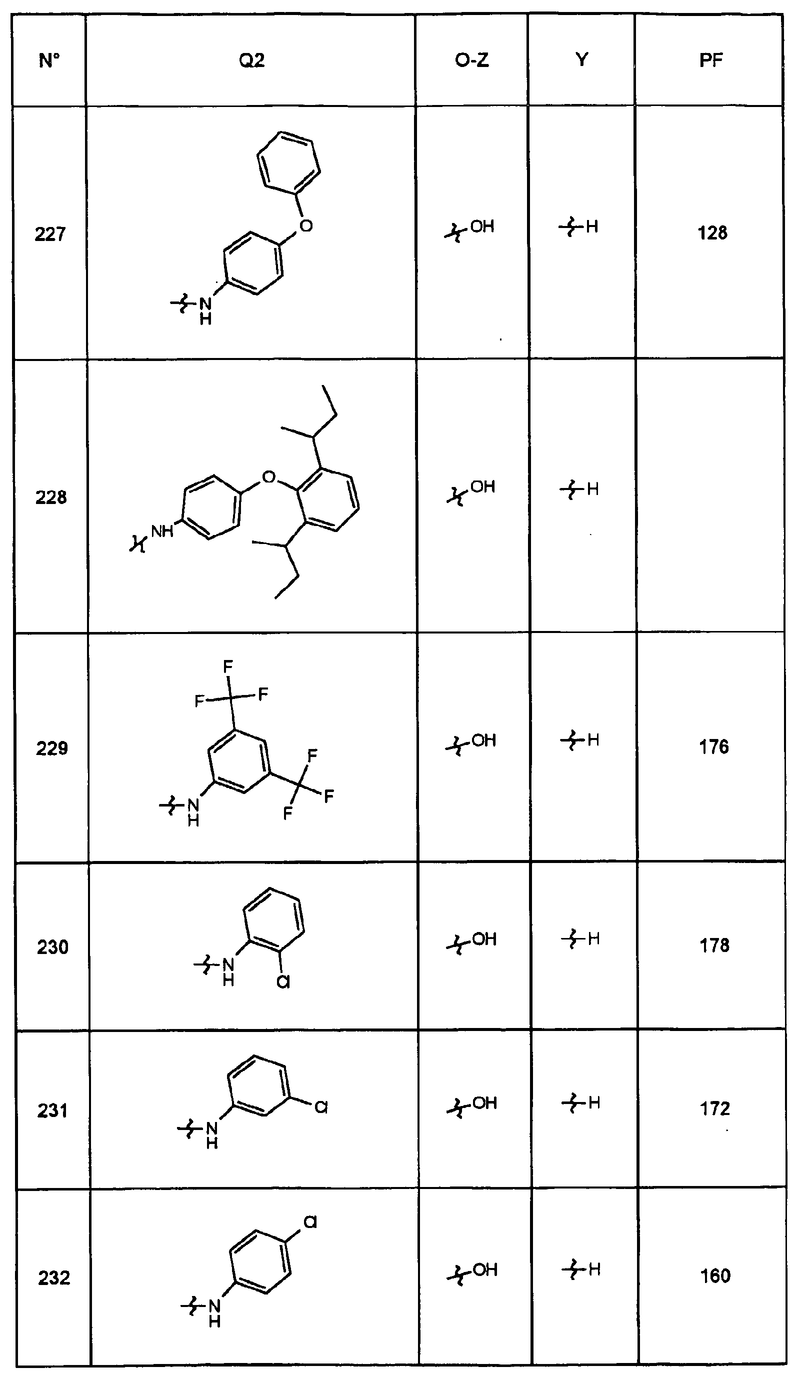

## Revendications

1. Procédé de préparation des composés de formule générale (I) : 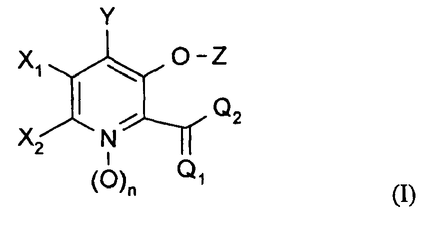 dans lesquels :
• n représente 0 ou 1,
• Q₁ est choisi parmi l'atome d'oxygène, de soufre, le groupe NR₁ et le groupe N-NR₄R₅,
• Q₂ est choisi parmi le groupe OR₂, SR₃ et le groupe -NR₄R₅,
ou bien
• Q₁ et Q₂ peuvent former ensemble un cycle de 5 à 7 atomes contenant 2 à 3 atomes d'oxygène et/ou d'azote, éventuellement substitué par un ou plusieurs radicaux identiques ou différents choisis parmi les halogènes, les radicaux alkyle et haloalkyle,
• Z est choisi parmi l'atome d'hydrogène, le radical cyano, un radical alkyle, allyle, aryle, arylalkyle, propargyle, cycloalkyle, halocycloalkyle, alcényle, alcynyle, cyanoalkyle, haloalkyle, alkoxyalkyle, haloalkoxyalkyle, alkylthioalkyle, haloalkylthioalkyle, N-alkylaminoalkyle, N,N-dialkylaminoalkyle, acylaminoalkyle, alkoxycarbonylaminoalkyle, aminocarbonylaminoalkyle, alkoxycarbonyle, N-alkylamino-carbonyle, N,N-dialkylaminocarbonyle, acyle, thioacyle, alkoxythiocarbonyle, N-alkylaminothiocarbonyle, N,N-dialkylaminothiocarbonyle, alkylsulfinyle, haloalkylsulfinyle, alkylsulfonyle, haloalkylsulfonyle, alkoxysulfonyle, aminosulfonyle, N-alkylaminosulfonyle, N,N-dialkylaminosulfonyle, arylsulfinyle, arylsulfonyle, aryloxysulfonyle, N-arylaminosulfonyle, N,N-diarylaminosulfonyle, et N,N-arylalkylaminosulfonyle ;
• Y est choisi parmi l'atome d'hydrogène, un atome d'halogène,
le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, alkoxysulfonyle, un groupe cycloalkyle, halocycloalkyle, alcényle, alcynyle, alcényloxy, alcynyloxy, alcénylthio, alcynylthio,
un groupe amino, N-alkylamino, N,N-dialkylamino, -NHCOR₁₀, -NHCSR₁₀, N-alkylaminocarbonylamino, N,N-dialkylaminocarbonylamino, aminoalkyle, N-alkyl-aminoalkyle, N,N-dialkylaminoalkyle, acylaminoalkyle, thioacylamino, alkoxythiocarbonylamino, N-alkylaminothiocarbonylamino, N,N-dialkylaminothiocarbonylamino, N,N-arylalkylaminocarbonylamino, N-alkylsulfinylamino, N-alkylsulfonylamino, N-arylsulfinylamino, N-arylsulfonylamino, N-alkoxysulfonylamino, N-alkoxysulfinylamino, N-haloalkoxysulfinylamino, N-haloalkoxysulfonylamino,
N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino, N,N-arylalkylaminothiocarbonylamino,
un radical acyle, carboxy, carbamoyle, N-alkylcarbamoyle, N,N-dialkylcarbamoyle, alkoxycarbonyle inférieur, N-arylcarbamoyle, N,N-diarylcarbamoyle, aryloxycarbonyle, N,N-arylalkylcarbamoyle, et un groupe imino de formule : 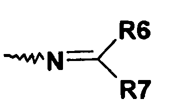
• X₁ et X₂ sont identiques ou différents et sont choisis indépendamment l'un de l'autre parmi l'atome d'hydrogène, un atome d'halogène, le radical hydroxy, mercapto, nitro, thiocyanato, azido, cyano, pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, cyanoalkyle, cyanoalkoxy, cyanoalkylthio, alkylsulfinyle, haloalkylsulfinyle, alkylsulfonyle, haloalkylsulfonyle, et alkoxysulfonyle, ou bien
• X₁ et X₂ peuvent également être joints ensemble, formant ainsi un cycle de 4 à 8 chaînons, saturé, partiellement insaturé ou totalement insaturé, et comportant éventuellement un ou plusieurs hétéroatomes choisis parmi le soufre, l'oxygène, l'azote et le phosphore,
• R₂ et R₃ sont identiques ou différents et sont choisis indépendamment l'un de l'autre parmi un radical alkyle comprenant de 1 à 12 atomes de carbone, haloalkyle, cycloalkyle, halocycloalkyle, alkoxy, haloalkoxy, alkylthio, haloalkylthio, alkoxyalkyle, haloalkoxyalkyle, alkylthioalkyle, haloalkylthioalkyle, cyanoalkyle, acyle, le radical nitro, cyano, carboxy, carbamoyle, 3-oxétanyloxycarbonyle, un radical N-alkylcarbamoyle, N,N-dialkylcarbamoyle, alkoxycarbonyle, alkylthiocarbonyle, haloalkoxycarbonyle, alkoxythiocarbonyle, haloalkoxythiocarbonyle, alkylthiothiocarbonyle, alcényle, alcynyle, N-alkylamino, N,N-dialkylamino, N-alkylaminoalkyle, et N,N-dialkylaminoalkyle, ou bien
un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement -T-R₈, ou bien,
• R₁, R₄, R₅, R₆ et R₇ sont identiques ou différents et sont choisis indépendamment l'un de l'autre parmi l'atome d'hydrogène, un radical alkyle comprenant de 1 à 12 atomes de carbone en chaîne linéaire ou ramifiée, éventuellement substitué, haloalkyle, cycloalkyle, halocycloalkyle, alkoxy, aryloxy, arylalkoxy, haloalkoxy, alkylthio, haloalkylthio, alkoxyalkyle, haloalkoxyalkyle, alkylthioalkyle, haloalkylthioalkyle, cyanoalkyle, acyle, le radical nitro, cyano, carboxy, carbamoyle, 3-oxétanyloxycarbonyle, un radical N-alkylcarbamoyle, N,N-dialkylcarbamoyle, alkoxycarbonyle, alkylthiocarbonyle, haloalkoxycarbonyle, alkoxythiocarbonyle, haloalkoxythiocarbonyle, alkylthiothiocarbonyle, alcényle, alcynyle, N-alkylamino, N,N-dialkylamino, N-alkylaminoalkyle, et N,N-dialkylaminoalkyle, ou bien
un radical choisi parmi aryle, arylalkyle, hétérocyclyle et hétérocyclylalkyle, éventuellement substitué par un ou plusieurs radicaux R₉ et/ou aryle et/ou arylalkyle, identiques ou différents et/ou un groupement-T-R₈, ou bien,
• R₄ et R₅ d'une part ou R₆ et R₇ d'autre part peuvent être joints ensemble, formant ainsi un cycle de 4 à 8 chaînons, saturé, partiellement insaturé ou totalement insaturé, et comportant éventuellement un ou plusieurs hétéroatomes choisis parmi le soufre, l'oxygène, l'azote et le phosphore,
• T représente une liaison directe ou un radical divalent choisi parmi un radical -(CH₂)ₘ-, m prenant une valeur comprise entre 1 et 12, bornes incluses, le dit radical étant éventuellement interrompu ou borné par un ou deux hétéroatomes choisis parmi l'azote, l'oxygène et/ou le soufre, un radical oxyalkylène, alkoxyalkylène, carbonyle (-CO-), oxycarbonyle(-O-CO-), carbonyloxy(-CO-O-), sulfinyle (-SO-), sulfonyle (-SO₂-), oxysulfonyle (-O-SO₂-), sulfonyloxy (-SO₂-O-), oxysulfinyle (-O-SO-), sulfinyloxy (-SO-O-), thio (-S-), oxy (-O-), vinyle (-C=C-), éthinyle (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O-, et -O-NR₉-CO-,
• R₈ est choisi parmi l'atome d'hydrogène et un radical aryle, ou hétérocyclyle,
• R₉ et R₁₀, identiques ou différents, sont choisis indépendamment l'un de l'autre parmi l'atome d'hydrogène, un atome d'halogène, le radical hydroxy, mercapto, nitro, thiocyanato, azido, cyano ou pentafluorosulfonyle, un radical alkyle, haloalkyle, alkoxy, haloalkoxy, alkylthio, haloalkylthio, alkoxyalkyle, haloalkoxyalkyle, alkylthioalkyle, haloalkylthioalkyle, arylalkyle, cyanoalkyle, cyanoalkoxy, cyanoalkylthio, alkylsulfinyle, haloalkylsulfinyle, alkylsulfonyle, haloalkylsulfonyle, et alkoxysulfonyle,
ainsi que les éventuels N-oxydes, isomères géométriques et/ou optiques, énantiomères et/ou diastéréoisomères, formes tautomères, leurs sels, complexes métalliques et métalloïdiques,
procédé **caractérisé en ce que** l'on fait réagir un composé de formule (II) : 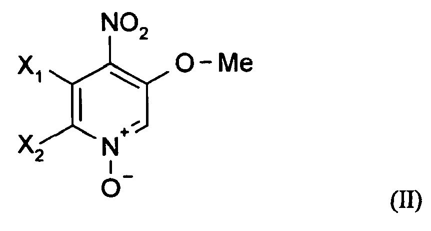 dans lequel X₁ et X₂ sont tels que définis précédemment,
d'un cyanure, de dérivés alcalins ou alcalino-terreux de l'acide cyanhydrique en présence d'un agent alkylant et d'un solvant ou de cyanure de triméthylsilyle en présence de chlorure de diméthylcarbamoyle et d'un solvant, pour conduire aux composés de formule (III) : 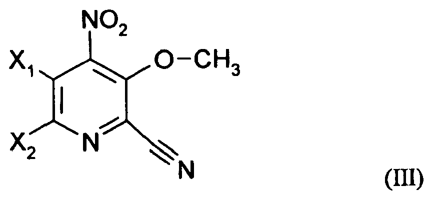 dans lesquels X₁ et X₂ sont tels que définis précédemment,
les composés de formule (III) ci-dessus pouvant être transformés en dérivés halogénés correspondants de formule (IVa) : 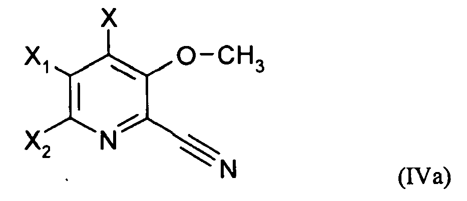 dans lesquels X₁ et X₂ sont tels que précédemment définis et X représente un atome d'halogène choisi parmi fluor, chlore, brome ou iode,
par réaction avec un halogénure d'acyle en présence d'un solvant,
les dérivés halogénés de formule (IVa) étant alors hydrolysés en composés de formule (Ia) : 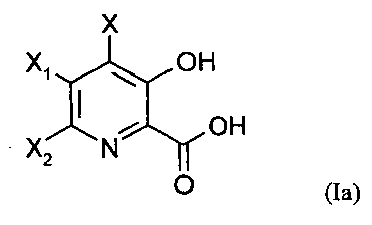 dans lesquels X, X₁ et X₂ sont tels que précédemment définis,
par action d'hydracide à chaud - ou d'une base forte minérale, éventuellement en présence d'eau oxygénée - et éventuellement de tribromure de bore,
les composés de formules (III) ou (IVa) pouvant également être mis au contact d'un alcool ou d'un alcoolate en présence d'un solvant tel que, de préférence, mais non exclusivement, un solvant protique ou polaire aprotique, pour conduire aux composés de formule (IVb) : 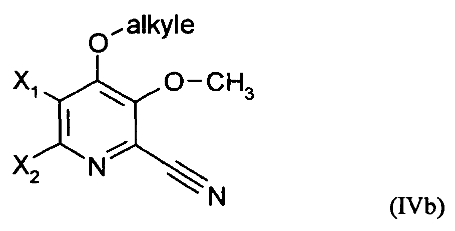 dans lesquels X₁ et X₂ sont tels que précédemment définis,
puis engagés dans une réaction d'hydrolyse selon des conditions opératoires similaires à celles utilisées pour la formation des composés de formule (Ia), pour conduire aux composés de formules respectives (Ib) et (Ib') : 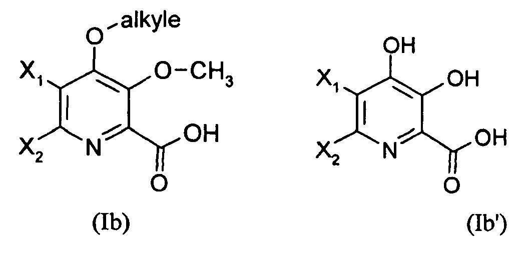 dans lesquels X₁ et X₂ sont tels que précédemment définis.
les composés de formule (IVa) pouvant également être transformés en dérivés d'acide picolinique de formule (Va) : 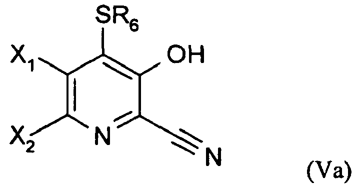 dans lesquels X₁, X₂ et R₆ sont tels que définis précédemment,
en faisant réagir un composé de formule R₆SH, ou un sel alcalin ou alcalino-terreux correspondant, dans un solvant aprotique polaire, à une température comprise entre 0°C et la température d'ébullition du solvant,
les nitriles de formule (Va) pouvant alors être engagés dans une réaction d'hydrolyse pour conduire aux acides correspondants de formule (Ic) : 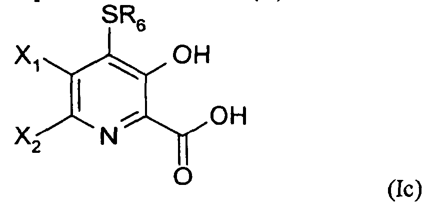 dans lesquels X₁, X₂ et R₆ sont tels que définis précédemment,
selon une réaction similaire à celle employée pour la formation des composés de formule (Ia),
les halogénures de formule (IVa) pouvant encore être traités avec un sel de l'acide azothydrique, pour conduire aux composés de formule (Vb) : 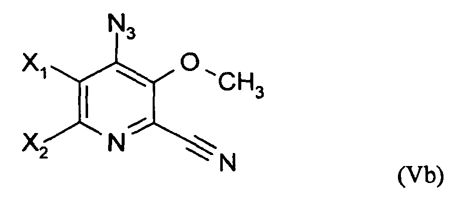 dans lesquels X₁ et X₂ sont tels que définis précédemment,
cette réaction étant conduite à une température comprise entre 0°C et la température d'ébullition du solvant,
les composés de formule (Vb) pouvant alors être hydrolysés selon des techniques similaires à celles présentées pour la préparation des acides de formule (Ia) ci-dessus, pour conduire aux acides de formule (Id) : 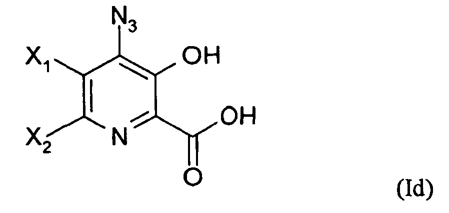 dans lesquels X₁ et X₂ sont tels que définis précédemment,
les nitrures de formule (Id) étant alors éventuellement réduits en dérivés aminés de formule (Ie) : 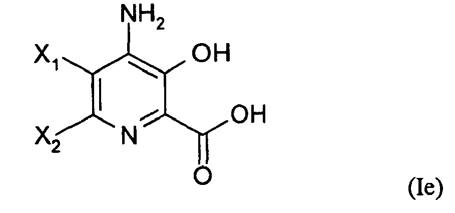 dans lesquels X₁ et X₂ sont tels que définis précédemment,
par action d'un agent réducteur,
les acides de formules (Ia) à (Ie) pouvant être transformés en thioacides, en dérivés imino (-C(=NR₁)) ou encore amino-imino (-C(=N-NR₄R₅)) selon des techniques classiques bien connues de l'homme du métier spécialiste en synthèse organique,
les acides (Ia) à (Ie), ou leurs dérivés thio, imino et imino-amino définis ci-dessus, substitués en position 3 (par rapport à l'atome d'azote pyridinique) par -OH ou -méthoxy pouvant être soumis à diverses réactions déjà connues dans l'art antérieur afin de conduire aux dérivés correspondants substitués en position 3 (par rapport à l'atome d'azote pyridinique) par -O-Z, Z étant tel que défini pour les composés de formule (I).

2. Procédé de préparation selon la revendication 1, **caractérisé en ce que** Y représente le radical amino (-NH₂) et que le procédé est complété par une étape comprenant la mise en contact du composé de formule (I) obtenu avec un agent acylant en présence d'un solvant et éventuellement d'une base afin de conduire aux composés de formules (If) et (Ig) : 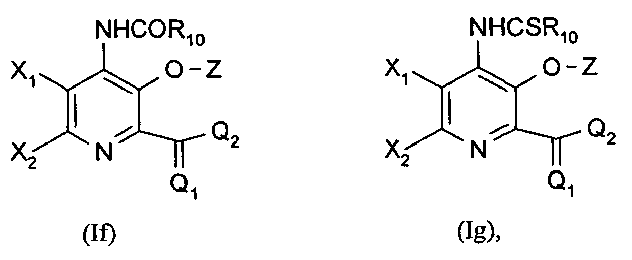 dans lesquels X₁, X₂, Q₁, Q₂, Z et R₁₀ sont tels que définis dans la revendication 1.

3. Procédé de préparation selon la revendication 1, **caractérisé en ce que** Q₂ représente -OH et que le procédé est complété par une étape comprenant la mise en contact du composé de formule (I) obtenu avec un réactif de formule R₂OH, R₃SH, ou HNR₄R₅ en présence d'un agent activant, pour conduire respectivement aux composés de formules (Ih), (Ii), et (Ij) : 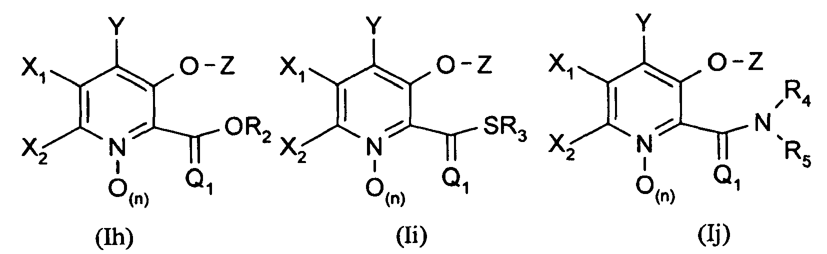 dans lesquels X₁, X₂, Q₁, Q₂, Y, Z, n, R₄ et R₅ sont tels que définis dans la revendication 1.

4. Procédé de préparation selon la revendication 1, **caractérisé en ce que** n est égal à 0 et que le procédé est complété par une étape comprenant la mise en contact du composé de formule (I) obtenu avec un agent oxydant pour obtenir un composé de formule (Ik) 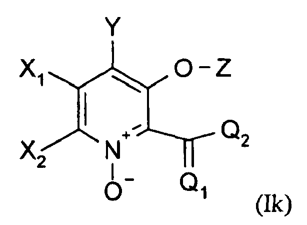 dans lesquels X₁, X₂, Q₁, Q₂, Y, Z sont tels que définis dans la revendication 1.

5. Procédé de préparation selon l'une quelconque des revendications précédentes, **caractérisé en ce que** le solvant utilisé pour la réaction d'halogénation des composés de formule (III) en composés de formule (IV) est choisi parmi le diéthyléther, le diisopropyléther, le tétrahydrofurane, le dioxane et le 1,2-diméthoxyéthane.

6. Procédé selon l'une quelconque des revendications précédentes, **caractérisé en ce que** la réaction d'hydrolyse des composés de formule (IV) en composés de formule (Ia) consiste à traiter le nitrile de formule (IVa) par un acidecette réaction d'hydrolyse étant réalisée dans l'acide en excès, en l'absence ou en présence d'un solvant, au reflux ou à une température comprise entre 20°C et 200°C.

7. Procédé selon l'une quelconque des revendications précédentes, **caractérisé en ce que** le solvant utilisé dans la réaction de transformation des composés de formule (IVa) en composés de formule (Va) est choisi parmi la diméthylformamide, la diméthylacétamide, la N-méthylpyrrolidone, la diméthylprolylène-urée et le diméthylsulfoxyde.

8. Procédé selon l'une quelconque des revendications précédentes, **caractérisé en ce que** le solvant utilisé dans la réaction de transformation des composés de formule (IVa) en composés de formule (Vb) est choisi parmi la diméthylformamide, la diméthylacétamide, la N-méthylpyrrolidone, la diméthylprolylène-urée ou le diméthylsulfoxyde.

9. Procédé selon l'une quelconque des revendications précédentes, **caractérisé en ce que** l'agent réducteur utilisé pour la réduction des composés de formule (Id) en composés de formule (Ie) est choisi parmi l'aluminohydrure de lithium, la triphénylphosphine et l'hydrogène en présence d'un catalyseur.

10. Procédé selon la revendication 2, **caractérisé en ce que** l'agent acylant utilisé dans l'étape de préparation des composés de formules (If) et (Ig) est choisi parmi un halogénure d'acyle, un anhydride, un acide, un ester, un amide primaire et leurs homologues thio.

11. Procédé selon la revendication 3, **caractérisé en ce que** l'agent activant employé pour la formation des composés de formule (Ih), (Ii) ou (Ij) est choisi parmi le chlorure de thionyle, le chlorure d'oxalyle, le dicyclocarbodi-imide, le chlorhydrate de 1-(3-diméthylaminopropyl)-3-éthylcarbodi-imide, le 1-hydroxy-benzotriazole et l'oxychlorure de phosphore.

12. Procédé selon la revendication 3 ou 11, **caractérisé en ce que** la réaction permettant l'obtention des composés de formule (Ih), (Ii) ou (Ij) est réalisé en présence d'une base organique ou inorganique choisie parmi les hydroxydes de métaux alcalins et alcalino-terreux, les alcoolates de métaux alcalins et alcalino-terreux, les hydrures de métaux alcalins et alcalino-terreux, les carbonates et bicarbonates de métaux alcalins et alcalino-terreux et les bases organiques azotées.

13. Procédé selon l'une quelconque des revendications précédentes, **caractérisé en ce que** les dérivés de l'acide 3-hydroxypicolinique de formule générale (I) sont :
• le 3-hydroxy-N-{[3-(trifluorométhyl)benzyl]oxy}-2-pyridine carboxamide,
• le 1-{3-hydroxy-2-[(4-phénoxyanilino)carbonyl]-4-pyridinyl}-1,2-triazadién-2-ium,
• le 4-amino-3-hydroxy-N-{4-[4-(trifluorométhyl)phénoxy]phényl}-2-pyridine carboxamide,
• le 4-amino-3-hydroxy-N-[4-(4-méthylphénoxy)phényl]-2-pyridine carboxamide,
• le 4-(formylamino)-3-hydroxy-N-{4-[3-(trifluorométhyl)phénoxy] phényl}-2-pyridine carboxamide,
• le N-[4-(4-chlorophénoxy)phényl]-4-(formylamino)-3-hydroxy-2-pyridine carboxamide,
• le 4-(formylamino)-3-hydroxy-N-{4-[4-(trifluorométhyl)phénoxy] phényl}-2-pyridine carboxamide, et
• le N-[4-(benzyloxy)phényl]-4-(formylamino)-3-hydroxy-2-pyridine carboxamide,
ainsi que les éventuels N-oxydes, isomères géométriques et/ou optiques, énantiomères et/ou diastéréoisomères, formes tautomères, les sels, complexes métalliques et métalloïdiques.

## Patentansprüche

1. Verfahren zur Herstellung von Verbindungen der allgemeinen Formel (I): 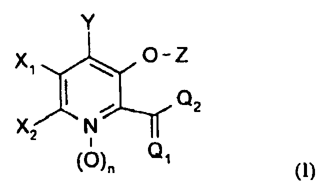 in denen:
• n 0 oder 1 bedeutet,
• Q₁ aus der Reihe Sauerstoffatom, Schwefelatom, NR₁-Gruppe und N-NR₄R₅-Gruppe stammt,
• Q₂ aus der Reihe OR₂-Gruppe, SR₃-Gruppe und NR₄R₅-Gruppe stammt oder
• Q₁ und Q₂ gemeinsam einen Ring mit 5 bis 7 Atomen, der 2 bis 3 Sauerstoff- und/oder Stickstoffatome enthält und gegebenenfalls durch einen oder mehrere gleiche oder verschiedene Reste aus der Reihe Halogene, Alkylreste und Halogenalkylreste substituiert ist, bilden können,
• Z aus der Gruppe Wasserstoffatom, Cyanorest, Alkyl-, Allyl-, Aryl-, Arylalkyl-, Propargyl-, Cycloalkyl-, Halogencycloalkyl-, Alkenyl-, Alkinyl-, Cyanoalkyl-, Halogenalkyl-, Alkoxyalkyl-, Halogenalkoxyalkyl-, Alkylthioalkyl-, Halogenalkylthioalkyl-, N-Alkylaminoalkyl-, N,N-Dialkylaminoalkyl-, Acylaminoalkyl-, Alkoxycarbonylaminoalkyl-, Aminocarbonylaminoalkyl-, Alkoxycarbonyl-, N-Alkylaminocarbonyl-, N,N-Dialkylaminocarbonyl-, Acyl-, Thioacyl-, Alkoxythiocarbonyl-, N-Alkylaminothiocarbonyl-, N,N-Dialkylaminothiocarbonyl-, Alkylsulfinyl-, Halogenalkylsulfinyl-, Alkylsulfonyl-, Halogenalkylsulfonyl-, Alkoxysulfonyl-, Aminosulfonyl-, N-Alkylaminosulfonyl-, N,N-Dialkylaminosulfonyl-, Arylsulfinyl-, Arylsulfonyl-, Aryloxysulfonyl-, N-Arylaminosulfonyl-, N,N-Diarylaminosulfonyl- und N,N-Arylalkylaminosulfonylrest stammt;
• Y aus der Reihe Wasserstoffatom, Halogenatom, Hydroxy-, Mercapto-, Nitro-, Thiocyanato-, Azido-, Cyano-, Pentafluorsulfonylrest, Alkyl-, Halogenalkyl-, Alkoxy-, Halogenalkoxy-, Alkylthio-, Halogenalkylthio-, Alkoxyalkyl-, Halogenalkoxyalkyl-, Alkylthioalkyl-, Halogenalkylthioalkyl-, Cyanoalkyl-, Cyanoalkoxy-, Cyanoalkylthio-, Alkylsulfinyl-, Halogenalkylsulfinyl-, Alkylsulfonyl-, Halogenalkylsulfonyl-, Alkoxysulfonylrest,
Cycloalkyl-, Halogencycloalkyl-, Alkenyl-, Alkinyl-, Alkenyloxy-, Alkinyloxy-, Alkenylthio-, Alkinylthiogruppe,
Amino-, N-Alkylamino-, N,N-Dialkylamino-, -NHCOR₁₀, -NHCSR₁₀, N-Alkylaminocarbonylamino-, N,N-Dialkylaminocarbonylamino, Aminoalkyl-, N-Alkylaminoalkyl-, N,N-Dialkylaminoalkyl-, Acylaminoalkyl-, Thioacylamino-, Alkoxythiocarbonylamino-, N-Alkylaminothiocarbonylamino-, N,N-Dialkylaminothiocarbonylamino-, N,N-Arylalkylaminocarbonylamino-, N-Alkylsulfinylamino-, N-Alkylsulfonylamino-, N-Arylsulfinylamino-, N-Arylsulfonylamino-, N-Alkoxysulfonylamino-, N-Alkoxysulfinylamino-, N-Halogenalkoxysulfinylamino-, N-Halogenalkoxysulfonylamino-, N-Arylamino-, N,N-Diarylamino-, Arylcarbonylamino-, Alkoxycarbonylamino-, N-Arylaminocarbonylamino-, N,N-Diarylaminocarbonylamino-, Arylthiocarbonylamino-, Aryloxythiocarbonylamino-, N-Arylaminothiocarbonylamino-, N,N-Diarylaminothiocarbonylamino-, N,N-Arylalkylaminothiocarbonylaminogruppe,
Acyl-, Carboxy-, Carbamoyl-, N-Alkylcarbamoyl-, N,N-Dialkylcarbamoyl-, Niederalkoxycarbonyl-, N-Arylcarbamoyl-, N,N-Diarylcarbamoyl-, Aryloxycarbonyl-, N,N-Arylalkylcarbamoylrest sowie
Iminogruppe der Formel: 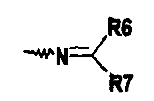 stammt;
• X₁ and X₂ gleich oder verschieden sind und unabhängig voneinander aus der Reihe Wasserstoffatom, Halogenatom, Hydroxy-, Mercapto-, Nitro-, Thiocyanato-, Azido-, Cyano, Pentafluorsulfonylrest, Alkyl-, Halogenalkyl-, Alkoxy, Halogenalkoxy, Alkylthio, Halogenalkylthio-, Alkoxyalkyl-, Halogenalkoxyalkyl-, Alkylthioalkyl-, Halogenalkylthioalkyl-, Cyanoalkyl-, Cyanoalkoxy-, Cyanoalkylthio-, Alkylsulfinyl-, Halogenalkylsulfinyl-, Alkylsulfonyl-, Halogenalkylsulfonyl- und Alkoxysulfonylrest stammen, oder
• X₁ and X₂ auch miteinander verbunden sein können, wodurch sie einen gesättigten, teilweise ungesättigten oder vollständig ungesättigten 4-bis 8gliedrigen Ring, der gegebenenfalls ein oder mehrere Heteroatome aus der Reihe Schwefel, Sauerstoff, Stickstoff und Phosphor enthält, bilden,
• R₂ and R₃, die gleich oder verschieden sind, unabhängig voneinander aus der Reihe Alkylrest mit 1 bis 12 Kohlenstoffatomen, Halogenalkyl-, Cycloalkyl, Halogencycloalkyl, Alkoxy, Halogenalkoxy-, Alkylthio-, Halogenalkylthio-, Alkoxyalkyl-, Halogenalkoxyalkyl-, Alkylthioalkyl-, Halogenalkylthioalkyl-, Cyanoalkyl-, Acylrest, Nitro-, Cyano-, Carboxy-, Carbamoyl-, 3-Oxetanyloxycarbonylrest, N-Alkylcarbamoyl-, N,N-Dialkylcarbamoyl-, Alkoxycarbonyl-, Alkylthiocarbonyl-, Halogenalkoxycarbonyl-, Alkoxythiocarbonyl-, Halogenalkoxythiocarbonyl-, Alkylthiothiocarbonyl-, Alkenyl-, Alkinyl-, N-Alkylamino-, N,N-Dialkylamino-, N-Alkylaminoalkyl- und N,N-Dialkylaminoalkylrest, oder auch
• Rest aus der Gruppe Aryl, Arylalkyl, Heterocyclyl und Heterocyclylalkyl, der gegebenenfalls durch einen oder mehrere gleiche oder verschiedene R₉ und/oder Aryl- und/oder Arylalkylreste und/oder eine -T-R₈-Gruppe substituiert ist, stammen, oder
• R₁, R₄, R₅, R₆ und R₇ gleich oder verschieden sind und unabhängig voneinander aus der Reihe Wasserstoffatom, Alkylrest mit 1 bis 12 Kohlenstoffatomen in Form einer geraden oder verzweigten Kette, der gegebenenfalls substituiert ist, Halogenalkyl-, Cycloalkyl-, Halogencycloalkyl-, Alkoxy-, Aryloxy-, Arylalkoxy-, Halogenalkoxy-, Alkylthio-, Halogenalkylthio-, Alkoxyalkyl-, Halogenalkoxyalkyl-, Alkylthioalkyl-, Halogenalkylthioalkyl-, Cyanoalkyl-, Acylrest, Nitro-, Cyano-, Carboxy-, Carbamoyl-, 3-Oxetanyloxycarbonylrest, N-Alkylcarbamoyl, N,N-Dialkylcarbamoyl-, Alkoxycarbonyl, Alkylthiocarbonyl-, Halogenalkoxycarbonyl, Alkoxythiocarbonyl-, Halogenalkoxythiocarbonyl, Alkylthiothiocarbonyl-, Alkenyl, Alkinyl, N-Alkylamino-, N,N-Dialkylamino, N-Alkylaminoalkyl- und N,N-Dialkylaminoalkylrest oder
Rest aus der Gruppe Aryl, Arylalkyl, Heterocyclyl und Heterocyclylalkyl, der gegebenenfalls durch einen oder mehrere gleiche oder verschiedene R₉ und/oder Aryl- und/oder Arylalkylreste und/oder eine -T-R₈-Gruppe substituiert ist, stammen, oder
• R₄ und R₅, oder R₆ and R₇, auch miteinander verbunden sein können, wodurch sie einen gesättigten, teilweise ungesättigten oder vollständig ungesättigten 4- bis 8gliedrigen Ring, der gegebenenfalls ein oder mehrere Heteroatome aus der Reihe Schwefel, Sauerstoff, Stickstoff und Phosphor enthält, bilden,
• T eine direkte Bindung oder einen zweiwertigen Rest aus der Reihe -(CH₂)ₘ-Rest, wobei m einen Wert zwischen inklusive 1 und inklusive 12 annimmt, wobei dieser Rest gegebenenfalls von einem oder zwei Heteroatomen aus der Reihe Stickstoff, Sauerstoff und/oder Schwefel unterbrochen oder begrenzt wird, Oxyalkylen-, Alkoxyalkylen-, Carbonylrest (-CO-), Oxycarbonylrest (-O-CO-), Carbonyloxyrest (-CO-O-), Sulfinylrest (-SO-), Sulfonylrest (-SO₂-), Oxysulfonylrest (-O-SO₂-), Sulfonyloxyrest (-SO₂-O-), Oxysulfinylrest (-O-SO-), Sulfinyloxyrest (-SO-O-), Thiorest (-S-), Oxyrest (-O-), Vinylrest (-C=C-), Ethinylrest (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O- und -O-NR₉-CO- bedeutet,
• R₈ aus der Reihe Wasserstoffatom und Arylrest oder Heterocyclyl stammt,
• R₉ und R₁₀, die gleich oder verschieden sind, unabhängig voneinander aus der Reihe Wasserstoffatom, Halogenatom, Hydroxy-, Mercapto-, Nitro-, Thiocyanato-, Azido-, Cyano- oder Pentafluorsulfonylrest, Alkyl-, Halogenalkyl-, Alkoxy-, Halogenalkoxy-, Alkylthio-, Halogenalkylthio, Alkoxyalkyl-, Halogenalkoxyalkyl-, Alkylthioalkyl-, Halogenalkylthioalkyl-, Arylalkyl-, Cyanoalkyl-, Cyanoalkoxy-, Cyanoalkylthio-, Alkylsulfinyl-, Halogenalkylsulfinyl-, Alkylsulfonyl-, Halogenalkylsulfonyl- und Alkoxysulfonylrest stammen,
sowie die möglichen N-Oxide, geometrischen und/oder optischen Isomere, Enantiomere und/oder Diastereoisomere, tautomere Formen, ihre Salze, Metall- und Nichtmetallkomplexe,
wobei dieses Verfahren **dadurch gekennzeichnet ist, daß** man eine Verbindung der Formel (II), 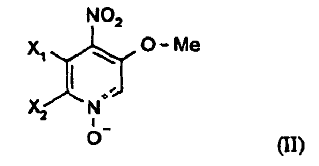 in der X₁ and X₂ wie oben definiert sind,
mit einem Cyanid oder Alkali- oder Erdalkalimetallderivaten der Cyanwasserstoffsäure in Gegenwart eines Alkylierungsmittels und eines Lösungsmittels oder mit Trimethylsilylcyanid in Gegenwart von Dimethylcarbamoylchlorid und einem Lösungsmittel umsetzt, wodurch man zu Verbindungen der Formel (III), 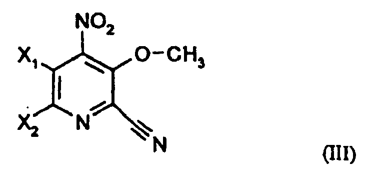 in denen X₁ and X₂ wie oben definiert sind, gelangt,
wobei diese Verbindungen der Formel (III) durch Umsetzen mit einem Acylhalogenit in Gegenwart eines Lösungsmittels in die entsprechenden Halogenderivate der Formel (IVa), 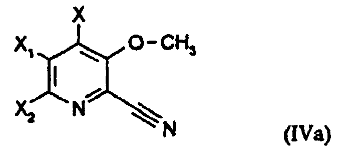 in denen X₁ and X₂ wie oben definiert sind und X ein Halogenatom aus der Reihe Fluor, Chlor, Brom oder Iod bedeutet, ungewandelt werden können,
wobei die Halogenderivate der Formel (IVa) nun durch Umsetzen mit Wasserstoffsäure im Heißen - oder mit einer starken Mineralsäure, gegebenenfalls in Gegenwart von Perhydrol - und gegebenenfalls mit Bortribromid zu Verbindungen der Formel (Ia), 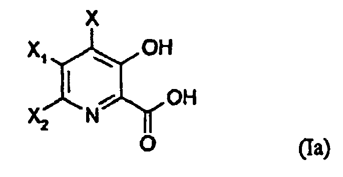 in denen X, X₁ und X₂ wie oben definiert sind, hydrolysiert werden,
wobei die Verbindungen der Formel (III) oder (IVa) auch mit einem Alkohol oder Alkoholat in Gegenwart eines Lösungsmittels, wie zum Beispiel vorzugsweise, jedoch nicht ausschließlich, einem protischen oder polaren aprotischen Lösungsmittel in Kontakt gebracht werden können, wodurch man zu den Verbindungen der Formel (IVb), 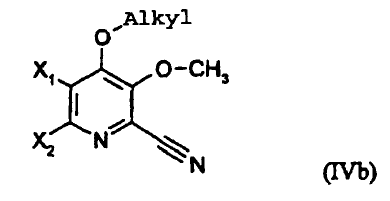 in denen X₁ und X₂ wie oben definiert sind, gelangt,
die anschließend unter ähnlichen Arbeitsbedingungen, wie sie für die Bildung der Verbindungen der Formel (Ia) verwendet wurden, hydrolysiert werden, wodurch man zu den Verbindungen der Formel (Ib) bzw. (Ib'), 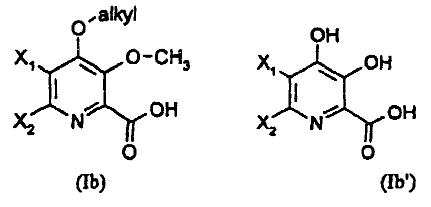 in denen X₁ und X₂ wie oben definiert sind, gelangt,
wobei die Verbindungen der Formel (IVa) auch in Picolinsäurederivate der Formel (Va), 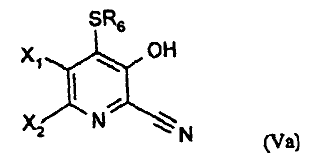 in denen X₁, X₂ und R₆ wie oben definiert sind,
umgewandelt werden können, und zwar dadurch, daß man eine Verbindung der Formel R₆SH oder ein entsprechendes Alkali- oder Erdalkalimetallsalz in einem aprotischen polaren Lösungsmittel bei einer Temperatur zwischen 0°C und der Siedetemperatur des Lösungsmittels umsetzt,
wobei die Nitrile der Formel (Va) nun gemäß einer Reaktion ähnlich der Reaktion, wie sie für die Bildung der Verbindungen der Formel (Ia) verwendet wurde, hydrolysiert werden können, wodurch man zu den entsprechenden Säuren der Formel (Ic), 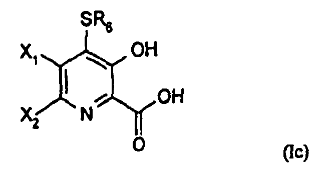 in denen X₁, X₂ und R₆ wie oben definiert sind, gelangt,
wobei die Halogenide der Formel (IVa) noch mit einem Salz der Stickstoffwasserstoffsäure behandelt werden können, wodurch man zu Verbindungen der Formel (Vb), 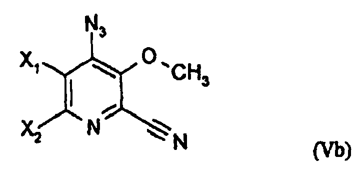 in denen X₁ und X₂ wie oben definiert sind, gelangt,
wobei diese Reaktion bei einer Temperatur zwischen 0°C und der Siedetemperatur des Lösungsmittels durchgeführt wird,
wobei die Verbindungen der Formel (Vb) nun nach ähnlichen Techniken, wie sie für die Herstellung der Säuren der Formel (Ia) oben beschrieben wurden, hydrolysiert werden können, wodurch man zu den Säuren der Formel (Id), 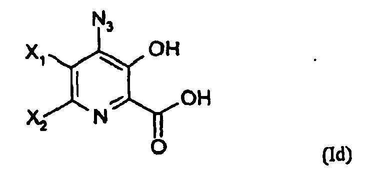 in denen X₁ und X₂ wie oben definiert sind, gelangt,
wobei die Nitride der Formel (Id) dann gegebenenfalls durch Einwirkung eines Reduktionsmittels zu Aminoderivaten der Formel (Ie), 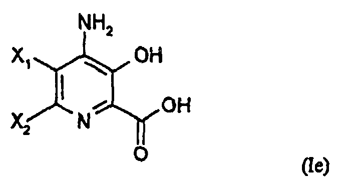 in denen X₁ und X₂ wie oben definiert sind, reduziert werden,
wobei die Säuren der Formeln (Ia) bis (Ie) nach traditionellen Techniken, mit denen der Fachmann auf dem Gebiet der organischen Synthese gut vertraut ist, zu Thiosäuren, Iminoderivaten (-C(=NR₁)) oder Amino-Iminoderivaten (-C(=N-NR₄R₅)) umgewandelt werden können,
wobei mit den Säuren (Ia) bis (Ie) oder ihren oben definierten Thio-, Imino- und Imino-Aminoderivaten, die in 3-Stellung (in Bezug auf das Pyridin-Stickstoffatom) durch -OH oder durch -Methoxy substituiert sind, verschiedene bereits im Stand der Technik bekannte Reaktionen durchgeführt werden können, um zu den entsprechenden Derivaten, die in 3-Stellung (in Bezug auf das Pyridin-Stickstoffatom) durch -O-Z substituiert sind, zu gelangen, wobei Z wie für die Verbindungen der Formel (I) definiert ist.

2. Herstellungsverfahren nach Anspruch 1, **dadurch gekennzeichnet, daß** Y den Aminorest (-NH₂) bedeutet und daß das Verfahren durch einen Schritt vervollständigt wird, der das Inkontaktbringen der erhaltenen Verbindung der Formel (I) mit einem Acylierungsmittel in Gegenwart eines Lösungsmittels und gegebenenfalls einer Base umfaßt, wodurch man zu den Verbindungen der Formeln (If) und (Ig), 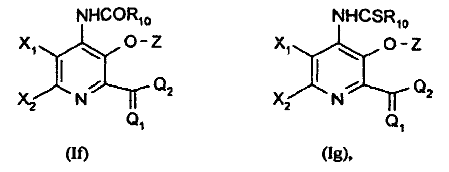 in denen X₁, X₂, Q₁, Q₂, Z und R₁₀ wie in Anspruch 1 definiert sind, gelangt.

3. Herstellungsverfahren nach Anspruch 1, **dadurch gekennzeichnet, daß** Q₂ -OH bedeutet und daß das Verfahren durch einen Schritt vervollständigt wird, der das Inkontaktbringen der erhaltenen Verbindung der Formel (I) mit einem Reaktanten der Formel R₂OH, R₃SH oder HNR₄R₅ in Gegenwart eines Aktivierungsmittels umfaßt, wodurch man zu den Verbindungen der Formeln (Ih), (Ii) bzw. (Ij), 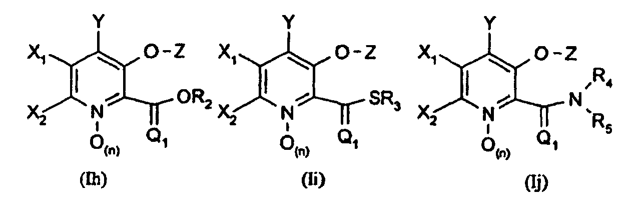 in denen X₁, X₂, Q₁, Q₂, Y, Z, n, R₄ und R₅ wie in Anspruch 1 definiert sind, gelangt.

4. Herstellungsverfahren nach Anspruch 1, **dadurch gekennzeichnet, daß** n gleich 0 ist und daß das Verfahren durch einen Schritt vervollständigt wird, der das Inkontaktbringen der erhaltenen Verbindung der Formel (I) mit einem Oxidationsmittel umfaßt, wodurch man zu den Verbindungen der Formel (Ik), 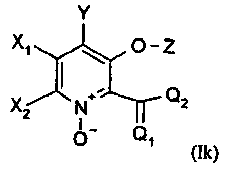 in denen X₁, X₂, Q₁, Q₂, Y und Z wie in Anspruch 1 definiert sind, gelangt.

5. Herstellungsverfahren nach einem der vorhergehenden Ansprüche, **dadurch gekennzeichnet, daß** das für die Halogenierung der Verbindungen der Formel (III) zu Verbindungen der Formel (IV) verwendete Lösungsmittel aus der Reihe Diethylether, Diisopropylether, Tetrahydrofuran, Dioxan und 1,2-Dimethoxyethan stammt.

6. Verfahren nach einem der vorhergehenden Ansprüche, **dadurch gekennzeichnet, daß** die Hydrolyse der Verbindungen der Formel (IV) zu Verbindungen der Formel (Ia) darin besteht, das Nitril der Formel (IVa) mit einer Säure zu behandeln, wobei diese Hydrolyse in einem Überschuß an Säure, in Gegenwart oder Abwesenheit eines Lösungsmittels, bei Rückfluß oder einer Temperatur zwischen 20°C und 200°C durchgeführt wird.

7. Herstellungsverfahren nach einem der vorhergehenden Ansprüche, **dadurch gekennzeichnet, daß** das für die Umwandlung der Verbindungen der Formel (IVa) zu Verbindungen der Formel (Va) verwendete Lösungsmittel aus der Reihe Dimethylformamid, Dimethylacetamid, N-Methylpyrrolidon, Dimethylprolylenharnstoff und Dimethylsulfoxid stammt.

8. Herstellungsverfahren nach einem der vorhergehenden Ansprüche, **dadurch gekennzeichnet, daß** das für die Umwandlung der Verbindungen der Formel (IVa) zu Verbindungen der Formel (Vb) verwendete Lösungsmittel aus der Reihe Dimethylformamid, Dimethylacetamid, N-Methylpyrrolidon, Dimethylprolylenharnstoff und Dimethylsulfoxid stammt.

9. Verfahren nach einem der vorhergehenden Ansprüche, **dadurch gekennzeichnet, daß** das für die Reduktion der Verbindungen der Formel (Id) zu Verbindungen der Formel (Ie) verwendete Reduktionsmittel aus der Reihe Lithiumaluminiumhydrid, Triphenylphosphin und Wasserstoff in Gegenwart eines Katalysators stammt.

10. Verfahren nach Anspruch 2, **dadurch gekennzeichnet, daß** das in dem Herstellungsschritt der Verbindungen der Formel (If) und (Ig) verwendete Acylierungsmittel aus der Reihe Acylhalogenid, Anhydrid, Säure, Ester, primäres Amid und ihre Thio-Homologen stammt.

11. Verfahren nach Anspruch 3, **dadurch gekennzeichnet, daß** das für die Bildung der Verbindungen der Formel (Ih), (Ii) bzw. (Ij) verwendete Aktivierungsmittel aus der Reihe Thionylchlorid, Oxalylchlorid, Dicyclocarbodiimid, 1-(3-Dimethylaminopropyl)-3-ethylcarbodiimidhydrochlorid, 1-Hydroxybenzotriazol und Phosphoroxychlorid stammt.

12. Verfahren nach Anspruch 3 oder 11, **dadurch gekennzeichnet, daß** die Reaktion, mit der die Herstellung von Verbindungen der Formel (Ih), (Ii) bzw. (Ij) ermöglicht wird, in Gegenwart einer organischen oder anorganischen Base aus der Reihe Alkali- und Erdalkalimetallhydroxide, Alkali- und Erdalkalimetallalkoholate, Alkali- und Erdalkalimetallhydride, Alkali- und Erdalkalimetallcarbonate und -hydrogencarbonate und organische Stickstoffbasen stammt.

13. Verfahren nach einem der vorhergehenden Ansprüche, **dadurch gekennzeichnet, daß** es sich bei den 3-Hydroxypicolinsäurederivaten der allgemeinen Formel (I) um folgende handelt:
• 3-Hydroxy-N-{[3-(trifluormethyl)benzyl]oxy}-2-pyridincarboxamid,
• 1-{3-Hydroxy-2-[(4-phenoxyanilino)carbonyl]-4-pyridinyl}-1,2-triazadien-2-ium,
• 4-Amino-3-hydroxy-N-{4-[4-(trifluormethyl)-phenoxy]phenyl}-2-pyridincarboxamid,
• 4-Amino-3-hydroxy-N-[4-(4-methylphenoxy)phenyl]-2-pyridincarboxamid,
• 4-(Formylamino)-3-hydroxy-N-{4-[3-(trifluormethyl)phenoxy]phenyl}-2-pyridincarboxamid
• N-[4-(4-Chlorphenoxy)phenyl]-4-(formylamino)-3-hydroxy-2-pyridincarboxamid,
• 4-(Formylamino)-3-hydroxy-N-{4-[4-(trifluoromethyl)phenoxy]phenyl}-2-pyridincarboxamid und
• N-[4-(Benzyloxy)phenyl]-4-(formylamino)-3-hydroxy-2-pyridincarboxamid,
sowie deren gegebenenfalls vorhandene N-Oxide, geometrische und/oder optische Isomere, Enantiomere und/oder Diastereoisomere, tautomere Formen, Salze, Metall- und Nichtmetallkomplexe.

## Claims

1. Process for preparing compounds of general formula (I) : 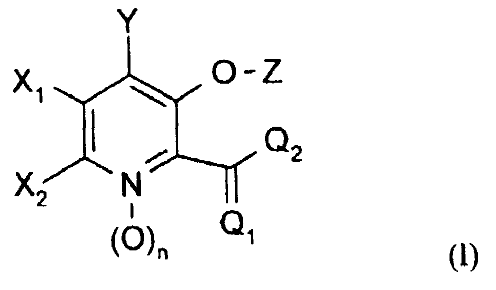 in which:
• n represents 0 or 1,
• Q₁ is chosen from an oxygen or sulfur atom, a group NR₁ and a group N-NR₄R₅,
• Q₂ is chosen from a group OR₂ or SR₃ and a group -NR₄R₅, or
• Q₁ and Q₂ may together form a ring of 5 to 7 atoms containing 2 to 3 oxygen and/or nitrogen atoms, optionally substituted with one or more radicals, which may be identical or different, chosen from halogens and alkyl and haloalkyl radicals,
• Z is chosen from a hydrogen atom, a cyano radical and an alkyl, allyl, aryl, arylalkyl, propargyl, cycloalkyl, halocycloalkyl, alkenyl, alkynyl, cyanoalkyl, haloalkyl, alkoxyalkyl, haloalkoxyalkyl, alkylthioalkyl, haloalkylthioalkyl, N-alkylaminoalkyl, N,N-dialkylaminoalkyl, acylaminoalkyl, alkoxycarbonylaminoalkyl, aminocarbonylaminoalkyl, alkoxycarbonyl, N-alkylaminocarbonyl, N,N-dialkylaminocarbonyl, acyl, thioacyl, alkoxythiocarbonyl, N-alkylaminothiocarbonyl, N,N-dialkylaminothiocarbonyl, alkylsulfinyl, haloalkylsulfinyl, alkylsulfonyl, haloalkylsulfonyl, alkoxysulfonyl, aminosulfonyl, N-alkylaminosulfonyl, N,N-dialkylaminosulfonyl, arylsulfinyl, arylsulfonyl, aryloxysulfonyl, N-arylaminosulfonyl, N,N-diarylaminosulfonyl or N,N-arylalkylaminosulfonyl radical;
• Y is chosen from a hydrogen atom, a halogen atom, a hydroxyl, mercapto, nitro, thiocyanato, azido, cyano or pentafluorosulfonyl radical, an alkyl, haloalkyl, alkoxy, haloalkoxy, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulfinyl, haloalkylsulfinyl, alkylsulfonyl, haloalkylsulfonyl or alkoxysulfonyl radical, a cycloalkyl, halocycloalkyl, alkenyl, alkynyl, alkenyloxy, alkynyloxy, alkenylthio or alkynylthio group,
an amino, N-alkylamino, N,N-dialkylamino, -NHCOR₁₀, -NHCSR₁₀, N-alkylaminocarbonylamino, N,N-dialkylaminocarbonylamino, aminoalkyl, N-alkylaminoalkyl, N,N-dialkylaminoalkyl, acylaminoalkyl, thioacylamino, alkoxythiocarbonylamino, N-alkylaminothiocarbonylamino, N,N-dialkylaminothiocarbonylamino, N,N-arylalkylaminocarbonylamino, N-alkylsulfinylamino, N-alkylsulfonylamino, N-arylsulfinylamino, N-arylsulfonylamino, N-alkoxysulfonylamino, N-alkoxysulfinylamino, N-haloalkoxysulfinylamino, N-haloalkoxysulfonylamino, N-arylamino, N,N-diarylamino, arylcarbonylamino, alkoxycarbonylamino, N-arylaminocarbonylamino, N,N-diarylaminocarbonylamino, arylthiocarbonylamino, aryloxythiocarbonylamino, N-arylaminothiocarbonylamino, N,N-diarylaminothiocarbonylamino or N,N-arylalkylaminothiocarbonylamino group, an acyl, carboxyl, carbamoyl, N-alkylcarbamoyl, N,N-dialkylcarbamoyl, lower alkoxycarbonyl, N-arylcarbamoyl, N,N-diarylcarbamoyl, aryloxycarbonyl or N,N-arylalkylcarbamoyl group, and an imino group of formula: 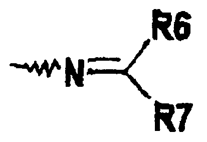
• X₁ and X₂ are identical or different and chosen, independently of each other, from a hydrogen atom, a halogen atom, a hydroxyl, mercapto, nitro, thiocyanato, azido, cyano or pentafluorosulfonyl radical, and an alkyl, haloalkyl, alkoxy, haloalkoxy, alkylthio, haloalkylthio, alkoxyalkyl, haloalkoxyalkyl, alkylthioalkyl, haloalkylthioalkyl, cyanoalkyl, cyanoalkoxy, cyanoalkylthio, alkylsulfinyl, haloalkylsulfinyl, alkylsulfonyl, haloalkylsulfonyl or alkoxysulfonyl radical, or
• X₁ and X₂ may also be joined together, thus forming a saturated, partially unsaturated or totally unsaturated 4- to 8-membered ring optionally comprising one or more hetero atoms chosen from sulfur, oxygen, nitrogen and phosphorus,
• R₂ and R₃ are identical or different and chosen, independently of each other, from an alkyl radical comprising from 1 to 12 carbon atoms, a haloalkyl, cycloalkyl, halocycloalkyl, alkoxy, haloalkoxy, alkylthio, haloalkylthio, alkoxyalkyl, haloalkoxyalkyl, alkylthioalkyl, haloalkylthioalkyl, cyanoalkyl or acyl radical, a nitro, cyano, carboxyl, carbamoyl or 3-oxetanyloxycarbonyl radical, and an N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl, alkynyl, N-alkylamino, N,N-dialkylamino, N-alkylaminoalkyl or N,N-dialkylaminoalkyl radical, or
a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
• R₁, R₄, R₅, R₆ and R₇ are identical or different and chosen, independently of each other, from a hydrogen atom, an optionally substituted alkyl radical comprising from 1 to 12 carbon atoms in a linear or branched chain, a haloalkyl, cycloalkyl, halocycloalkyl, alkoxy, aryloxy, arylalkoxy, haloalkoxy, alkylthio, haloalkylthio, alkoxyalkyl, haloalkoxyalkyl, alkylthioalkyl, haloalkylthioalkyl, cyanoalkyl or acyl radical, a nitro, cyano, carboxyl, carbamoyl or 3-oxetanyloxycarbonyl radical, and an N-alkylcarbamoyl, N,N-dialkylcarbamoyl, alkoxycarbonyl, alkylthiocarbonyl, haloalkoxycarbonyl, alkoxythiocarbonyl, haloalkoxythiocarbonyl, alkylthiothiocarbonyl, alkenyl, alkynyl, N-alkylamino, N,N-dialkylamino, N-alkylaminoalkyl or N,N-dialkylaminoalkyl radical, or
a radical chosen from aryl, arylalkyl, heterocyclyl and heterocyclylalkyl, optionally substituted with one or more radicals R₉ and/or aryl and/or arylalkyl, which may be identical or different, and/or a group -T-R₈, or
• R₄ and R₅, on the one hand, or R₆ and R₇, on the other hand, may be joined together, thus forming a saturated, partially unsaturated or totally unsaturated 4- to 8-membered ring optionally comprising one or more hetero atoms chosen from sulfur, oxygen, nitrogen and phosphorus,
• T represents a direct bond or a divalent radical chosen from a radical -(CH₂)ₘ-, m taking a value between 1 and 12, limits included, the said radical optionally being interrupted or ending with one or two hetero atoms chosen from nitrogen, oxygen and/or sulfur, and an oxyalkylene, alkoxyalkylene, carbonyl (-CO-), oxycarbonyl (-O-CO-), carbonyloxy (-CO-O-), sulfinyl (-SO-), sulfonyl (-SO₂-), oxysulfonyl (-O-SO₂-), sulfonyloxy (-SO₂-O-), oxysulfinyl (-O-SO-), sulfinyloxy (-SO-O-), thio (-S-), oxy (-O-), vinyl (-C=C-), ethynyl (-C≡C-), -NR₉-, -NR₉O-, -ONR₉-, -N=N-, -NR₉-NR₁₀-, -NR₉-S-, -NR₉-SO-, -NR₉-SO₂-, -S-NR₉-, -SO-NR₉-, -SO₂-NR₉-, -CO-NR₉-O- or -O-NR₉-CO- radical,
• R₈ is chosen from a hydrogen atom and an aryl or heterocyclyl radical,
• R₉ and R₁₀, which may be identical or different, are chosen, independently of each other, from a hydrogen atom, a halogen atom, a hydroxyl, mercapto, nitro, thiocyanato, azido, cyano or pentafluorosulfonyl radical, and an alkyl, haloalkyl, alkoxy, haloalkoxy, alkylthio, haloalkylthio, alkoxyalkyl, haloalkoxyalkyl, alkylthioalkyl, haloalkylthioalkyl, arylalkyl, cyanoalkyl, cyanoalkoxy, cyanoalkylthio, alkylsulfinyl, haloalkylsulfinyl, alkylsulfonyl, haloalkylsulfonyl or alkoxysulfonyl radical,
as well as the optional N-oxides, geometrical and/or optical isomers, enantiomers and/or diastereoisomers, tautomeric forms, their salts and metal and metalloid complexes,
a process **characterized in that** a compound of formula (II) 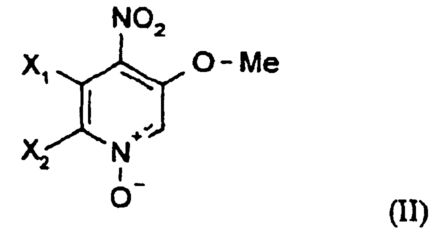
in which X₁ and X₂ are as previously defined, is reacted with a cyanide, alkali metal derivatives or alkaline-earth metal derivatives of hydrocyanic acid in the presence of an alkylating agent and a solvent, or with trimethylsilyl cyanide in the presence of dimethylcarbamoyl chloride and a solvent,
to give the compounds of formula (III): 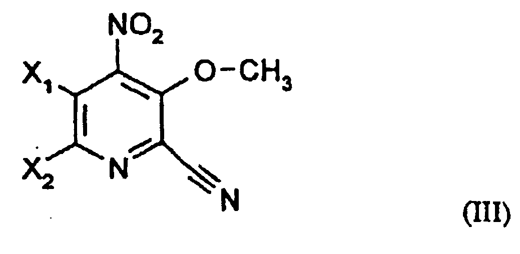 in which X₁ and X₂ are as previously defined,
the compounds of formula (III) above can be converted into corresponding halo derivatives of formula (IVa): 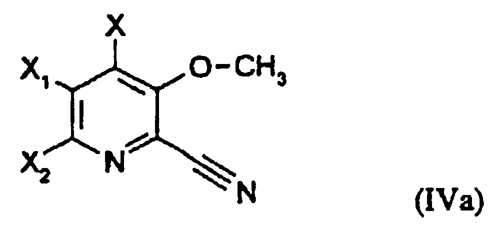 in which X₁ and X₂ are as previously defined and X represents a halogen atom chosen from fluorine, chlorine, bromine and iodine,
by reaction with an acyl halide in the presence of a solvent,
the halo derivatives of formula (IVa) then being hydrolysed to compounds of formula (Ia): 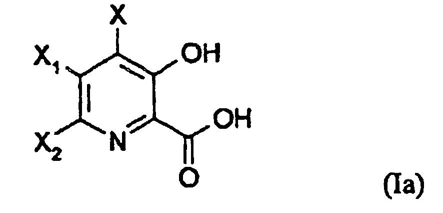 in which X, X₁ and X₂ are as previously defined,
by the action of hot hydracid - or of a strong mineral base, optionally in the presence of aqueous hydrogen peroxide solution - and optionally of boron tribromide,
the compounds of formula (III) or (IVa) then possibly being placed in contact with an alcohol or an alkoxide in the presence of a solvent such as, preferably, but not exclusively, a protic or aprotic polar solvent, to give the compounds of formula (IVb): 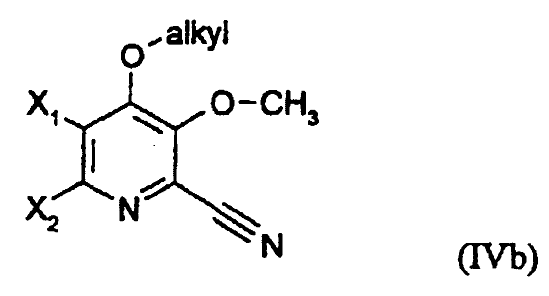 in which X₁ and X₂ are as previously defined, and then used in a hydrolysis reaction under operating conditions similar to those used for the formation of the compounds of formula (Ia), to give the compounds of respective formulae (Ib) and (Ib') 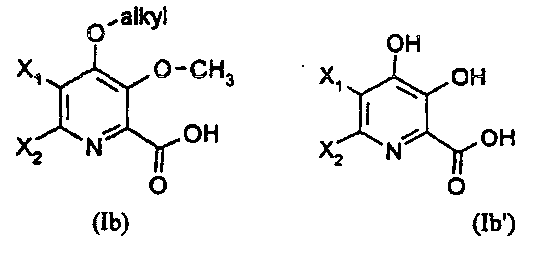 in which X₁ and X₂ are as previously defined, the compounds of formula (IVa) also possibly being converted into picolinic acid derivatives of formula (Va): 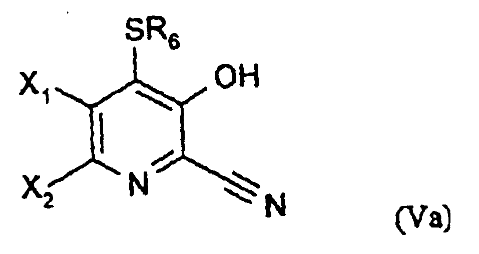 in which X₁, X₂ and R₆ are as previously defined, by reacting a compound of formula R₆SH, or a corresponding alkali metal salt or alkaline-earth metal salt, in an aprotic polar solvent, at a temperature of between 0°C and the boiling point of the solvent,
the nitriles of formula (Va) then being used in a hydrolysis reaction to give the corresponding acids of formula (Ic): 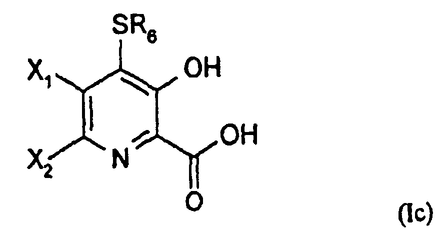 in which X₁, X₂ and R₆ are as previously defined, according to a reaction similar to that used to form the compounds of formula (Ia),
the halides of formula (IVa) also possibly being treated with an azothydric acid salt, to give the compounds of formula (Vb): 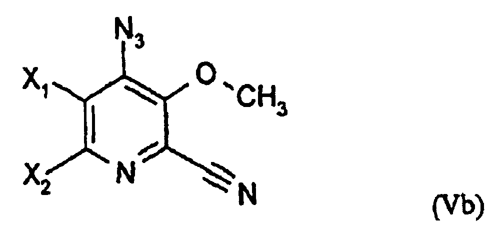 in which X₁ and X₂ are as previously defined,
this reaction being carried out at a temperature of between 0°C and the boiling point of the solvent,
the compounds of formula (Vb) can then be hydrolysed according to techniques similar to those presented for the preparation of the acids of formula (Ia) above, to give the acids of formula (Id): 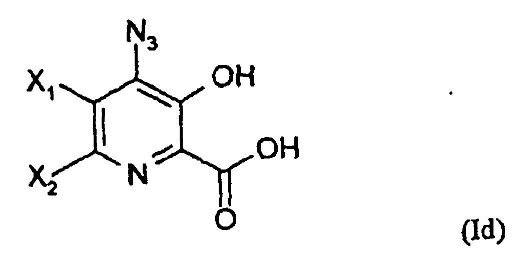 in which X₁ and X₂ are as previously defined,
the azides of formula (Id) then being optionally reduced to amine derivatives of formula (Ie): 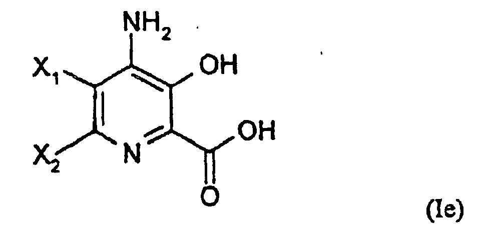 in which X₁ and X₂ are as previously defined, by the action of a reducing agent,
the acids of formulae (Ia) to (Ie) possibly being converted into thio acids, imino derivatives (-C(-NR₁)) or amino imino derivatives (-C(=N-NR₄R₅)) according to conventional techniques, that are well known to those skilled in the art of organic synthesis, the acids (Ia) to (Ie), or the thio, imino and imino-amino derivatives thereof defined above, substituted in position 3 (relative to the pyridine nitrogen atom) with -OH or -methoxy then possibly being subjected to various reactions already known in the prior art in order to give the corresponding derivatives substituted in position 3 (relative to the pyridine nitrogen atom) with -O-Z, Z being as defined for the compounds of formula (I).

2. Preparation process according to Claim 1, **characterized in that** Y represents the amino radical (-NH₂) and **in that** the process is completed by a step that includes placing the compound of formula (I) obtained in contact with an acylating agent in the presence of a solvent and optionally of a base in order to give the compounds of formulae (If) and (Ig): 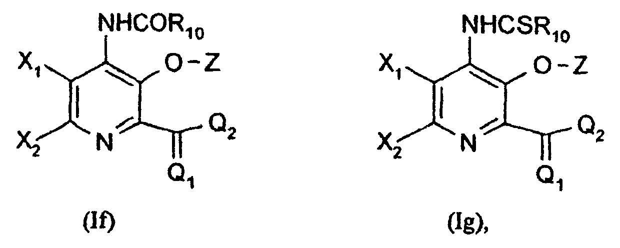 in which X₁, X₂, Q₁, Q₂, Z and R₁₀ are as defined in Claim 1.

3. Preparation process according to Claim 1, **characterized in that** Q₂ represents -OH and **in that** the process is completed by a step that includes placing the contact of formula (I) obtained in contact with a reagent of formula R₂OH, R₃SH or HNR₄R₅ in the presence of an activating agent, to give the compounds of formulae (Ih), (Ii) and (Ij), respectively: 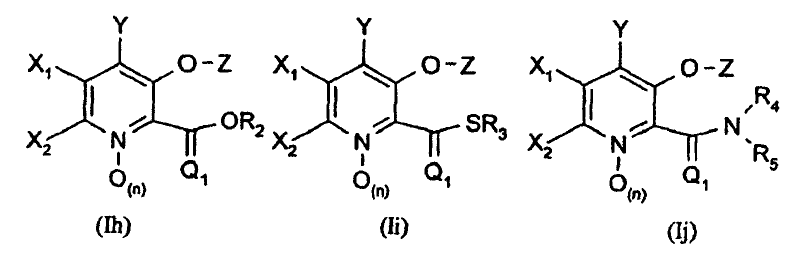 in which X₁, X₂, Q₁, Q₂, Y, Z, n, R₄ and R₅ are as defined in Claim 1.

4. Preparation process according to Claim 1, **characterized in that** n is equal to 0 and **in that** the process is completed by a step that includes placing the compound of formula (I) obtained in contact with an oxidizing agent to obtain a compound of formula (Ik) 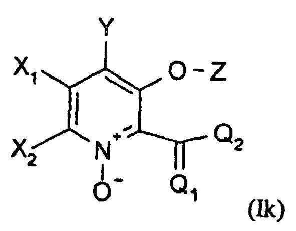 in which X₁, X₂, Q₁, Q₂, Y and Z are as defined in Claim 1.

5. Preparation process according to any one of the preceding claims, **characterized in that** the solvent used for the halogenation reaction of the compounds of formula (III) to the compounds of formula (IV) is chosen from diethyl ether, diisopropyl ether, tetrahydrofuran, dioxane and 1,2-dimethoxyethane.

6. Process according to any of the preceding claims, **characterized in that** the hydrolysis reaction of the compounds of formula (IV) into compounds of formula (Ia) consists in treating the nitrile of formula (IVa) with an acid, this hydrolysis reaction being performed in the excess acid, in the absence or presence of a solvent, at reflux or at a temperature of between 20°C and 200°C.

7. Process according to any one of the preceding claims, **characterized in that** the solvent used in the reaction for conversion of the compounds of formula (IVa) into compounds of formula (Va) is chosen from dimethylformamide, dimethylacetamide, N-methylpyrrolidone, dimethylprolyleneurea and dimethyl sulfoxide.

8. Process according to any one of the preceding claims, **characterized in that** the solvent used in the reaction for conversion of the compounds of formula (IVa) into compounds of formula (Vb) is chosen from dimethylformamide, dimethylacetamide, N-methylpyrrolidone, dimethylprolyleneurea and dimethyl sulfoxide.

9. Process according to any one of the preceding claims, **characterized in that** the reducing agent used for the reduction of the compounds of formula (Id) to compounds of formula (Ie) is chosen from lithium aluminium hydride, triphenylphosphine and hydrogen in the presence of a catalyst.

10. Process according to Claim 2, **characterized in that** the acylating agent used in the step for preparing the compounds of formulae (If) and (Ig) is chosen from an acyl halide, an anhydride, an acid, an ester and a primary amide, and thio homologues thereof.

11. Process according to Claim 3, **characterized in that** the activating agent used for the formation of the compounds of formula (Ih), (Ii) or (Ij) is chosen from thionyl chloride, oxalyl chloride, dicyclocarbodiimide, 1-(3-dimethylaminopropyl)-3-ethylcarbodiimide hydrochloride, 1-hydroxybenzotriazole and phosphorus oxychloride.

12. Process according to Claim 3 or 11, **characterized in that** the reaction for obtaining the compounds of formula (Ih), (Ii) or (Ij) is performed in the presence of an organic or mineral base chosen from alkali metal hydroxides and alkaline-earth metal hydroxides, alkali metal alkoxides and alkaline-earth metal alkoxides, alkali metal hydrides and alkaline-earth metal hydrides, alkali metal carbonates and bicarbonates and alkaline-earth metal carbonates and bicarbonates, and organonitrogen bases.

13. Process according to any one of the preceding claims, **characterized in that** the 3-hydroxypicolinic acid derivatives of general formula (I) are:
• 3-hydroxy-N-{[3-(trifluoromethyl)benzyl]oxy}-2-pyridine carboxamide,
• 1-{3-hydroxy-2-[(4-phenoxyanilino)carbonyl]-4-pyridinyl}-1,2-triazadien-2-ium,
• 4-amino-3-hydroxy-N-{4-[4-(trifluoromethyl)-phenoxy]phenyl}-2-pyridine carboxamide,
• 4-amino-3-hydroxy-N-[4-(4-methylphenoxy)phenyl]-2-pyridine carboxamide,
• 4-(formylamino)-3-hydroxy-N-{4-[3-(trifluoromethyl)phenoxy]phenyl}-2-pyridine carboxamide
• N-[4-(4-chlorophenoxy)phenyl]-4-(formylamino)-3-hydroxy-2-pyridine carboxamide,
• 4-(formylamino)-3-hydroxy-N-{4-[4-(trifluoromethyl)phenoxy]phenyl}-2-pyridine carboxamide and
• N-[4-(benzyloxy)phenyl]-4-(formylamino)-3-hydroxy-2-pyridine carboxamide,
and also the possible N-oxides, geometrical and/or optical isomers, enantiomers and/or diastereoisomers, tautomeric forms, salts, metallic and metalloid complexes thereof.
